# EUROPEAN PATENT SPECIFICATION

(11) **EP 1 053 009 B1**
(45) Date of publication and mention of the grant of the patent: **22.09.2004**
(21) Application number: 99905717.7
(22) Date of filing: 03.02.1999
(51) Int. Cl.: A61K 38/18, A61K 39/395, A61K 38/17, A61K 35/12, C07K 14/475

(54) **USE OF HEREGULIN AS AN EPITHELIAL CELL GROWTH FACTOR**
VERWENDUNG DES HEREGULINS ALS EPITHELZELLENWACHSTUMSFAKTOR
UTILISATION DE L'HEREGULINE COMME FACTEUR DE CROISSANCE DE CELLULE EPITHELIALE

(30) Priority: 04.02.1998 US 20598
(43) Date of publication of application: 22.11.2000
(73) Proprietor: Genentech, Inc., South San Francisco, CA 94080-4990 (US); University of Iowa Research Foundation, Iowa City, IA 55242-5000 (US)
(72) Inventor: SLIWKOWSKI, Mark, Iowa City, IA 94070 (US); KERN, Jeffrey, A., Iowa City, IA 52240 (US)
(74) Representative: Kiddle, Simon John
(86) International application number: PCT/US1999/002390
(87) International publication number: WO 1999/039729

(56) References cited:
- WO-A-96/15244
- US-A- 5 367 060
- PATEL, N. V. ET AL: "Heregulin (HRG) and human epidermal receptor ( HER ) 2 and 3 are modulated during in vitro human lung growth and differentiation." JOURNAL OF INVESTIGATIVE MEDICINE, (1997) VOL. 45, NO. 3, PP. 284A, XP002113357

## Description

### BACKGROUND OF THE INVENTION

### Field of the Invention

This invention relates to the use of HER2, HER3 and/or HER4 ligands, in particular heregulin polypeptides, as epithelial cell growth factors.

### Description of Background and Related Art

The HER (ErbB) family belongs to the subclass I receptor tyrosine kinase superfamily and consists of three distinct receptors, HER2, HER3, and HER4. A ligand for this ErbB family is the protein heregulin (HRG), a multidomain containing protein with at least 15 distinct isoforms.

Transduction of signals that regulate cell growth and differentiation is regulated in part by phosphorylation of various cellular proteins. Protein tyrosine kinases are enzymes that catalyze this process. Receptor protein tyrosine kinases are believed to direct cellular growth via ligand-stimulated tyrosine phosphorylation of intracellular substrates. Growth factor receptor protein tyrosine kinases of the class I subfamily include the 170 kDa epidermal growth factor receptor (EGFR) encoded by the *erb*B1 gene. *erb*B1 has been causally implicated in human malignancy. In particular. increased expression of this gene has been observed in more aggressive carcinomas of the breast, bladder, lung and stomach.

The second member of the class I subfamily, p185^{neu} was originally identified as the product of the transforming gene from neuroblastomas of chemically treated rats. The *neu* gene (also called *erb*B2 and HER2) encodes a 185 kDa receptor protein tyrosine kinase. Amplification and/or overexpression of the human HER2 gene correlates with a poor prognosis in breast and ovarian cancers (Slamon *et al., Science* 235:177-182 (1987); and Slamon *et al., Science* 244:707-712 (1989)). Overexpression of HER2 has been correlated with other carcinomas including carcinomas of the stomach, endometrium, salivary gland, lung, kidney, colon and bladder. Accordingly, Slamon *et al*. in US Pat No. 4,968,603 describe and claim various diagnostic assays for determining HER2 gene amplification or expression in tumor cells. Slamon *et al*. discovered that the presence of multiple gene copies of HER2 oncogene in tumor cells indicates that the disease is more likely to spread beyond the primary tumor site, and that the disease may therefore require more aggressive treatment than might otherwise be indicated by other diagnostic factors. Slamon *et al*. conclude that the HER2 gene amplification test, together with the determination of lymph node status, provides greatly improved prognostic utility.

A further related gene, called *erb*B3 or HER3, has also been described. See US Pat. No. 5,183,884; Kraus *et al., Proc. Natl*. *Acad Sci. USA* 86:9193-9197 (1989); EP Pat Appln No 444,961A1; and Kraus *et al., Proc. Natl. Acad. Sci. USA* 90:2900-2904 (1993). Kraus *et al*. (1989) discovered that markedly elevated levels of *erb*B3 mRNA were present in certain human mammary tumor cell lines indicating that *erb*B3, like *erb*B1 and *erb*B2, may play a role in human malignancies. Also, Kraus *et al.* (1993) showed that EGF-dependent activation of the ErbB3 catalytic domain of a chimeric EGFR/ErbB3 receptor resulted in a proliferative response in transfected NIH-3T3 cells. This is now believed to be the result of endogenous ErbB1 or ErbB2 in NIH-3T3. Furthermore, these researchers demonstrated that some human mammary tumor cell lines display a significant elevation of steady-state ErbB3 tyrosine phosphorylation further indicating that this receptor may play a role in human malignancies. The role of *erb*B3 in cancer has been explored by others. It has been found to be overexpressed in breast (Lemoine *et al*., *Br. J. Cancer* 66:1116-1121 (1992)), gastrointestinal (Poller *et al., J. Pathol*. 168:275-280 (1992), Rajkumer *et al., J. Pathol*. 170:271-278 (1993), and Sanidas *et al., Int. J. Cancer* 54:935-940 (1993)), and pancreatic cancers (Lemoine *et al., j. Pathol*. 168:269-273 (1992), and Friess *et al., Clinical Cancer Research* 1:1413-1420 (1995)).

The class 1 subfamily of growth factor receptor protein tyrosine kinases has been further extended to include the HER4/Erb4 receptor. See EP Pat Appin No 599,274; Plowman *et al*., *Proc. Natl*. *Acad Sci*. *USA* 90:1746-1750 (1993); and Plowman *et al*., *Nature* 366:473-475 (1993). Plowman *et al*. found that increased HER4 expression closeiy correlated with certain carcinomas of epithelial origin, including breast adenocarcinomas. Diagnostic methods for detection of human neoplastic conditions (especially breast cancers) which evaluate HER4 expression are described in EP Pat Appln No. 599,274.

The quest for the activator of the HER2 oncogene has lead to the discovery of a family of heregulin polypeptides. These proteins appear to result from alternate splicing of a single gene which was mapped to the short arm of human chromosome 8 by Orr-Urtreger *et al*., *Proc. Natl*. *Acad Sci. USA* 90:1867-1871(1993). See also Lee and Wood, *Genomics*, 16:790-791 (1993).

Holmes *et al*. isolated and cloned a family of polypeptide activators for the HER2 receptor which they called heregulin-αα (HRG-α), heregulin-β1 (HRG-β1), heregulin-β2 (HRG-β2), heregulin-β2-like (HRG-β2-like), and heregulin-β3 (HRG-β3). See Holmes *et al*., *Science* 256:1205-1210 (1992); WO 92/20798; and U.S. Patent 5,367,060. The 45 kDa polypeptide, HRG-α, was purified from the conditioned medium of the MDA-MB-231 human breast cancer cell line. These researchers demonstrated the ability of the purified heregulin polypeptides to activate tyrosine phosphorylation of the HER2 receptor in MCF7 breast tumor cells. Furthermore, the mitogenic activity of the heregulin polypeptides on SK-BR-3 cells (which express high levels of the HER2 receptor) was illustrated. Like other growth factors which belong to the EGF family, soluble HRG polypeptides appear to be derived from a membrane bound precursor (called pro-HRG) which is proteolytically processed to release the 45kDa soluble form. These pro-HRGs lack a N-terminal signal peptide.

While heregulins are substantially identical in the first 213 amino acid residues, they are classified into two major types, α and β, based on two variant EGF-like domains which differ in their C-terminal portions. Nevertheless, these EGF-like domains are identical in the spacing of six cysteine residues contained therein. Based on an amino acid sequence comparison, Holmes *et al*. found that between the first and sixth cysteines in the EGF-like domain, HRGs were 45% similar to heparin-binding EGF-like growth factor (HB-EGF), 35% identical to amphiregulin (AR), 32% identical to TGF-α, and 27% identical to EGF.

The 44 kDa *neu* differentiation factor (NDF), which is the rat equivalent of human HRG, was first described by Peles *et al., Cell*, 69:205-216 (1992); and Wen *et al., Cell*, 69:559-572 (1992). Like the HRG polypeptides, NDF has an immunoglobulin (1g) homology domain followed by an EGF-like domain and lacks a N-terminal signal peptide. Subsequently, Wen *et al., Mol. Cell. Biol*., 14(3): 1909-1919 (1994) carried out "exhaustive cloning" to extend the family of NDFs. This work revealed six distinct fibroblastic pro-NDFs. Adopting the nomenclature of Holmes *et al*., the NDFs are classified as either α or β polypeptides based on the sequences of the EGF-like domains. Isoforms I to 4 are characterized on the basis of the variable justamembrane stretch (between the EGF-like domain and transmembrane domain). Also, isoforms a, b and c are described which have variable length cytoplasmic domains. These researchers conclude that different NDF isoforms are generated by alternative splicing and perform distinct tissue-specific functions. See also EP 505 148; WO 93/22424; and WO 94/28133 concerning NDF.

Falls *et al., Cell*, 72:801-815 (1993) describe another member of the heregulin family which they call acetylcholine receptor inducing activity (ARIA) polypeptide. The chicken-derived ARIA polypeptide stimulates synthesis of muscle acetylcholine receptors. See also WO 94/08007. ARIA is a β-type heregulin and lacks the entire spacer region rich in glycosylation sites between the Ig-like domain and EGF-like domain of HRGα, and HRGβ1-β3.

Marchionni *et al., Nature*, 362:312-318 (1993) identified several bovine-derived proteins which they call glial growth factors (GGFs). These GGFs share the Ig-like domain and EGF-like domain with the other heregulin proteins described above, but also have an amino-terminal kringle domain. GGFs generally do not have the complete glycosylated spacer region between the Ig-like domain and EGF-like domain. Only one of the GGFs, GGFII, possessed a N-terminal signal peptide. See also WO 92/18627; WO 94/00140; WO 94/04560; WO 94/26298; and WO 95/32724 which refer to GGFs and uses thereof.

Ho *et al*. in *J*. *Biol*. *Chem.* 270(4):14523-14532 (1995) describe another member of the heregulin family called sensory and motor neuron-derived factor (SMDF). This protein has an EGF-like domain characteristic of all other heregulin polypeptides but a distinct N-terminal domain. The major structural difference between SMDF and the other heregulin polypeptides is the lack in SMDF of the Ig-like domain and the "glyco" spacer characteristic of all the other heregulin polypeptides. Another feature of SMDF is the presence of two stretches of hydrophobic amino acids near the N-terminus.

While the heregulin polypeptides were first identified based on their ability to activate the HER2 receptor (see Holmes *et al., supra*), it was discovered that certain ovarian cells expressing *neu* and *neu-* transfected fibroblasts did not bind or crosslink to NDF, nor did they respond to NDF to undergo tyrosine phosphorylation (Peles *et al*., *EMBO J*. 12:961-971 (1993)). This indicated another cellular component was necessary for conferring full heregulin responsiveness. Carraway *et al*. subsequently demonstrated that ¹²⁵I-rHRGβ1₁₇₇₋₂₄₄ bound to NIH-3T3 fibroblasts stably transfected with bovine *erb*B3 but not to non-transfected parental cells. Accordingly, they conclude that ErbB3 is a receptor for HRG and mediates phosphorylation of intrinsic tyrosine residues as well as phosphorylation of ErbB2 receptor in cells which express both receptors. Carraway *et al., J. Biol. Chem*. 269(19):14303-14306 (1994). Sliwkowski *et al., J. Biol. Chem*. 269(20):14661-14665 (1994) found that cells transfected with HER3 alone show low affinities for heregulin, whereas cells transfected with both HER2 and HER3 show higher affinities.

This observation correlates with the "receptor cross-talking" described previously by Kokai *et al., Cell* 58:287-292 (1989); Stern *et al., EMBO J*. 7:995-1001 (1988); and King *et al*., 4:13-18 (1989). These researchers found that binding of EGF to the EGFR resulted in activation of the EGFR kinase domain and cross-phosphorylation of p185^{HER2}. This is believed to be a result of ligand-induced receptor heterodimerization and the concomitant cross-phosphorylation of the receptors within the heterodimer (Wada *et al., Cell* 61:1339-1347 (1990)).

Plowman and his colleagues have similarly studied p185^{HER4}/p185^{HER2} activation. They expressed p185^{HER2} alone, p 185^{HER4} alone, or the two receptors together in human T lymphocytes and demonstrated that heregulin is capable of stimulating tyrosine phosphorylation of p185^{HER4}, but could only stimulate p185^{HER2} phosphorylation in cells expressing both receptors. Plowman *et al., Nature* 336:473-475 (1993).

The biological role of heregulin has been investigated by several groups. For example, Falls *et al*., (discussed above) found that ARIA plays a role in myotube differentiation, namely affecting the synthesis and concentration of neurotransmitter receptors in the postsynaptic muscle cells of motor neurons. Corfas and Fischbach demonstrated that ARIA also increases the number of sodium channels in chick muscle. Corfas and Fischbach, *J. Neuroscience*, 13(5): 2118-2125 (1993). It has also been shown that GGFII is mitogenic for subconfluent quiescent human myoblasts and that differentiation of clonal human myoblasts in the continuous presence of GGFII results in greater numbers of myotubes after six days of differentiation (Sklar *et al., J. Cell Biochem*., Abst. W462, 18D, 540 (1994)). See also WO 94/26298 published November 24, 1994.

Holmes *et at., supra*, found that HRG exerted a mitogenic effect on mammary cell lines (such as SK-BR-3 and MCF-7). The mitogenic activity of GGFs on Schwann cells has also been reported. See, e.g., Brockes *et al., J. Biol. Chem*. 255(18):8374-8377 (1980); Lemke and Brockes, *J. Neurosci*. 4:75-83 (1984 Lemke and Brockes, *J. Neurosci*. 4:75-83 (1984); Brockes *et al., Ann. Neurol*. 20(3):317-322 (1986); Brockes, J., *Methods in Enzym*., 147: 217-225 (1987) and Marchionni *et al., supra*. Schwann cells constitute important glial cells which provide myelin sheathing around the axons of neurons, thereby forming individual nerve fibers. Thus, it is apparent that Schwann cells play an important role in the development, function and regeneration of peripheral nerves. The implications of this from a therapeutic standpoint have been addressed by Levi *et al*., *J*. *Neuroscience* 14(3):1309-1319 (1994). Levi *et al*. discuss the potential for construction of a cellular prosthesis comprising human Schwann cells which could be transplanted into areas of damaged spinal cord. Methods for culturing Schwann cells *ex vivo* have been described. See WO 94/00140 and Li *et al*., *J*. *Neuroscience* 16(6):2012-2019 (1996).

Pinkas-Kramarski *et al*. found that NDF seems to be expressed in neurons and glial cells in embryonic and adult rat brain and primary cultures of rat brain cells, and suggested that it may act as a survival and maturation factor for astrocytes (Pinkas-Kramarski *et al*., *PNAS*, *USA* 91:9387-9391 (1994)). Meyer and Birchmeier, *PNAS*, *USA* 91:1064-1068 (1994) analyzed expression of heregulin during mouse embryogenesis and in the perinatal animal using in situ hybridization and RNase protection experiments. See also Meyer *et al*., *Development* 124(18):3575-3586 (1997). These authors conclude that, based on expression of this molecule, heregulin plays a role *in vivo* as a mesenchymal and neuronal factor. Similarly, Danilenko *et al*., Abstract 3101, *FASEB* 8(4-5):A535 (1994); Danilenko et al., *Journal of Clinical Investigation* 95(2): 842-851 (1995), found that the interaction of NDF and the HER2 receptor is important in directing epidermal migration and differentiation during wound repair.

Ram *et al*., *Journal of Cellular Physiology* 163:589-596 (1995) evaluated the mitogenic activity of NDF on the immortalized human mammary epithelial cell line MCF-10A. Danilenko *et al*., *J. Clin*. *Invest*. 95:842-851 (1995) investigated whether NDF would influence epidermal migration in an *in vivo* model of excisional deep partial-thickness wound repair. It is reported that there were no statistically significant differences in proliferating basal and superbasal keratinocytes in control wounds vs. wounds treated with rhNDF-α₂. Marikovsky *et al*., *Oncogene* 10:1403-1411 (1995), studied the proliferative responses of an aneuploid BALB/MK continuous keratinocyte cell line and evaluated the effects of α- and β-isoforms of NDF on epidermal keratinocytes.

The relationship between the structure and function of new proteins can be investigated using any of a variety of available mutational analysis techniques. Examples of such techniques include alanine scanning mutagenesis and phagemid display. Alanine scanning can be used to identify active residues (i.e., residues that have a significant effect on protein function) in a protein or protein domain. For example, Cunningham and Wells used alanine scanning to identify residues in human growth hormone that were important for binding its receptor. Cunningham and Wells, *Science* **244**:1081-1085 (1989). In alanine scanning, a gene encoding the protein or domain to be scanned is inserted into an expression vector, and mutagenesis is carried out to generate a series of vectors that encode proteins or domains in which sequential residues are converted to alanine. The encoded proteins or domain are expressed from these vectors, and the activities of the alanine-substituted variants are then tested to identify those with altered activity. An alteration in activity indicates that the residue at the alanine-substituted position is an active residue.

Phagemid display was developed to allow the screening of a large number of variant polypeptides for a particular binding activity. Smith and Parmley demonstrated that foreign peptides can be "displayed" efficiently on the surface of filamentous phage by inserting short gene fragments into gene III of the fd phage. Smith, *Science* **228**:1315-1317 (1985); Parmley and Smith, *Gene* **73**:305-318 (1985). The gene III coat protein is present in about five copies at one end of the phage particle. The modified phage were termed "fusion phage" because they displayed the foreign peptides fused to the gene III coat protein. As each fusion phage particle displayed approximately five copies of the fusion protein, this mode of phage display was termed "polyvalent display."

Scott *et al*. and Cwirla *et al*. showed that fusion phage libraries could be screened by sequential affinity selections known as "panning." *Scott et al*., *Science* **249**:386-390 (1990); Cwirla *et al*., *PNAS USA* 87:6378-6382 (1990). However, early efforts to select high affinity fusion phage failed, presumably due to the polyvalence of the phage particles. This problem was solved with the development of a "monovalent" phage display system in which the fusion protein is expressed at a low level from a phagemid and a helper phage provides a large excess of wild-type coat protein. Bass *et al*., *Proteins* **8**:309-314 (1990); Lowman *et al*., *Biochem.* **30**:10832-10838 (1991). Monovalent phage display can be used to generate and screen a large number of variant polypeptides to isolate those that bind with high affinity to a target of interest.

Approximately 50,000 infants are born in the United States every year with birth weights, less than 1.5 kg. About two thirds of these very low birth weight infants have evidence of pulmonary immaturity manifested as respiratory distress shortly after birth. The majority of these infants require mechanical ventilation. Respiratory distress syndrome, caused by insufficient pulmonary surfactant production, as well as structural immaturity of the lung, is responsible for respiratory difficulties observed in these prematurely born neonates. Well developed alveoli are necessary to provide efficient oxygen transfer from the air-liquid interface of the lung to the systemic circulation. Surfactant proteins are critical in reducing the alveolar surface tension at low lung volumes and preventing alveolar collapse.

A need continues to exist for a method of treatment for respiratory distress syndrome and other diseases associated with immature lung development and low lung surfactant production.

### SUMMARY OF THE INVENTION

In general an object of the invention is to provide a method of inducing epithelial cell growth and development for the purpose of promoting repair and healing of tissue damage or injury.

Accordingly, one object of this invention is to provide a method of treating respiratory distress syndrome in patients, primarily human patients, in need of such treatment. A further object is to provide a method of inducing lung epithelial cell growth and development. A further object is to provide a method of increasing lung surfactant protein A production in the lung of persons with impaired oxygen transfer in the lung alveoli. This invention is useful in treating infants/neonates with respiratory distress as well as youth and adults with poor lung function due to lung injury or damage.

It has now been discovered that these objects and the broader objective of treating conditions associated with epithelial cell damage and injury are achieved by administering to a patient in need of such treatment an effective amount of a heregulin ligand, preferably a polypeptide or fragment thereof. These heregulin (HRG) polypeptides, include HRG-α, HRG-β1, HRG-β2, HRG-β3 and other HRG polypeptides which cross-react with antibodies directed against these family members and/or which are substantially homologous as defined below and includes HRG variants such as N-terminal and C-terminal fragments thereof. A preferred HRG is the ligand disclosed in Fig. 1A - 1D and further designated HRG-α. Other preferred HRGs are the ligands disclosed in Figure 2A - 2E, and designated HRG-β1; disclosed in Figure 3A - 3E designated HRG-β2; and disclosed in Figure 4A - 4C designated HRG-β3.

Accordingly, the invention provides the uses and methods set out in the appended claims.

In one embodiment, the invention makes use of HRG agonist antibodies. In this embodiment, HER2/HER3 or fragments thereof (which also may be synthesized by *in vitro* methods) are fused (by recombinant expression or an *in vitro* peptidyl bond) to an immunogenic polypeptide and this fusion polypeptide, in turn, is used to raise antibodies against a HER2/HER3 epitope. Agonist antibodies are recovered from the serum of immunized animals. Alternatively, monoclonal antibodies are prepared from *in vitro* cells or *in vivo* immunized animals in conventional fashion. If desired, the agonist antibodies may be obtained by phage display selection from a phage library of antibodies or antibody fragments. Preferred antibodies identified by routine screening will bind to the receptor, but will not substantially cross-react with any other known ligands such as EGF, and will activate the HER receptors HER2, HER3 and/or HER4. In addition, antibodies may be selected that are capable of binding specifically to individual family members of HRG family, e.g. HRG-α, HRG-β1, HRG-β2, HRG-β3, and which are agonists thereof.

In general, the invention relates to regenerating and/or repairing epithelial cell injury by stimulating growth and proliferation of epithelial cells, in particular ductal and ciliated epithelial cells. The epithelial cells may be injured by many types of insults, for example, injury due to surgical incision or resection, chemical or smoke inhalation or aspiration, chemical or biochemical ulceration, cell damage due to viral or bacterial infection, etc. The epithelial cells which may be affected include any epithelial cell which expresses HER2, HER3 and/or HER4; suitable cells are located, for example, in the lung, gastric mucosa, endometrium, oviducts, mammary glands, pancreas, salivary gland, etc. The medicament prepared in accordance with the invention stimulates growth and proliferation of the epithelial cells, repairing and reestablishing the cellular barriers of organs and allowing the affected tissues to develop normal physiological functions more quickly. For example, lung epithelial cells are damaged by inhalation of smoke resulting in emphysema. Treatment of the lung cells by the medicament prepared in accordance with the invention regenerates the barrier layer of lung epithelial cells, improves oxygenation and speeds the development of a barrier to infection. Similarly, cell damage due to aspiration of gastric acid can be treated by the medicament prepared in accordance with the invention to facilitate regeneration of epithelial cells.

### BRIEF DESCRIPTION OF THE DRAWINGS

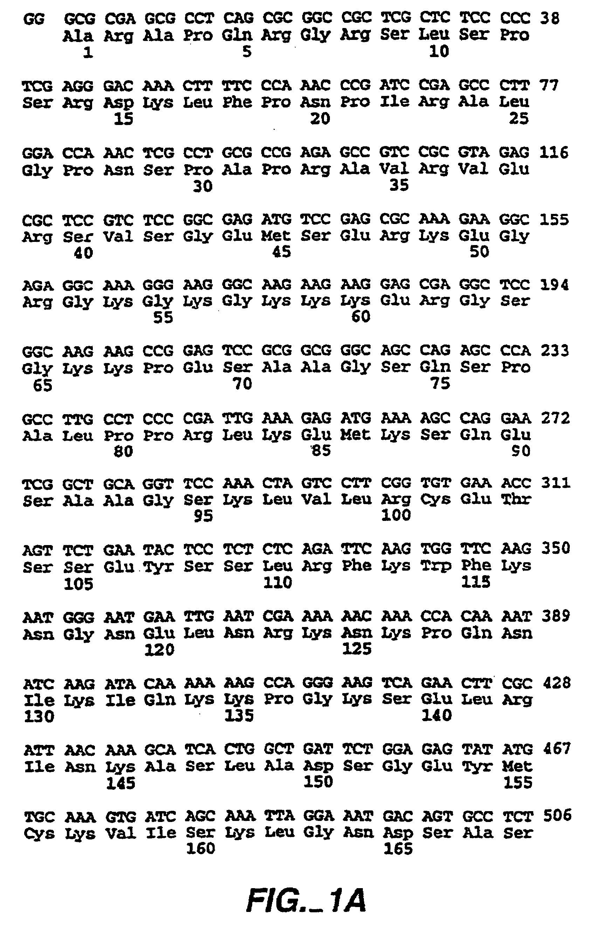

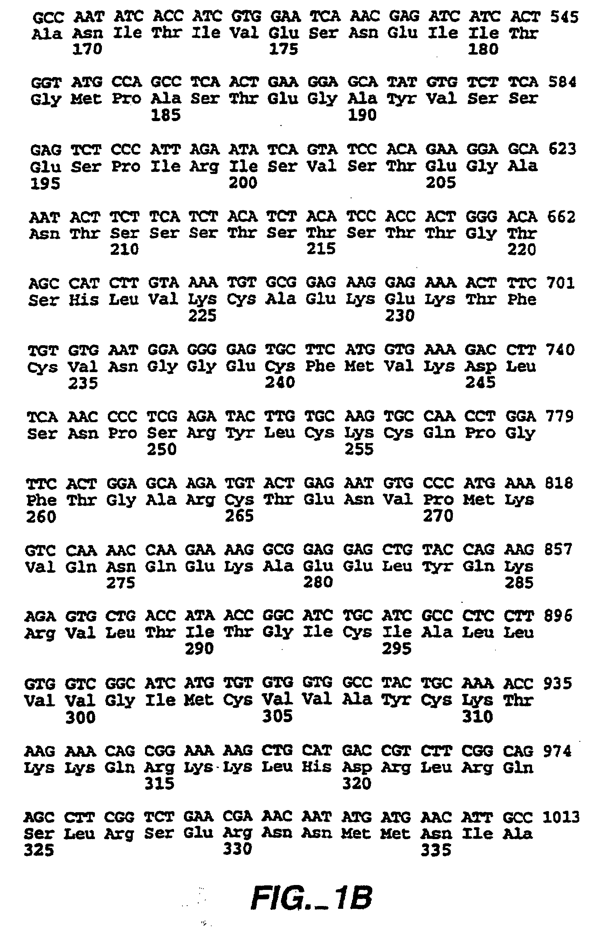

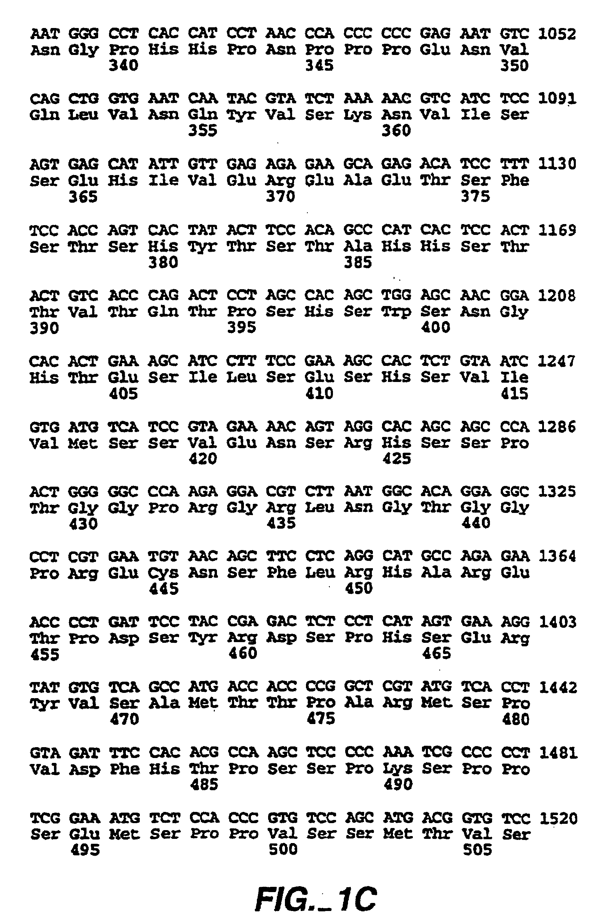

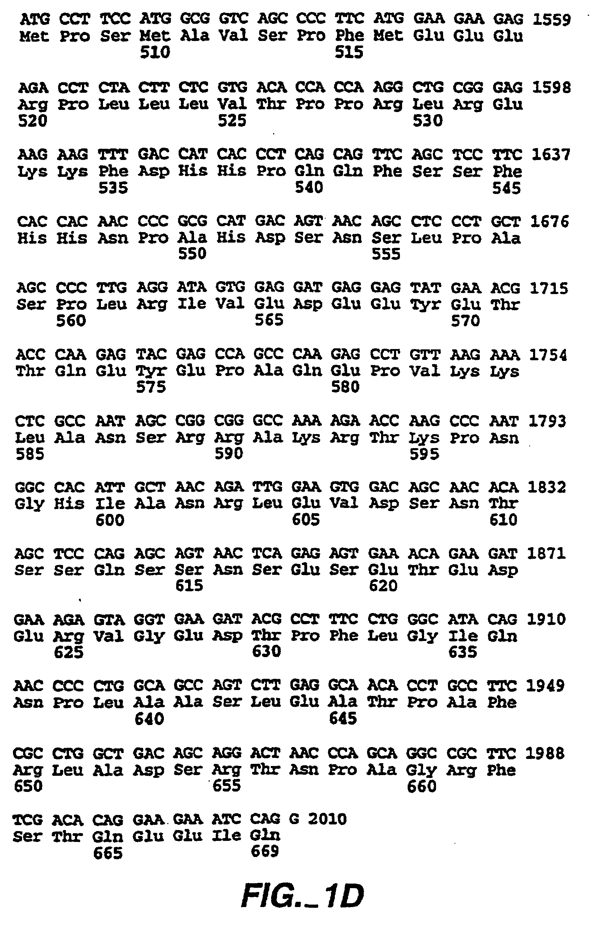

Figure 1A - 1D show the deduced amino acid sequence (SEQ ID NO:1) for the cDNA sequence (SEQ ID NO:2) contained in a clone obtained according to U.S. 5,367,060. The initiating methionine (Met) of HRG-α is at position 45.

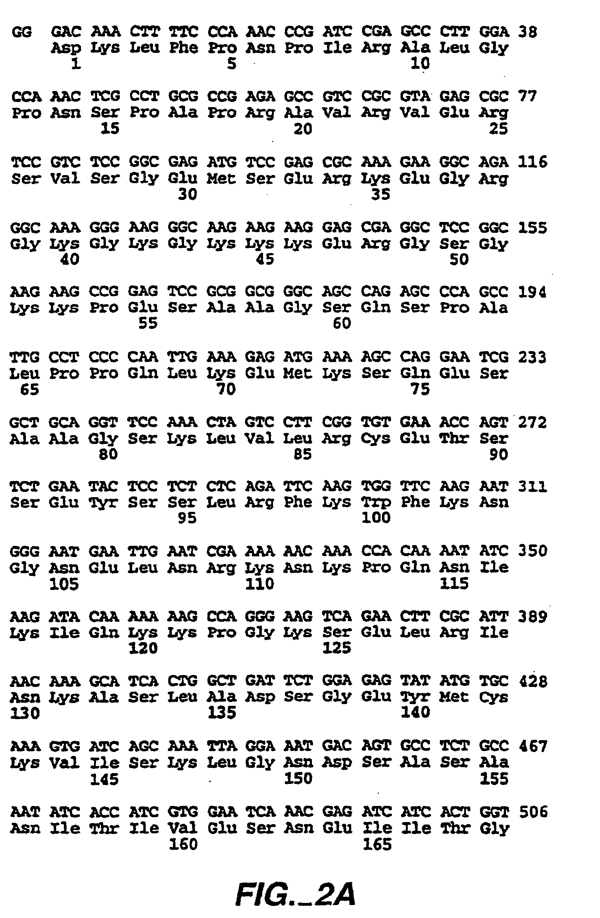

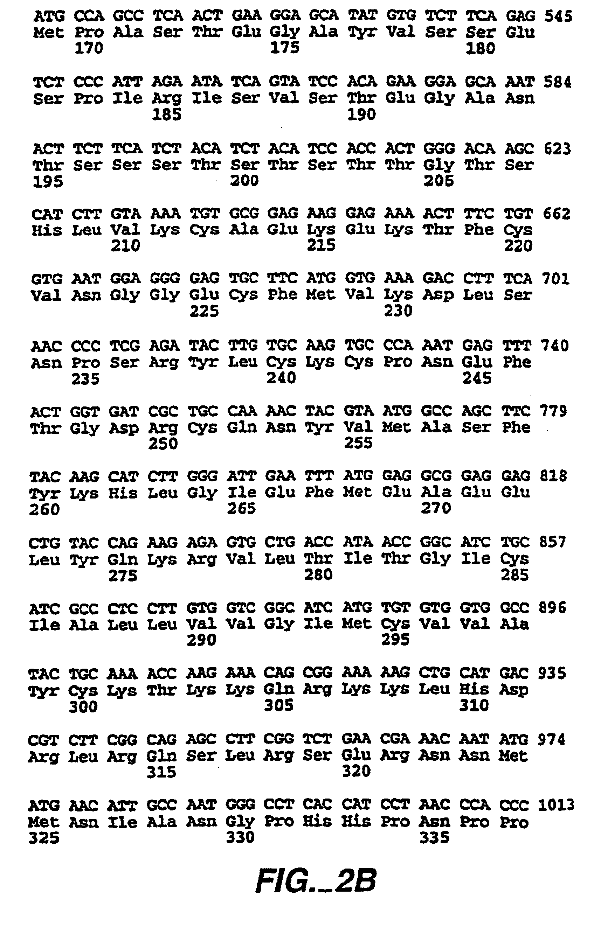

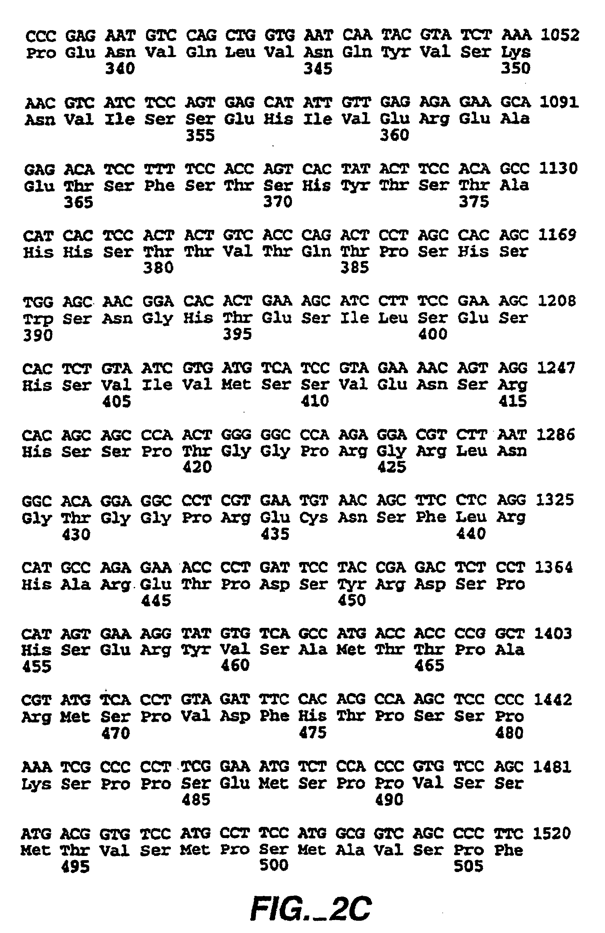

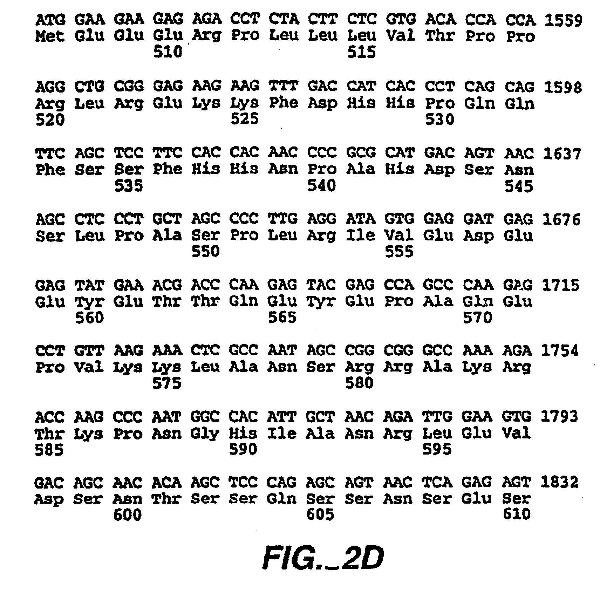

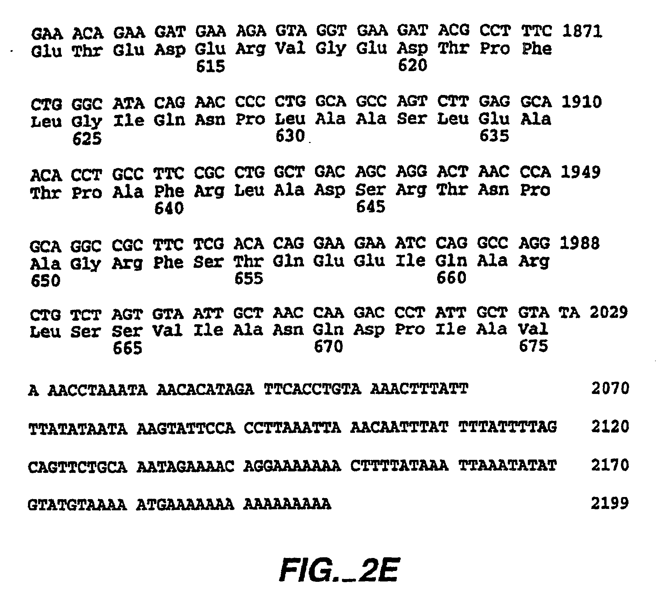

Figure 2A - 2E show the deduced amino acid sequence (SEQ ID NO:3) and cDNA sequence (SEQ ID NO:4) of a potential coding sequence of a clone obtained according to U.S. 5,367,060 for HRG-β1. The initiating Met is at M31.

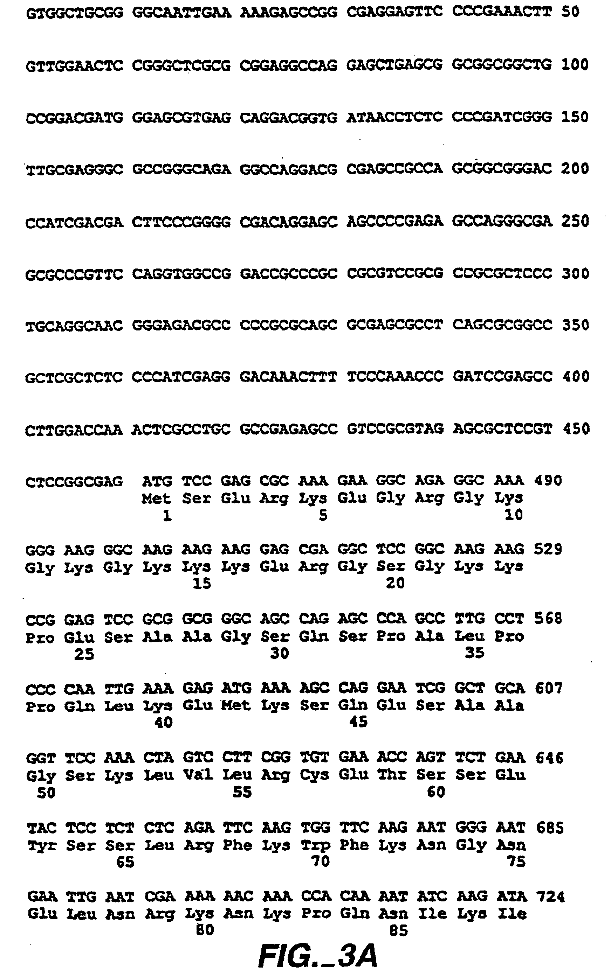

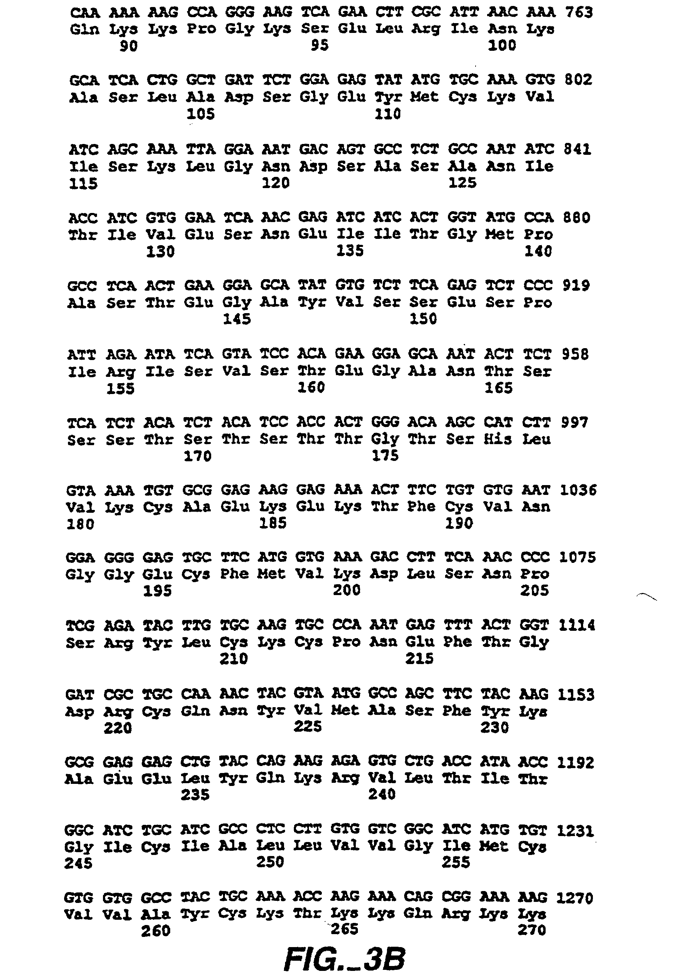

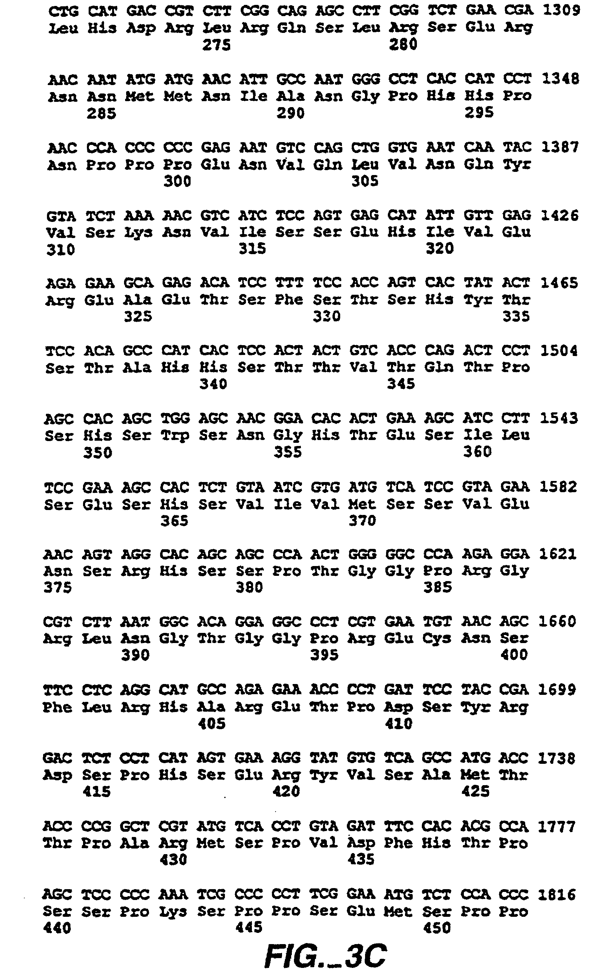

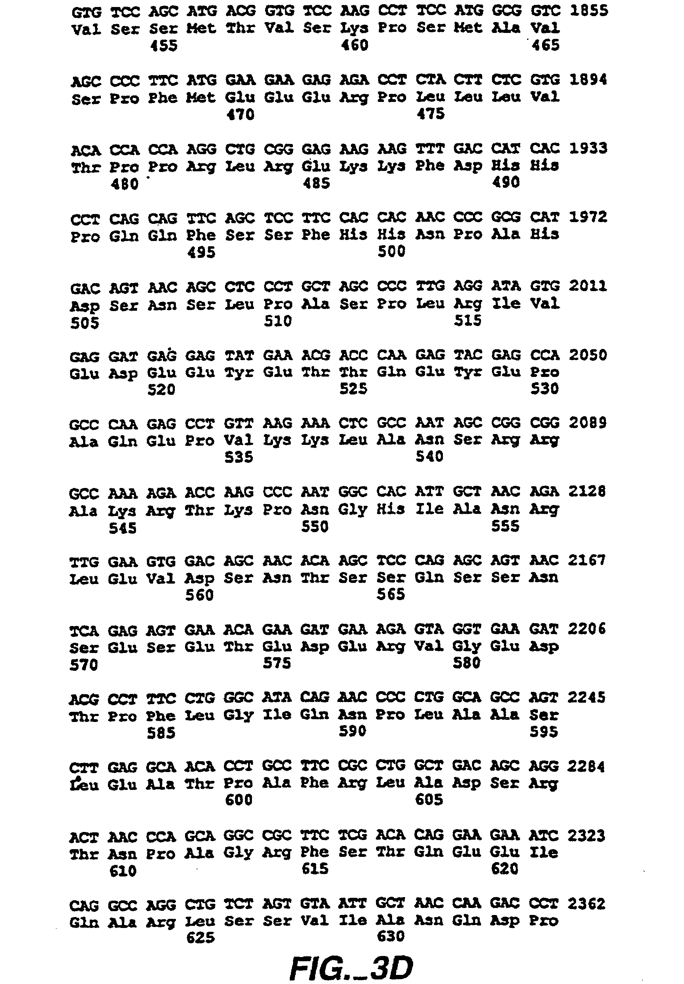

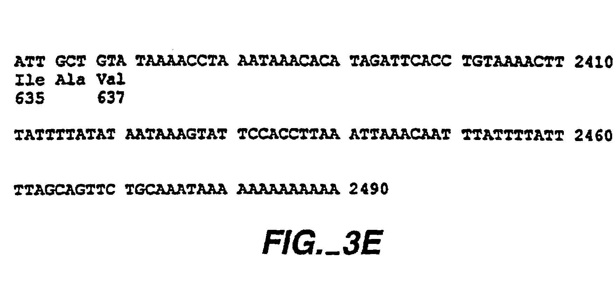

Figure 3A - 3E show the deduced amino acid sequence (SEQ ID NO:5) and cDNA sequence (SEQ ID NO:6) of a nucleotide sequence of a clone obtained according to U.S. 5,367,060 for HRG-β2.

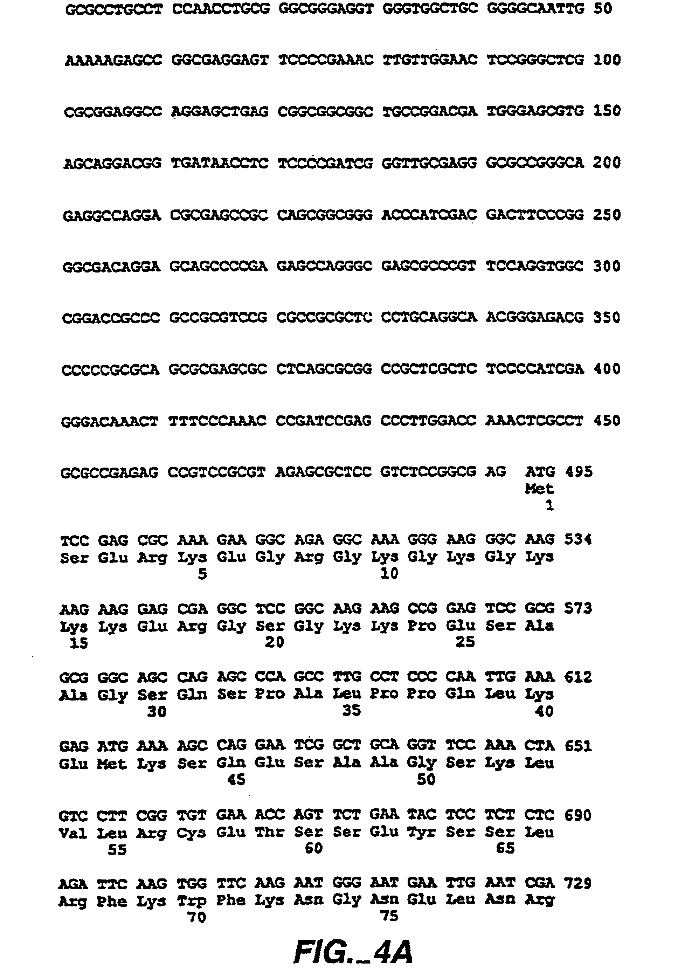

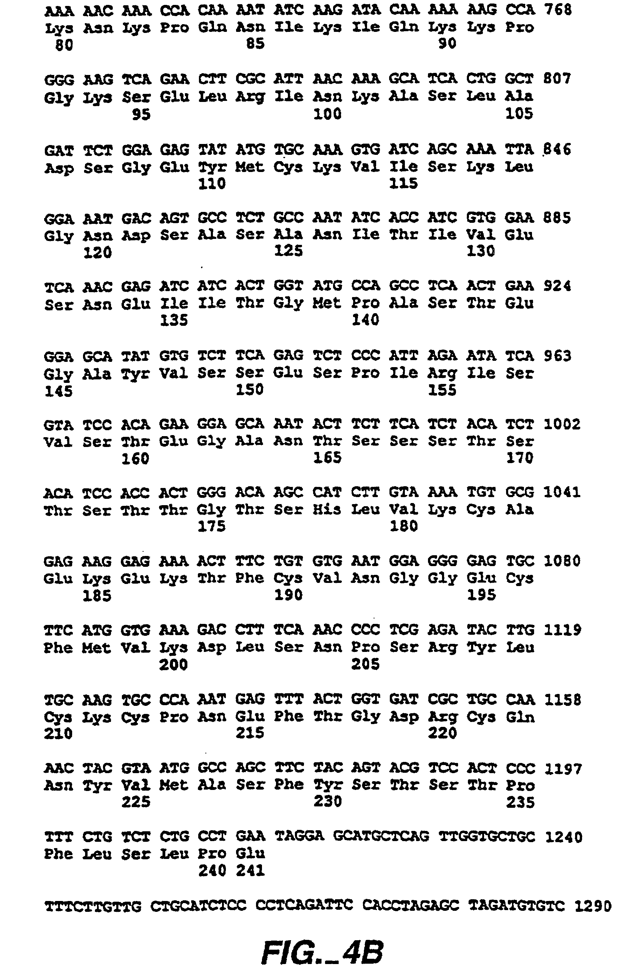

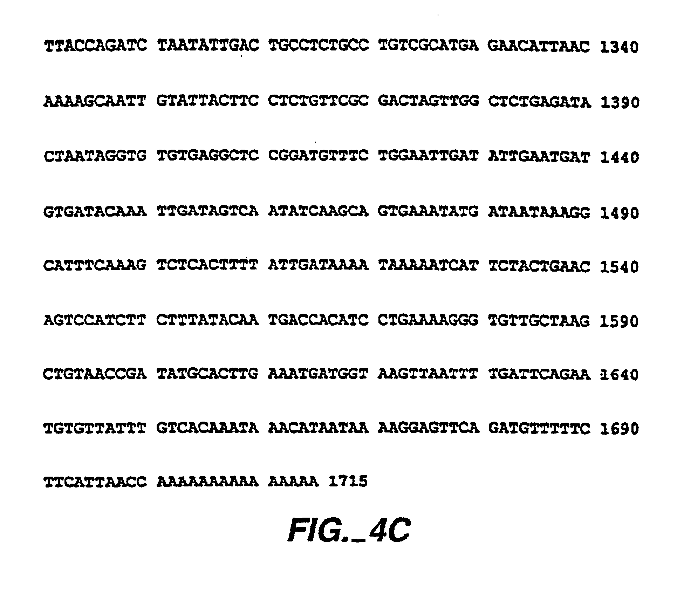

Figure 4A - 4C show the deduced amino acid sequence (SEQ ID NO:7) and cDNA sequence (SEQ ID NO:8) of a nucleotide sequence of a clone obtained according to U.S. 5,367,060 for HRG-β3.

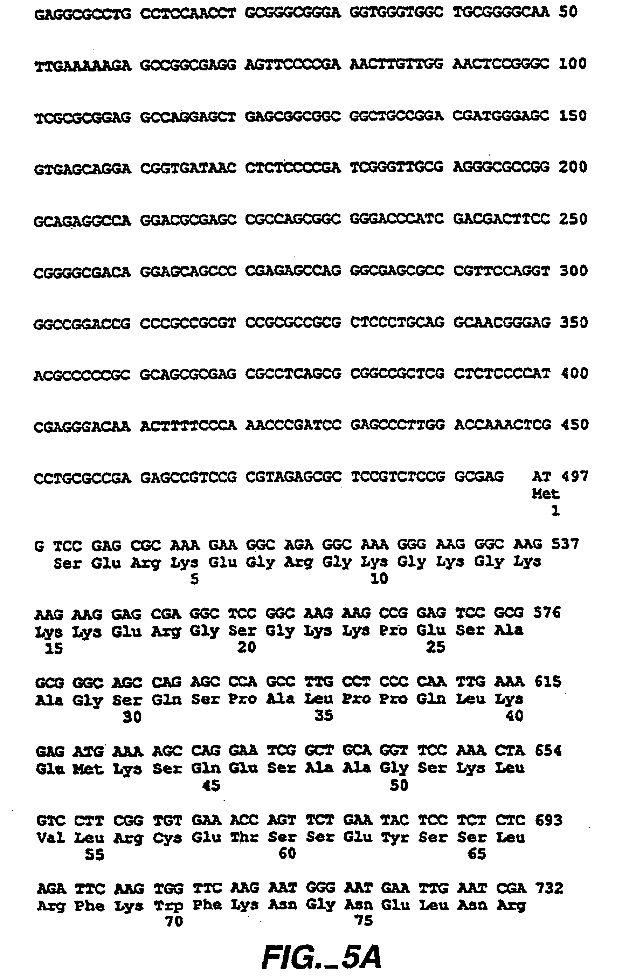

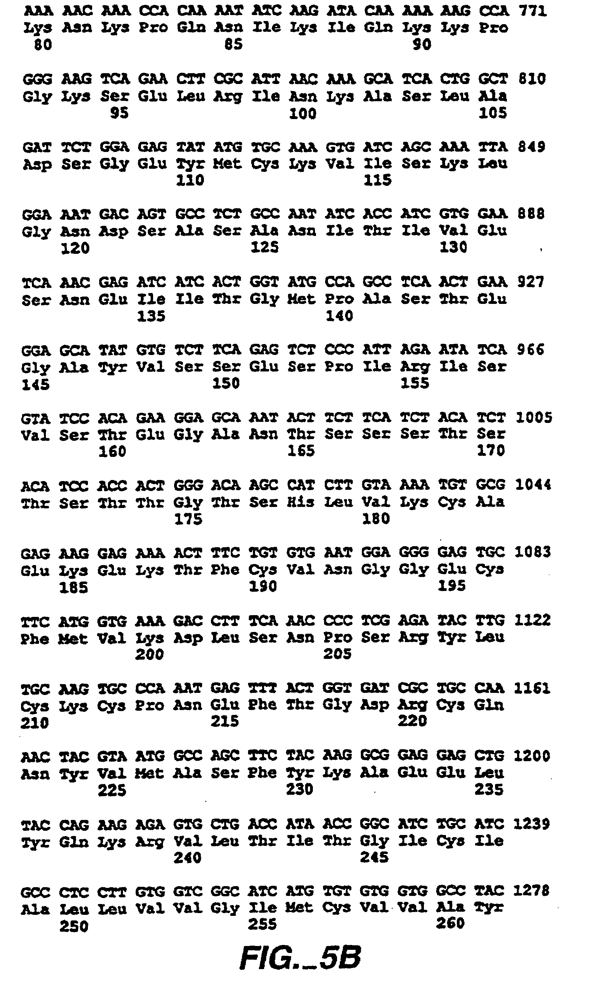

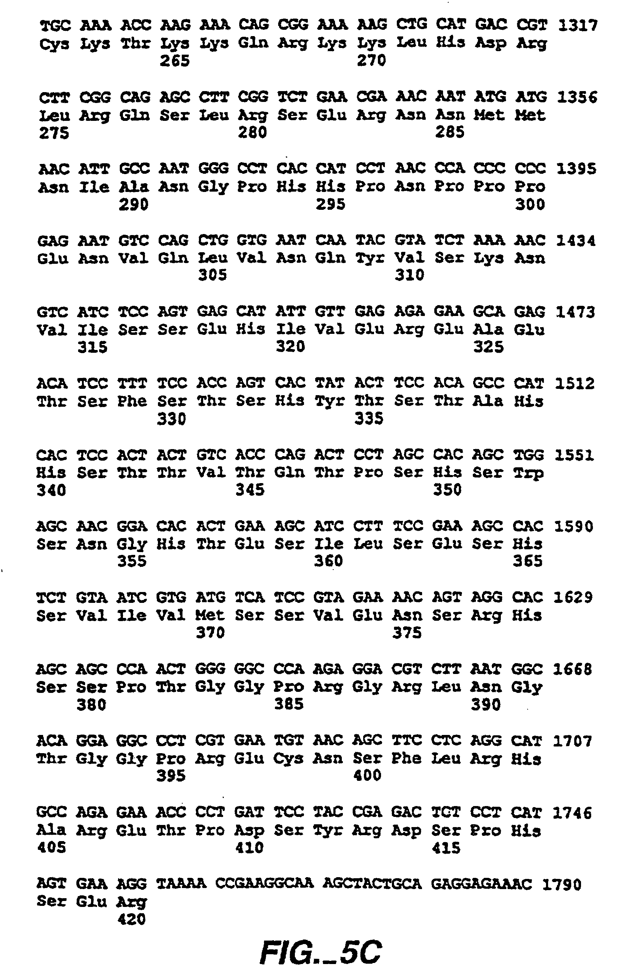

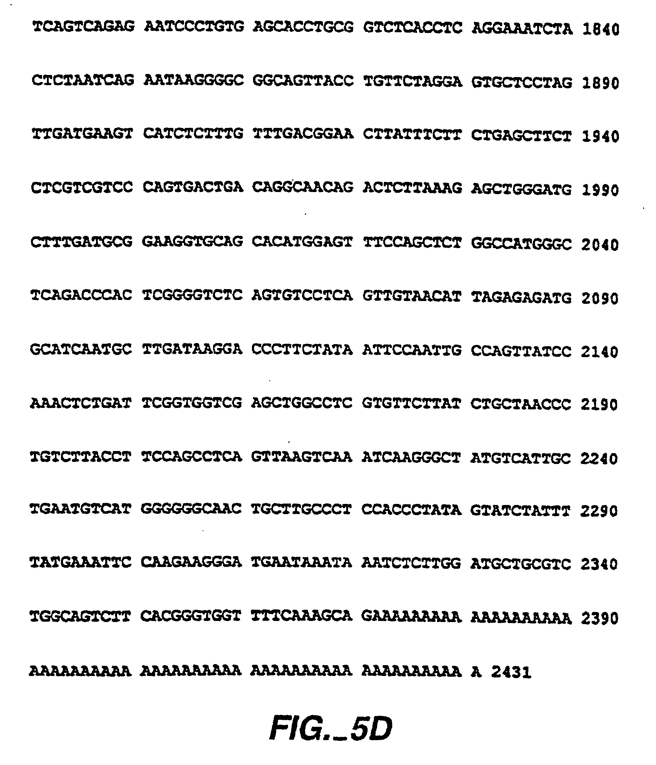

Figure 5A - 5D show the deduced amino acid sequence (SEQ ID NO:9) and cDNA sequence (SEQ ID NO:10) of a nucleotide sequence of a clone obtained according to U.S. 5,367,060 for HRG-β2-like protein.

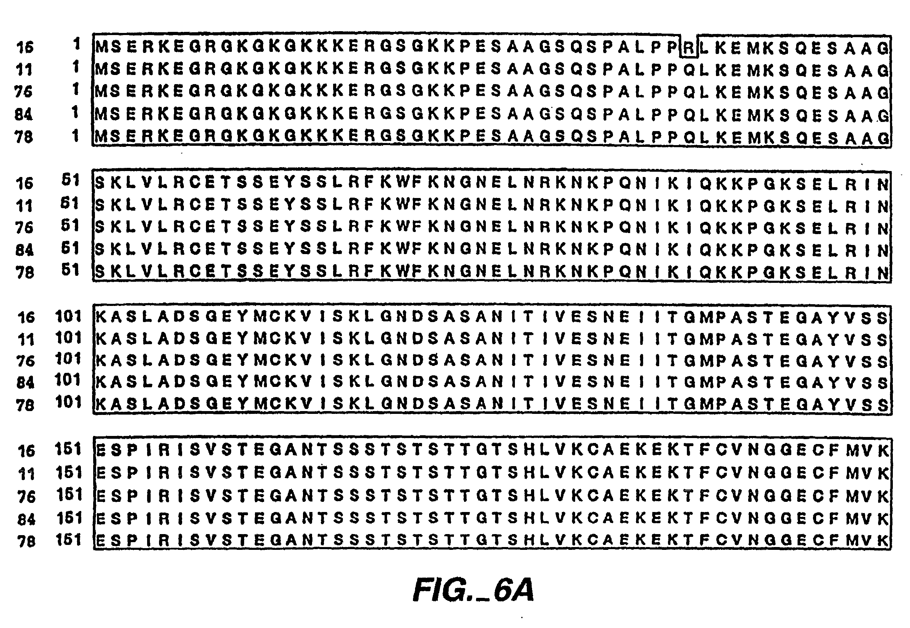

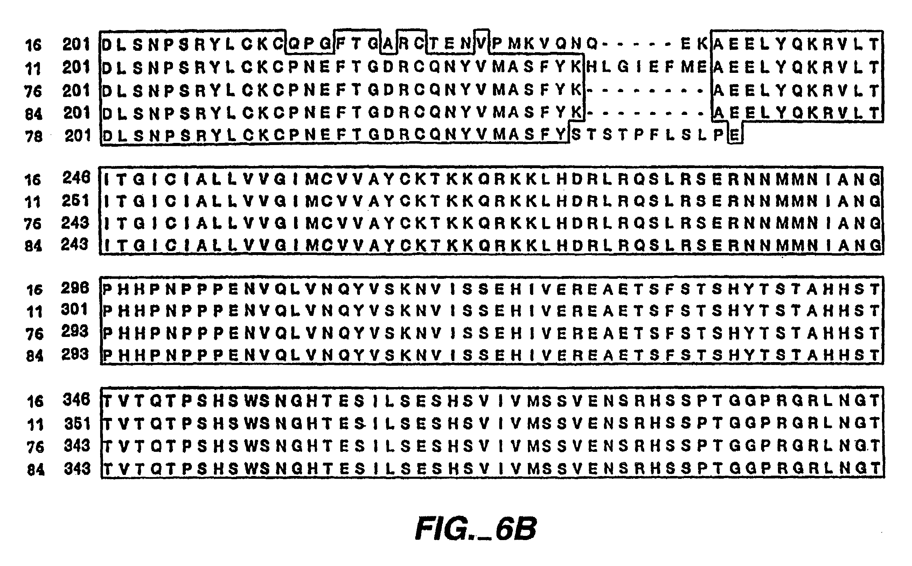

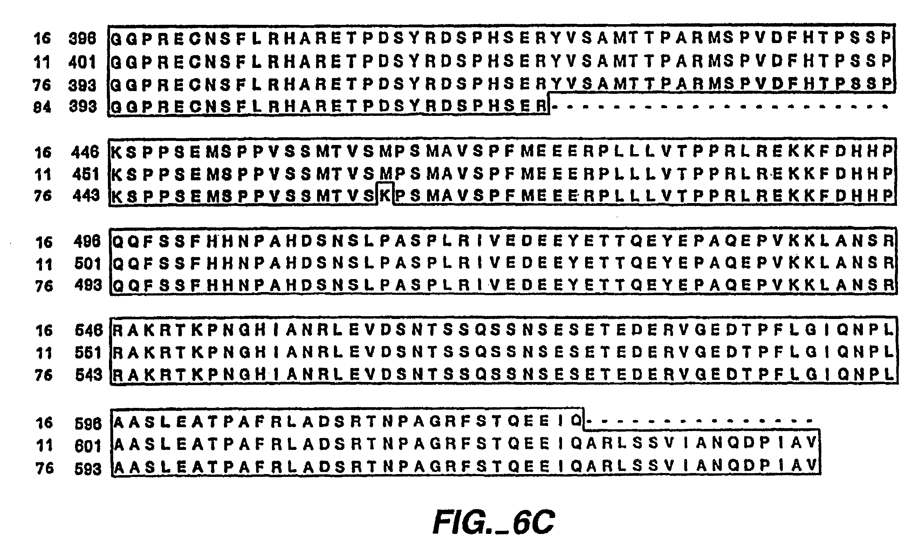

Figure 6A - 6C show a comparison of the amino acid homologies of several known heregulins α, β1, β2, β2-like and β3 in descending order and illustrates the amino acid insertions, deletions, and substitutions that characterize these forms of HRG (SEQ ID NOS: 1, 3, 5, 9, and 7).

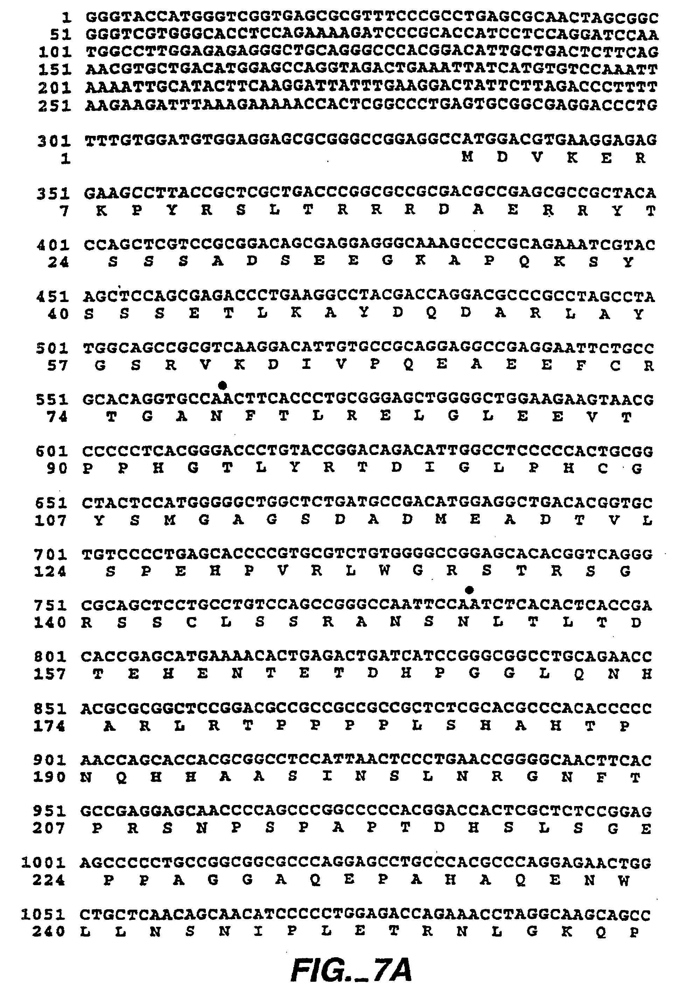

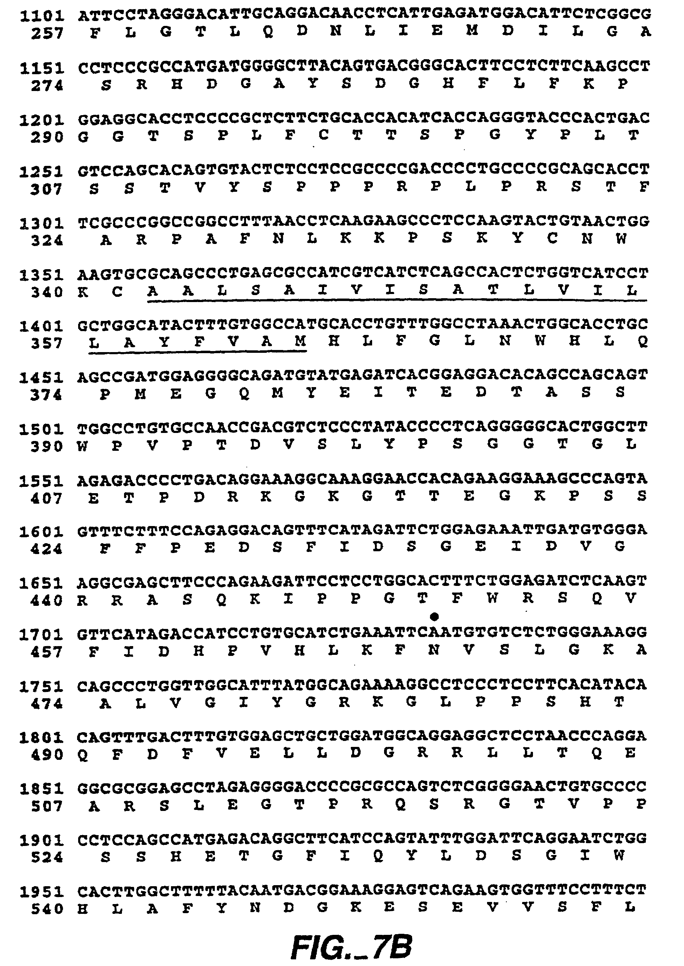

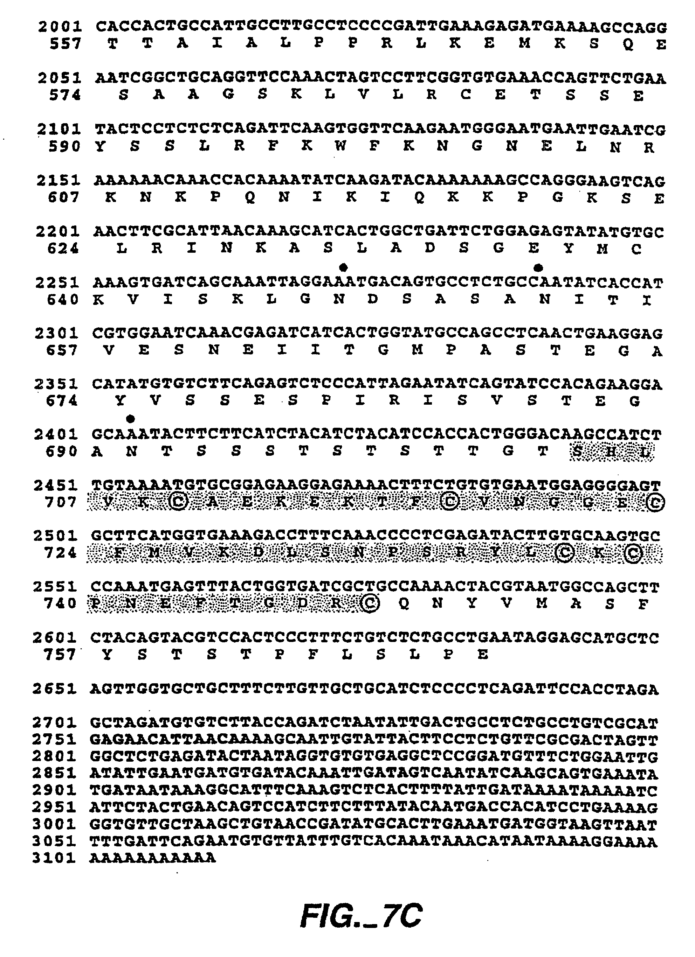

Figure 7A - 7C show the deduced amino acid sequence (SEQ ID NO:11) and cDNA sequence (SEQ ID NO:12) of γ-HRG obtained as described in U.S. Serial No. 08/891,845. The hydrophobic region is underlined. The EGF-like domain is shaded, cysteine residues in the EGF-like domain are circled. N-linked glycosylation sites are marked above the nucleic acid sequence with a ( ).

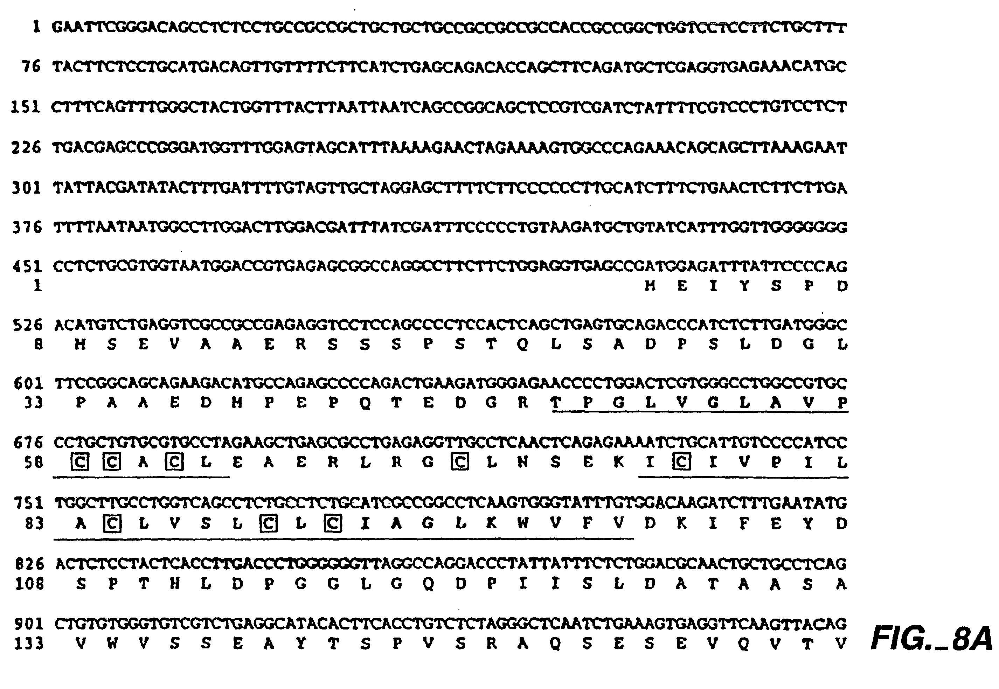

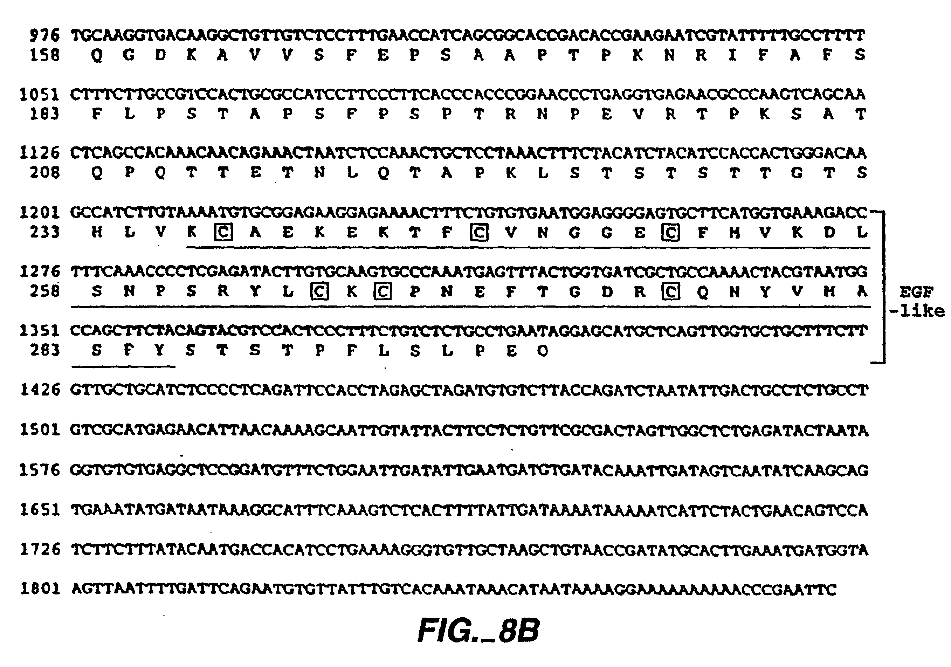

Figure 8 shows the cDNA sequence (SEQ ID NO:13) and amino acid sequence (SEQ ID NO:14) of SMDF obtained as described in U.S. Serial No. 08/339,517. An EGF-like domain and the apolar and uncharged domains (*i.e*. "apolar I" consisting of residues from about 48-62 and "apolar II" consisting of residues from about 76-100) are underlined. Cysteines in the EGF-like domain and in the "cysteine knot" in the unique N-terminal domain ("NTD-cys knot") are boxed. The stop codon is denoted by the letter "O".

### DETAILED DESCRIPTION OF THE PREFERRED EMBODIMENTS

HRG ligands, in particular polypeptides and agonist antibodies thereof, have affinity for and stimulate the HER2, HER3 and/or HER4 receptors or combinations thereof in autophosphorylation. Included within the definition of HRG ligands, in addition to HRG-α, HRG-β1, HRG-β2, HRG-β3 and HRG-β2-like, are other polypeptides binding to the HER2, HER3 and/or HER4 receptor, which bear substantial amino acid sequence homology to HRG-α or HRG-β1. Such additional polypeptides fall within the definition of HRG as a family of polypeptide ligands that bind to the HER2, HER3 and/or HER4 receptors.

Heregulin polypeptides bind with varying affinities to the HER2. HER3 and/or HER4 receptors. Generally, the HER3 and HER4 receptors are bound with high affinity. It is also known that heterodimerization of HER2 with HER3 and of HER2 with HER4 occurs with subsequent receptor cross-phosphorylation as described above. In the present invention, epithelial cell growth and/or proliferation is induced when a heregulin protein interacts and binds with an individual receptor molecule or a receptor dimer such that receptor phosphorylation is induced. Binding and activation of HER2, HER3, HER4 or combinations thereof, therefore, is meant to include activation of any form of the receptor necessary for receptor activation and biologic function including monomeric receptor and dimeric receptor forms. Dimeric receptor forms may be referred to below, for example, as HER2/HER3, HER2/HER4, and HER3/HER4.

### I. DEFINITIONS

In general, the following words or phrases have the indicated definition when used in the description, examples, and claims.

"Heregulin" (HRG) ligand is defined herein to be any isolated ligand, preferably a polypeptide sequence which possesses a biological property of a naturally occurring HRG polypeptide. Ligands within the scope of this invention include the NDF, ARIA and GGF growth factor heregulin proteins identified above as well as the SMDF and HRG polypeptides discussed in detail herein. These isolated NDF, ARIA and GGF heregulin polypeptides are well known in the art. HRG includes the polypeptides shown in Figs. 1A-1D, 2A-2E, 3A-3E, 4A-4C, 5A-5D, 6A-6C, 7A-7C and 8 and mammalian analogues thereof. Included are HRG variants such as the γ-HRG described in WO 98/02541, published 22 January 1998; the variants described in WO 98/35036, published 13 August 1998; and the SMDF variants described in US Patent NO. 5,770,567 granted 23 June 1998 (WO 96/15244). These applications are incorporated herein in their entirety. These variants can be prepared by the methods described below, optionally together with alanine scanning and phage display techniques known in the art. Cunningham and Wells, Science 244:1081-85 (1989); Bass et al., Proteins 8:309-14 (1990); Lowman et al., Biochem. 30:10832-38 (1991).

The term a "normal" epithelial cell means an epithelial cell which is not transformed, i.e., is non-cancerous and/or non-immortalized. Further, the normal epithelial cell is preferably not aneuploid. Aneuploidy exists when the nucleus of a cell does not contain an exact multiple of the haploid number of chromosomes, one or more chromosomes being present in greater or lesser number than the rest. Typical properties of transformed cells which fall outside the scope of this invention include the ability to form tumors when implanted into immune-deprived mice (nude mice), the ability to grow in suspension or in semi-solid media such as agar, a loss of contact inhibition allowing piling up of cells into colonies or foci, a loss of dependence on growth factors or serum, cell death if cells are inhibited from growing, and disorganization of actin filaments. Specifically included within the invention are normal epithelial cells which will not form tumors in mice, grow attached to plastic or glass (are anchorage dependent), exhibit contact inhibition, require serum-containing hormones and growth factors, remain viable if growth is arrested by lack of serum, and contain well-organized actin filaments. Although the normal epithelial cells are preferably not cultured cells, also suitable for the invention are non-transformed, non-immortalized epithelial cells isolated from mammalian tissue. These isolated cells may be cultured for several generations (up to about 10 or even 50 generations) in the presence of a heregulin in order to induce growth and/or proliferation of the isolated epithelial cell sample, that is, to expand the sample. The expanded sample can then be reintroduced into the mammal for the purpose of repopulating the epithelial cell tissue (reepithelialization). This is particularly useful for repairing tissue injury or damage.

An "epithelial" cell is a cell located in a cellular, avascular layer covering the free surface (cutaneous, mucous or serous) of an organ or lining a tube or cavity of an animal body. Lung epithelial cells include bronchial epithelial cells, Type II cells and Clara cells. The term "epithelial cell" as used herein is consistent with the art recognized definition of epithelial cells in epithelium. See, for example, the definition in Taber's Encyclopedic Medical Dictionary, Edition 12, (1973) F.A. Davis Company, publisher.

"Biological property" for the purposes herein means an *in vivo* biologic or antigenic function or activity that is directly or indirectly performed by an HRG sequence (whether in its native or denatured conformation), or by any subsequence thereof. Biologic functions include receptor binding, any enzyme activity or enzyme modulatory activity, any carrier binding activity, any hormonal activity, any activity in promoting or inhibiting adhesion of cells to extracellular matrix or cell surface molecules, or any structural role. However, biologic functions do not include antigenic functions, i.e. possession of an epitope or antigenic site that is capable of cross-reacting with antibodies raised against a naturally occurring HRG polypeptide.

"Biologically active" HRG is defined herein as a polypeptide sharing a biologic function of an HRG sequence which may (but need not) in addition possess an antigenic function. A principal known effect or function of HRG is as a ligand polypeptide having a qualitative biological activity of binding to HER2, HER3 and/or HER4 resulting in the activation of the receptor tyrosine kinase (an "activating ligand"). One test for activating ligands is the HRG tyrosine autophosphorylation assay described below. Included within the scope of HRG as that term is used herein are HRG having translated mature amino acid sequences of the complete human HRG as set forth herein; deglycosylated or unglycosylated derivatives of HRG, amino acid sequence variants of HRG sequence, and derivatives of HRG, which are capable of exhibiting a biological property in common with HRG. While native HRG is a membrane-bound polypeptide, soluble forms, such as those forms lacking a functional transmembrane domain, are also included within this definition. In particular, included are polypeptide fragments of HRG sequence which have an N-terminus at any residue from about S216 to about A227, and its C-terminus at any residue from about K268 to about R286, and the homologous sequences shown in Fig. 6A-C, hereinafter referred to collectively for all HRGs as the growth factor domain (GFD).

"Antigenically active" HRG is defined as a polypeptide that possesses an antigenic function of an HRG and which may (but need not) in addition possess a biologic function.

In preferred embodiments, antigenically active HRG is a polypeptide that binds with an affinity of at least about 10⁻⁹ l/mole to an antibody raised against a naturally occurring HRG sequence. Ordinarily the polypeptide binds with an affinity of at least about 10⁻⁸ l/mole. Most preferably, the antigenically active HRG is a polypeptide that binds to an antibody raised against one of HRGs in its native conformation. HRG in its native conformation generally is HRG as found in nature which has not been denatured by chaotropic agents, heat or other treatment that substantially modifies the three dimensional structure of HRG as determined, for example, by migration on nonreducing, nondenaturing sizing gels. Antibody used in this determination may be rabbit polyclonal antibody raised by formulating native HRG from a non rabbit species in Freund's complete adjuvant, subcutaneously injecting the formulation, and boosting the immune response by intraperitoneal injection of the formulation until the titer of anti-HRG antibody plateaus.

Ordinarily, biologically or antigenically active HRG will have an amino acid sequence having at least 75% amino acid sequence identity with a given HRG sequence, more preferably at least 80%, even more preferably at least 90%, and most preferably at least 95%. Identity or homology with respect to an HRG sequence is defined herein as the percentage of amino acid residues in the candidate sequence that are identical with HRG residues in the HRG of Fig. 6A-6C, after aligning the sequences and introducing gaps, if necessary, to achieve the maximum percent homology, and not considering any conservative substitutions as part of the sequence identity. None of N-terminal, C-terminal or internal extensions, deletions, or insertions into HRG sequence shall be construed as affecting homology.

Thus, the biologically active and antigenically active HRG polypeptides that are the subject of this invention include each entire HRG sequence; fragments thereof having a consecutive sequence of at least 5, 10, 15, 20, 25, 30 or 40 amino acid residues from HRG sequence; amino acid sequence variants of HRG sequence wherein an amino acid residue has been inserted N- or C-terminal to, or within, HRG sequence or its fragment as defined above; amino acid sequence variants of HRG sequence or its fragment as defined above has been substituted by another residue. HRG polypeptides include those containing predetermined mutations by, e.g., site-directed or PCR mutagenesis, and other animal species of HRG polypeptides such as rabbit, rat, porcine, non-human primate, equine, murine, and ovine HRG and alleles or other naturally occurring variants of the foregoing and human sequences; derivatives of HRG or its fragments as defined above wherein HRG or its fragments have been covalently modified by substitution, chemical, enzymatic, or other appropriate means with a moiety other than a naturally occurring amino acid (for example a detectable moiety such as an enzyme or radioisotope); glycosylation variants of HRG (insertion of a glycosylation site or deletion of any glycosylation site by deletion, insertion or substitution of appropriate amino acid); and soluble forms of HRG, such as HRG-GFD or those that lack a functional transmembrane domain.

"Isolated" means a ligand, such as HRG, which has been identified and separated and/or recovered from a component of its natural environment. Contaminant components of its natural environment are materials which would interfere with diagnostic or therapeutic uses for HRG, and may include proteins, hormones, and other substances. In preferred embodiments, HRG will be purified (1) to greater than 95% by weight of protein as determined by the Lowry method or other validated protein determination method, and most preferably more than 99% by weight, (2) to a degree sufficient to obtain at least 15 residues of N-terminal or internal amino acid sequence by use of the best commercially available amino acid sequenator marketed on the filing date hereof, or (3) to homogeneity by SDS-PAGE using Coomassie blue or, preferably, silver stain. Isolated HRG includes HRG in situ within recombinant cells since at least one component of HRG natural environment will not be present. Isolated HRG includes HRG from one species in a recombinant cell culture of another species since HRG in such circumstances will be devoid of source polypeptides. Ordinarily, however, isolated HRG will be prepared by at least one purification step.

In accordance with this invention, HRG nucleic acid is RNA or DNA containing greater than ten bases that encodes a biologically or antigenically active HRG, is complementary to nucleic acid sequence encoding such HRG, or hybridizes to nucleic acid sequence encoding such HRG and remains stably bound to it under stringent conditions.

Preferably, HRG nucleic acid encodes a polypeptide sharing at least 75% sequence identity, more preferably at least 80%, still more preferably at least 85%, even more preferably at 90%, and most preferably 95%, with an HRG sequence. Preferably, the HRG nucleic acid that hybridizes contains at least 20, more preferably at least about 40, and most preferably at least about 90 bases.

Isolated HRG nucleic acid includes a nucleic acid that is identified and separated from at least one containment nucleic acid with which it is ordinarily associated in the natural source of HRG nucleic acid. Isolated HRG nucleic acid thus is present in other than in the form or setting in which it is found in nature. However, isolated HRG encoding nucleic acid includes HRG nucleic acid in ordinarily HRG-expressing cells where the nucleic acid is in a chromosomal location different from that of natural cells or is otherwise flanked by a different DNA sequence than that found in nature. Nucleic acid encoding HRG may be used in specific hybridization assays, particularly those portions of HRG encoding sequence that do not hybridize with other known DNA sequences. "Stringent conditions" are those that (1) employ low ionic strength and high temperature for washing, for example, 0.015 M NACl/0.0015 M sodium citrate/0/1% NaDodSO₄ at 50° C; (2) employ during hybridization a denaturing agent such as formamide, for example, 50% (vol/vol) formamide with 0.1% bovine serum albumin, 0.1% Ficoll, 0.1% polyvinylpyrrolidone, 50 mM sodium phosphate buffer at pH 6.5 with 750 mM NaCl, 75 mM sodium citrate at 42° C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 g/ml), 0.1% SDS, and 10% dextran sulfate at 42° C, with washes at 42° C in 0.2 x SSC and 0.1% SDS.

The term "control sequences" refers to DNA sequences necessary for the expression of an operably linked coding sequence in a particular host organism. The control sequences that are suitable for prokaryotes, for example, include a promoter, optionally an operator sequence, a ribosome binding site, and possibly, other as yet poorly understood sequences. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However enhancers do not have to be contiguous. Linking is accomplished by ligation at convenient restriction sites. If such sites do not exist, then synthetic oligonucleotide adaptors or linkers are used in accord with conventional practice.

An "exogenous" element is defined herein to mean nucleic acid sequence that is foreign to the cell, or homologous to the cell but in a position within the host cell nucleic acid in which the element is ordinarily not found.

As used herein, the expressions "cell", "cell line", and "cell culture" are used interchangeably, and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. It will be clear from the context where distinct designations are intended.

"Plasmids" are designated by a lower case "p" preceded and/or followed by capital letters and/or numbers. The starting plasmids herein are commercially available, are publicly available on an unrestricted basis, or can be constructed from such available plasmids in accord with published procedures. In addition, other equivalent plasmids are known in the art and will be apparent to the ordinary artisan.

"Restriction enzyme digestion" of DNA refers to catalytic cleavage of the DNA with an enzyme that acts only at certain locations in the DNA. Such enzymes are called restriction endonucleases, and the sites for which each is specific is called a restriction site. The various restriction enzymes used herein are commercially available and their reaction conditions, cofactors, and other requirements as established by the enzyme suppliers are used. Restriction enzymes commonly are designated by abbreviations composed of a capital letter followed by other letters representing the microorganism from which each restriction enzyme originally was obtained, and then a number designating the particular enzyme. In general, about 1 mg of plasmid or DNA fragment is used with about 1-2 units of enzyme in about 20 ml of buffer solution. Appropriate buffers and substrate amounts for particular restriction enzymes are specified by the manufacturer. Incubation of about 1 hour at 37°C is ordinarily used, but may vary in accordance with the supplier's instructions. After incubation, protein or polypeptide is removed by extraction with phenol and chloroform, and the digested nucleic acid is recovered from the aqueous fraction by precipitation with ethanol. Digestion with a restriction enzyme may be followed with bacterial alkaline phosphatase hydrolysis of the terminal 5' phosphates to prevent the two restriction cleaved ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional as described in sections 1.56-1.61 of Sambrook *et al.,* (*Molecular Cloning: A Laboratory Manual* New York: Cold Spring Harbor Laboratory Press, 1989).

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally. For example, see Lawn *et al., Nucleic Acids Res*. **9:**6103-6114 (1981), and Goeddel *et al., Nucleic Acids Res*. **8:**4057 1980).

"Northern analysis" is a method used to identify RNA sequences that hybridize to a known probe such as an oligonucleotide, DNA fragment, cDNA or fragment thereof, or RNA fragment. The probe is labeled with a radioisotope such as ³²P, or by biotinylation, or with an enzyme. The RNA to be analyzed is usually electrophoretically separated on an agarose or polyacrylamide gel, transferred to nitrocellulose, nylon. or other suitable membrane. and hybridized with the probe. using standard techniques well known in the art such as those described in sections 7.39-7.52 of Sambrook *et al*., *supra*.

"Ligation" refers to the process of forming phosphodiester bonds between two nucleic acid fragments. To ligate the DNA fragments together, the ends of the DNA fragments must be compatible with each other. In some cases, the ends will be directly compatible after endonuclease digestion. However, it may be necessary to first convert the staggered ends commonly produced after endonuclease digestion to blunt ends to make them compatible for ligation. To blunt the ends, the DNA is treated in a suitable buffer for at least 15 minutes at 15°C with about 10 units of the Klenow fragment of DNA polymerase 1 or T4 DNA polymerase in the presence of the four deoxyribonucleotide triphosphates. The DNA is then purified by phenol-chloroform extraction and ethanol precipitation. The DNA fragments that are to be ligated together are put in solution in about equimolar amounts. The solution will also contain ATP, ligase buffer, and a ligase such as T4 DNA ligase at about 10 units per 0.5 mg of DNA. If the DNA is to be ligated into a vector, the vector is first linearized by digestion with the appropriate restriction endonuclease(s). The linearized fragment is then treated with bacterial alkaline phosphatase, or calf intestinal phosphatase to prevent self-ligation during the ligation step.

"Preparation" of DNA from cells means isolating the plasmid DNA from a culture of the host cells. Commonly used methods for DNA preparation are the large and small-scale plasmid preparations described in sections 1.25-1.33 of Sambrook *et al*., *supra*. After preparation of the DNA, it can be purified by methods well known in the art such as that described in section 1.40 of Sambrook *et al., supra*.

"Oligonucleotides" are short-length, single- or double-stranded polydeoxynucleotides that are chemically synthesized by known methods (such as phosphotriester, phosphite, or phosphoramidite chemistry, using solid phase techniques such as described in EP 266,032, published 4 May 1988, or via deoxynucleoside H-phosphonate intermediates as described by Froehler *et al., Nucl. Acids Res*. **14:**5399-5407, 1986. They are then purified on polyacrylamide gels.

The technique of "polymerase chain reaction," or "PCR," as used herein generally refers to a procedure wherein minute amounts of a specific piece of nucleic acid, RNA and/or DNA, are amplified as described in U.S. Pat. No. 4,683,195, issued 28 July 1987. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage or plasmid sequences, etc. See generally Mullis *et al., Cold Spring Harbor Symp. Quant. Biol*. **51**: 263 (1987); Erlich, ed., *PCR Technology*, (Stockton Press, NY, 1989). As used herein, PCR is considered to be one, but not the only, example of a nucleic acid polymerase reaction method for amplifying a nucleic acid test sample. comprising the use of a known nucleic acid (DNA or RNA) as a primer, and utilizes a nucleic acid polymerase to amplify or generate a specific piece of nucleic acid or to amplify or generate a specific piece of nucleic acid which is complementary to a particular nucleic acid.

The "HRG tyrosine autophosphorylation assay to detect the presence or bioactivity of HRG ligands can be used to monitor the purification of a ligand for the HER2 and HER3 receptors. This assay is based on the assumption that a specific ligand for the receptor will stimulate autophosphorylation of the receptor, in analogy with EGF and its stimulation of EGF receptor autophosphorylation. See *Sadich et al*., *Anal*. *Biochem.* 235:207-214 (1996). MDA-MB-453 cells or MCF7 cells which contain high levels of p185^{HER2} receptors but negligible levels of human EGF receptors, were obtained from the American Type Culture Collection, Rockville, Md. (ATCC No HTB-131) and maintained in tissue culture with 10% fetal calf serum in DMEM/Hams F12 (1:1) media. For assay, the cells were trypsinized and plated at 150,000 cells/well in 24 well dishes (Costar). After incubation with serum containing media overnight, the cells were placed in serum free media for 2-18 hours before assay. Test samples of 100 uL aliquots were added to each well. The cells were incubated for 5-30 minutes (typically 30 min) at 37°C and the media removed. The cells in each well were treated with 100 uL SDS gel denaturing buffer (SEPROSOL, Enpotech, Inc.) and the plates heated at 100°C for 5 minutes to dissolve the cells and denature the proteins. Aliquots from each well were electrophoresed on 5-20% gradient SDS gels (NOVEX, Encinitas, CA) according to the manufacturer's directions. After the dye front reached the bottom of the gel, the electrophoresis was terminated and a sheet of PVDF membrane (PROBLOTT, ABI) was placed on the gel and the proteins transferred from the gel to the membrane in a blotting chamber (BioRad) at 200 mAmps for 30-60 min. After blotting, the membranes were incubated with TRIS buffered saline containing 0.1% TWEEN 20 detergent buffer with 5% BSA for 2-18 hrs to block nonspecific binding, and then treated with a mouse anti-phosphotyrosine antibody (Upstate Biological Inc., N.Y.). Subsequently, the membrane blots were treated with goat anti-mouse antibody conjugated to alkaline phosphatase. The gels were developed using the PROTOBLOT System from Promega. After drying the membranes, the density of the bands corresponding to p185^{HER2} in each sample lane was quantitated with a Hewlett Packard SCANJET Plus Scanner attached to a Macintosh computer. The number of receptors per cell in the MDA-MB-453 cells is such that under these experimental conditions the p185^{HER2} receptor protein is the major protein which is labeled.

"Protein microsequencing" was accomplished based upon the following procedures. Proteins from the final HPLC step were either sequenced directly by automated Edman degradation with a model 470A Applied Biosystems gas phase sequencer equipped with a 120A PTH amino acid analyzer or sequenced after digestion with various chemicals or enzymes. PTH amino acids were integrated using the CHROMPERFECT data system (Justice Innovations. Palo Alto, CA). Sequence interpretation was performed on a VAX 11/785 Digital Equipment Corporation computer as described (Henzel *et al*., *J*. *Chromatography* **404**:41-52 (1987)). In some cases, aliquots of the HPLC fractions were electrophoresed on 5-20% SDS polyacrylamide gels, electrotransferred to a PVDF membrane (PROBLOTT, ABI, Foster City, CA) and stained with Coomassie Brilliant Blue (Matsudaira, P.. *J*. *Biol*. *Chem*. **262**:10035-10038, 1987). The specific protein was excised from the blot for N terminal sequencing. To determine internal protein sequences, HPLC fractions were dried under vacuum (SPEEDVAC), resuspended in appropriate buffers, and digested with cyanogen bromide, the lysine-specific enzyme Lys-C (Wako Chemicals, Richmond, VA) or Asp-N (Boehringer Mannheim, Indianapolis, Ind.). After digestion, the resultant peptides were sequenced as a mixture or were resolved by HPLC on a C4 column developed with a propanol gradient in 0.1% TFA before sequencing as described above.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment which contains a complete antigen-recognition and -binding site. This region consists of a dimer of one heavy chain and one light chain variable domain in tight, noncovalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxyl terminus of the heavy chain CH1 domain including one or more cysteine(s) from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), *e*.*g*., IgG1, IgG2, IgG3, IgG4, IgA, and IgA2. The heavy-chain constant domains that correspond to the different classes of immunoglobulins are called α, δ, ε, γ, and µ, respectively. The subunit structures and three-dimensional configurations of different classes of immunoglobulins are well known.

The term "antibody" herein is used in the broadest sense and specifically covers monoclonal antibodies (including fall length monoclonal antibodies), polyclonal antibodies, multispecific antibodies (e.g., bispecific antibodies), and antibody fragments so long as they exhibit the desired biological activity.

"Antibody fragments" comprise a portion of a full length antibody, generally the antigen binding or variable domain thereof. Examples of antibody fragments include Fab, Fab', F(ab')₂, and Fv fragments; diabodies; linear antibodies; single-chain antibody molecules; and multispecific antibodies formed from antibody fragments.

The term "monoclonal antibody" as used herein refers to an antibody obtained from a population of substantially homogeneous antibodies, *i*.*e*., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler *et al., Nature* 256:495 (1975), or may be made by recombinant DNA methods (see, *e.g*., U.S. Patent No. 4,816,567). The "monoclonal antibodies" may also be isolated from phage antibody libraries using the techniques described in Clackson *et al*., *Nature* 352:624-628 (1991) and Marks *et al., J. Mol. Biol*. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison *et al. Proc. Natl. Acad Sci. USA* 81:6851-6855 (1984)).

"Humanized" forms of non-human (*e*.*g*., murine) antibodies are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones *et al., Nature* 321:522-525 (1986); Reichmann *et al., Nature* 332:323-329 (1988); and Presta, *Curr. Op. Struct. Biol*. 2:593-596 (1992).

"Single-chain Fv" or "sFv" antibody fragments comprise the V_{H} and V_{L} domains of antibody, wherein these domains are present in a single polypeptide chain. Generally, the Fv polypeptide further comprises a polypeptide linker between the V_{H} and V_{L} domains which enables the sFv to form the desired structure for antigen binding. For a review of sFv see Pluckthun in *The Pharmacology of Monoclonal Antibodies*, vol. 113, Rosenburg and Moore eds. Springer-Verlag, New York, pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger *et al*., *Proc*. *Natl*. *Acad*. *Sci*. *USA* 90:6444-6448 (1993).

The expression "linear antibodies" when used throughout this application refers to the antibodies described in Zapata *et al*. *Protein Eng*. 8(10):1057-1062 (1995). Briefly, these antibodies comprise a pair of tandem Fd segments (V_{H}-C_{H}I-V_{H}-C_{H}I) which form a pair of antigen binding regions. Linear antibodies can be bispecific or monospecific.

### II. USE AND PREPARATION OF HRG SEQUENCES

### H. PREPARATION OF HRG SEQUENCES, INCLUDING VARIANTS

The system to be employed in preparing HRG sequence will depend upon the particular HRG sequence selected. If the sequence is sufficiently small HRG may be prepared by *in vitro* polypeptide synthetic methods. Most commonly, however, HRG will be prepared in recombinant cell culture using the host-vector systems described below. Suitable HRG includes any biologically active and antigenetically active HRG.

In general, mammalian host cells will be employed, and such hosts may or may not contain post-translational systems for processing HRG preprosequences in the normal fashion. If the host cells contain such systems then it will be possible to recover natural subdomain fragments such as HRG-GFD from the cultures. If not, then the proper processing can be accomplished by transforming the hosts with the required enzyme(s) or by supplying them in an *in vitro* method. However, it is not necessary to transform cells with the complete prepro or structural genes for a selected HRG when it is desired to only produce fragments of HRG sequences such as an HRG-GFD. For example, a start codon is ligated to the 5' end of DNA encoding an HRG-GFD, this DNA is used to transform host cells and the product expressed directly as the Met N-terminal form (if desired, the extraneous Met may be removed *in vitro* or by endogenous N-terminal demethionylases). Alternatively, HRG-GFD is expressed as a fusion with a signal sequence recognized by the host cell. which will process and secrete the mature HRG-GFD as is further described below. Amino acid sequence variants of native HRG-GFD sequences are produced in the same way.

HRG sequences located between the first N-terminal mature residue and the first N-terminal residue of HRG-GFD sequence, termed HRG-NTD, may function at least in part as an unconventional signal sequence or as a normally circulating carrier/precursor for HRG-GFD having unique biological activity. HRG-NTD is produced in the same fashion as the full length molecule but from expression of DNA in which a stop codon is located at the C-terminus of HRG-NTD. In addition, HRG variants are expressed from DNA encoding protein in which both the GFD and NTD domains are in their proper orientation but which contain an amino acid insertion, deletion or substitution at the GFD-NTD cleavage site (located within the sequence VKC) which inhibits or prevents proteolytic cleavage of the NTD-GFD joining site *in vivo*, and wherein a stop codon is positioned at the 3' end of the GFD- encoding sequence. In an example of this group of variants (termed HRG-NTDXGFD), (1) the lysine residue found in the NTD-GFD joining sequence VKC is deleted or (preferably) substituted by another residue other than arginyl such as histidyl, alanyl, threonyl or seryl and (2) a stop codon is introduced in the sequence RCT or RCQ in place of cysteinyl, or threonyl (for HRG-α) or glutaminyl (for HRG-β).

A preferred HRG-αligand with binding affinity to p185^{HER2} comprises amino acids 226-265 of figure 1A-D. This HRG-α ligand further may comprise up to an additional 1-20 amino acids preceding amino acid 226 and 1-20 amino acids following amino acid 265. A preferred HRG-β ligand with binding affinity to p185^{HER2} comprises amino acids 226-265 of figure 2A-E. This HRG-β ligand may comprise up to an additional 1-20 amino acids preceding amino acid 226 and 1-20 amino acids following amino acid 265.

As noted above, other HRG sequences to be prepared in accordance with this invention are those of the GFD. These are synthesized *in vitro* or are produced in recombinant cell culture. These are produced most inexpensively in yeast or *E.coli* by secretion under the control of a HRG-heterologous signal as described infra, although preparation in mammalian cells is also contemplated using a mammalian protein signal such as that of tPA, UK or a secreted viral protein. The GFD can be the sequence of a native HRG or may be a variant thereof as described below. GFD sequences include those in which one or more residues from a member of the EGF family are substituted into or onto the GFD sequence.

An additional HRG is one which contains the GFD and the sequence between the C-terminus of GFD and the N-terminus of the transmembrane domain (the later being termed the C-terminal cleavage domain or CTC). In this variant (HRG-GFD-CTC) the DNA start codon is present at the 5' end of HRG-heterologous signal sequence or adjacent the 5' end of the GFD-encoding region, and a stop codon is found in place of one of the first about 1 to 3 extra-cellular domain (ECD) residues or first about 1-2 transmembrane region residues. In addition, in some HRG-GFD-CTC variants the codons are modified in the GFD-CTC proteolysis site by substitution, insertion or deletion. The GFD-CTC proteolysis site is the domain that contains the GFD C-terminal residue and about 5 residues N- and 5 residues C-terminal from this residue. It is known that Met-227 terminal and Val-229 terminal HRG-α-GFD are biologically active. The C-terminus for HRG-α-GFD may be Met-227, Lys-228, Val-229, Gln-230, Asn-231 or Gln-232, and for HRG-β-GFD may be Met-226, Ala-227, Ser-228, Phe-229, Trp-230, or Lys231/Ser231. The native C-terminus is determined readily by C- terminal sequencing, although it is not critical that HRG-GFD have the native terminus so long as the GFD sequence possesses the desired activity. In some embodiments of HRG-GFD-CTC variants, the amino acid change(s) in the CTC are screened for their ability to resist proteolysis in *vitro* and inhibit the protease responsible for generation of HRG-GFD.

HRG-ECD variants are made by providing a stop codon at the same location as for HRG-GFD-CTC variants. HRG·ECD may comprise any one or more of the variants described above in connection with its subfragments, e.g. the GFD-CTC variants containing CTC-proteolysis site modifications.

If it is desired to prepare the longer HRG polypeptides and the 5' or 3' ends of the given HRG are not described herein, it may be necessary to prepare nucleic acids in which the missing domains are supplied by homologous regions from more complete HRG nucleic acids. Alternatively, the missing domains can be obtained by probing libraries using the DNAs disclosed in the Figures or fragments thereof.

### A. Isolation of DNA Encoding Heregulin

The DNA encoding HRG may be obtained from any cDNA library prepared from tissue believed to possess HRG mRNA and to express it at a detectable level. HRG-α gene thus may be obtained from a genomic library. Similar procedures may be used for the isolation of other HRG, such as HRG-β1, HRG-β2, or HRG-β3 encoding genes.

Libraries are screened with probes designed to identify the gene of interest or the protein encoded by it. For cDNA expression libraries, suitable probes include monoclonal or polyclonal antibodies that recognize and specifically bind to HRG-α; oligonucleotides of about 20-80 bases in length that encode known or suspected portions of HRG-α cDNA from the same or different species; and/or complementary or homologous cDNAs or fragments thereof that encode the same or a similar gene. Appropriate probes for screening genomic DNA libraries include, but are not limited to, oligonucleotides; cDNAs or fragments thereof that encode the same or a similar gene; and/or homologous genomic DNAs or fragments thereof. Screening the cDNA or genomic library with the selected probe may be conducted using standard procedures as described in chapters 10-12 of Sambrook *et al*., *supra*.

An alternative means to isolate the gene encoding HRG-α is to use polymerase chain reaction (PCR) methodology as described in section 14 of Sambrook *et al*., *supra*. This method requires the use of oligonucleotide probes that will hybridize to HRG-α. Strategies for selection of oligonucleotides are described below.

Another alternative method for obtaining the gene of interest is to chemically synthesize it using one of the methods described in Engels *et al*. (*Agnew*. *Chem*. *Int. Ed Engl*., **28**: 716-734,1989. These methods include triester, phosphite, phosphoramidite and H-Phosphonate methods, PCR and other autoprimer methods, and oligonucleotide syntheses on solid supports. These methods may be used if the entire nucleic acid sequence of the gene is known, or the sequence of the nucleic acid complementary to the coding strand is available, or alternatively, if the target amino acid sequence is known, one may infer potential nucleic acid sequences using known and preferred coding residues for each amino acid residue.

A preferred method of practicing this invention is to use carefully selected oligonucleotide sequences to screen cDNA libraries from various tissues, preferably human breast, colon, salivary gland, placental, fetal, brain, and carcinoma cell lines. Other biological sources of DNA encoding an heregulin-like ligand include other mammals and birds. Among the preferred mammals are members of the following orders: bovine, ovine, equine, murine, and rodentia.

The oligonucleotide sequences selected as probes should be of sufficient length and sufficiently unambiguous that false positives are minimized. The actual nucleotide sequence(s) may, for example, be based on conserved or highly homologous nucleotide sequences or regions of HRG-α. The oligonucleotides may be degenerate at one or more positions. The use of degenerate oligonucleotides may be of particular importance where a library is screened from a species in which preferential codon usage in that species is not known. The oligonucleotide must be labeled such that it can be detected upon hybridization to DNA in the library being screened. The preferred method of labeling is to use ³²P-labeled ATP with polynucleotide kinase, as is well known in the art, to radiolabel the oligonucleotide. However, other methods may be used to label the oligonucleotide, including, but not limited to, biotinylation or enzyme labeling.

Of particular interest is HRG-α nucleic acid that encodes a full-length polypeptide. In some preferred embodiments, the nucleic acid sequence includes the native HRG-α signal sequence. Nucleic acid having all the protein coding sequence is obtained by screening selected cDNA or genomic libraries, and, if necessary, using conventional primer extension procedures as described in section 7.79 of Sambrook *et al*., *supra*, to detect precursors and processing intermediates of mRNA that may not have been reverse-transcribed into cDNA.

HRG-α encoding DNA of Figures 1A-1D may be used to isolate DNA encoding the analogous ligand from other animal species via hybridization employing the methods discussed above. The preferred animals are mammals, particularly bovine, ovine, equine, feline, canine and rodentia, and more specifically rats, mice and rabbits.

### B. Amino Acid Sequence Variants of Heregulin

Amino acid sequence variants of HRG are prepared by introducing appropriate nucleotide changes into HRG DNA, or by *in vitro* synthesis of the desired HRG polypeptide. Such variants include, for example, deletions from, or insertions or substitutions of. residues within the amino acid sequence shown for human HRG sequences. Any combination of deletion, insertion, and substitution can be made to arrive at the final construct, provided that the final construct possesses the desired characteristics. Excluded from the scope of this invention are HRG variants or polypeptide sequences that are not novel and unobvious over the prior art. The amino acid changes also may alter post-translational processes of HRG-α, such as changing the number or position of glycosylation sites, altering the membrane anchoring characteristics, altering the intra-cellular location of HRG by inserting, deleting, or otherwise affecting the leader sequence of the native HRG, or modifying its susceptibility to proteolytic cleavage.

The HRG sequence may be proteolytically processed to create a number of HRG fragments. HRG-GFD sequences of HRG-α all contain the amino acid sequence between HRG-α cysteine 226 and cysteine 265. The amino terminus of HRG-α fragment may result from the cleavage of any peptide bond between alanine 1 and cysteine 226, preferably adjacent to an arginine, lysine, valine, or methionine, and most preferably between methionine 45 and serine 46. The carboxy terminus of HRG-α fragment may result from the cleavage of any peptide bond between cysteine 265, preferably adjacent to an arginine, lysine, valine, or methionine, and most preferably between lysine 272 and valine 273, between lysine 278 and alanine 279, or between lysine 285 and arginine 286. The resulting HRG-α ligands resulting from such proteolytic processing are the preferred ligands.

HRG-β-GFD's are analogous to those discussed above for HRG-α-GFD's. Each HRG-β-GFD contains the polypeptide segment from cysteine 212 to cysteine 251 of figure 2A-E. The amino terminus of HRG-β1 fragment may result from the cleavage of any peptide bond between alanine 1 and cysteine 212, preferably adjacent to an arginine, lysine, valine, or methionine, and most preferably between methionine 31 and serine 32. The carboxy terminus of HRG-β1 fragment may result from the cleavage of any peptide bond between cysteine 251 of Fig. 2A-2E, preferably adjacent to an arginine, lysine, valine, or methionine, and most preferably between valine 255 and methionine 256, between lysine 261 and histidine 262, between lysine 276 and alanine 277, or between lysine 301 and thrionine 302. The resulting HRG-β1 ligands resulting from such proteolytic processing are among the preferred ligands. Similarly, processing to produce preferred fragment ligands of HRG-β2 based upon the Fig. 3A-3E and HRG-β3 based upon Fig. 4A-4C may be accomplished by cleaving HRG sequences of Figs. 3A-3E and 4A-4C preferably adjacent to an arginine, lysine, valine or methionine.

In designing amino acid sequence variants of HRG, the location of the mutation site and the nature of the mutation will depend on HRG characteristic(s) to be modified. The sites for mutation can be modified individually or in series, e.g., by (1) substituting first with conservative amino acid choices and then with more radical selections depending upon the results achieved, (2) deleting the target residue, or (3) inserting residues of other receptor ligands adjacent to the located site.

A useful method for identification of certain residues or regions of HRG polypeptide that are preferred locations for mutagenesis is called "alanine scanning mutagenesis" as described by Cunningham and Wells (*Science*. **244**: 1081-1085, 1989). Here, a residue or group of target residues are identified (e.g., charged residues such as arg, asp, his, lys, and glu) and replaced by a neutral or negatively charged amino acid (most preferably alanine or polyalanine) to affect the interaction of the amino acids with the surrounding aqueous environment in or outside the cell. Those domains demonstrating functional sensitivity to the substitutions then are refined by introducing further or other variants at or for the sites of substitution. Thus, while the site for introducing an amino acid sequence variation is predetermined, the nature of the mutation *per se* need not be predetermined. For example, to optimize the performance of a mutation at a given site, ala scanning or random mutagenesis may be conducted at the target codon or region and the expressed HRG variants are screened for the optimal combination of desired activity.

There are two principal variables in the construction of amino acid sequence variants: the location of the mutation site and the nature of the mutation. These are variants from HRG sequence, and may represent naturally occurring alleles (which will not require manipulation of HRG DNA) or predetermined mutant forms made by mutating the DNA, either to arrive at an allele or a variant not found in nature. In general, the location and nature of the mutation chosen will depend upon HRG characteristic to be modified. Obviously, such variations that, for example, convert HRG into a known receptor ligand, are not included within the scope of this invention, nor are any other HRG variants or polypeptide sequences that are not novel and unobvious over the prior art.

Amino acid sequence deletions generally range from about 1 to 30 residues, more preferably about I to 10 residues, and typically about 1 to 5 are contiguous. Deletions may be introduced into regions of low homology with other EGF family precursors to modify the activity of HRG. Deletions from HRG in areas of substantial homology with other EGF family sequences will be more likely to modify the biological activity of HRG more significantly. The number of consecutive deletions will be selected so as to preserve the tertiary structure of HRG in the affected domain, e.g., cysteine crosslinking, beta-pleated sheet or alpha helix.

Amino acid sequence insertions include amino- and/or carboxyl-terminal fusions ranging in length from one residue to polypeptides containing a hundred or more residues, as well as intrasequence insertions of single or multiple amino acid residues. Intrasequence insertions (i.e., insertions within HRG sequence) may range generally from about 1 to 10 residues, more preferably 1 to 5, and most preferably 1 to 3. Examples of terminal insertions include HRG with an N-terminal methionyl residue (an artifact of the direct expression of HRG in bacterial recombinant cell culture), and fusion of a heterologous N-terminal signal sequence to the N-terminus of HRG to facilitate the secretion of mature HRG from recombinant host cells. Such signal sequences generally will be obtained from, and thus be homologous to, the intended host cell species. Suitable sequences include STII, tPA or lpp for *E*. *coli,* alpha factor for yeast, and viral signals such as herpes gD for mammalian cells.

Other insertional variants of HRG include the fusion to the N- or C-terminus of HRG of an immunogenic polypeptide, e.g., bacterial polypeptides such as beta-lactamase or an enzyme encoded by the *E. coli trp* locus, or yeast protein, bovine serum albumin, and chemotactic polypeptides. C-terminal fusions of HRG-ECD with proteins having a long half-life such as immunoglobulin constant regions (or other immunoglobulin regions), albumin, or ferritin, as described in WO 89/02922, published 6 April 1989 are contemplated.

Another group of variants are amino acid substitution variants. These variants have at least one amino acid residue in HRG molecule removed and a different residue inserted in its place. The sites of greatest interest for substitutional mutagenesis include sites identified as the active site(s) of HRG, and sites where the amino acids found in HRG ligands from various species are substantially different in terms of side-chain bulk, charge, and/or hydrophobicity. A likely sub-domain of HRG-GFD having biological activity as a growth factor is the C-terminal segment, in particular within the sequence about from glycine 218 to valine 226 (HRG-α), and glycine 218 to lysine 228/serine 228 (HRG-β) based upon analogy to the EGF subsequence found to have EGF activity.

Other sites of interest are those in which particular residues of HRG-like ligands obtained from various species are identical. These positions may be important for the biological activity of HRG. These sites, especially those falling within a sequence of at least three other identically conserved sites, are substituted in a relatively conservative manner. Such conservative substitutions are shown in Table 1 under the heading of "preferred substitutions". If such substitutions result in a change in biological activity, then more substantial changes, denominated exemplary substitutions in Table 1, or as further described below in reference to amino acid classes, are introduced and the products screened.

**TABLE 1**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his: lys; arg | gln |
| Asp (D) | glu | glu |
| Cys (C) | ser | ser |
| Gln (Q) | asn | asn |
| Glu (E) | asp | asp |
| Gly (G) | pro | pro |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala | leu |
| Pro (P) | gly | gly |
| Ser (S) | thr | thr |
| Thr (T) | ser | ser |
| Trp (W) | tyr | tyr |
| Tyr (Y) | trp; phe; thr; ser | phe |
| Val (V) | ile; leu; met; phe; ala; norieucine | leu |

Substantial modifications in function or immunological identity of HRG are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side chain properties:
1) hydrophobic: norleucine, met, ala, val. leu, ile;
2) neutral hydrophilic: cys, ser, thr;
3) acidic: asp, glu;
4) basic: asn, gln, his, lys, arg;
5) residues that influence chain orientation: gly, pro; and
6) aromatic: trp, tyr, phe.

Non-conservative substitutions will entail exchanging a member of one of these classes for another. Such substituted residues may be introduced into regions of HRG that are homologous with other receptor ligands, or, more preferably, into the non-homologous regions of the molecule.

In one embodiment of the invention, it is desirable to inactivate one or more protease cleavage sites that are present in the molecule. These sites are identified by inspection of the encoded amino acid sequence. Where protease cleavage sites are identified, they are rendered inactive to proteolytic cleavage by substituting the targeted residue with another residue, preferably a basic residue such as glutamine or a hydrophylic residue such as serine; by deleting the residue; or by inserting a prolyl residue immediately after the residue.

In another embodiment, any methionyl residue other than the starting methionyl residue of the signal sequence, or any residue located within about three residues N- or C-terminal to each such methionyl residue, is substituted by another residue (preferably in accord with Table 1) or deleted. Alternatively, about 1-3 residues are inserted adjacent to such sites.

Any cysteine residues not involved in maintaining the proper conformation of HRG also may be substituted, generally with serine, to improve the oxidative stability of the molecule and prevent aberrant crosslinking.

Sites particularly suited for substitutions, deletions or insertions, or use as fragments, include, numbered from the N-terminus of HRG-α of Figure 1A - 1D:
1) potential glycosaminoglycan addition sites at the serine-glycine dipeptides at 42-43, 64-65, 151-152;
2) potential asparagine-linked glycosylation at positions 164, 170, 208 and 437, sites (NDS) 164-166, (NIT) 170-172, (NTS) 208-210, and NTS (609-611);
3) potential O-glycosylation in a cluster of serine and threonine at 209-218;
4) cysteines at 226, 234, 240, 254, 256 and 265;
5) transmembrane domain at 287-309;
6) loop I delineated by cysteines 226 and 240;
7) loop 2 delineated by cysteines 234 and 254;
8) loop 3 delineated by cysteines 256 and 265; and
9) potential protease processing sites at 2-3, 8-9, 23-24, 33-34, 36-37, 45-46, 48-49, 62-63, 66-67, 86-87, 110-111, 123-124, 134-135, 142-143, 272-273, 278-279 and 285-286;

Analogous regions in HRG-β1 may be determined by reference to its' sequence. The analogous HRG-β1 amino acids may be mutated or modified as discussed above for HRG-α. Analogous regions in HRG-β2 may also be determined by reference to its' sequence. The analogous HRG-β2 amino acids may be mutated or modified as discussed above for HRG-α or HRG-β1. Analogous regions in HRG-β3 may be determined by reference to its' sequence. Further, the analogous HRG-β3 amino acids may be mutated or modified as discussed above for HRG-α, HRG-β1, or HRG-β2.

Another HRG variant is γ-HRG (or gamma-heregulin). γ-HRG is any polypeptide sequence that possesses at least one biological property of native sequence γ-HRG having SEQ ID NO:11. The biological property of this variant is the same as for HRG noted above. This variant encompasses not only the polypeptide isolated from a native γ-HRG source such as human MDA-MB-175 cells or from another source, such as another animal species, but also the polypeptide prepared by recombinant or synthetic methods. It also includes variant forms including functional derivatives, allelic variants, naturally occurring isoforms and analogues thereof. Sometimes the γ-HRG is "native γ-HRG" which refers to endogenous γ-HRG polypeptide which has been isolated from a mammal. The γ-HRG can also be "native sequence γ-HRG" insofar as it has the same amino acid sequence as a native γ-HRG (*e*.*g*. human γ-HRG shown in Fig. 7A-7C). Amino acid sequence variants of the native sequence are prepared by introducing appropriate nucleotide changes into the native sequence DNA, or by *in vitro* synthesis of the desired polypeptide. Such variants include, for example, deletions from, or insertions or substitutions of, residues within the amino acid sequence shown for the human protein in Fig. 7A-7C as generally described above for other HRG. Any combination of deletion, insertion, and substitution is made to arrive at the final construct, provided that the final construct possesses the desired characteristics. The amino acid changes also may alter post-translational processes of the native sequence, such as changing the number or position of O-linked glycosylation sites.

Another variant is the polypeptide referred to as sensory and motor neuron derived factor (SMDF) whose nucleic acid and amino acid sequences (SEQ ID NOS:13 and 14) are shown in Fig. 8 which can be prepared as described in WO 96/15244. The SMDF polypeptides of the invention exliibit the properties of binding to the HER2/HER3 receptors and stimulating epithelial cell growth and differentiation in a manner similar to HRG polypeptides discussed above. Amino acid sequence variants of native sequence SMDF are prepared by introducing appropriate nucleotide changes into the native sequence SMDF DNA, or by *in vitro* synthesis of the desired SMDF polypeptide as observed generally above for other HRG. Such variants include, for example, deletions from, or insertions or substitutions of, residues within the amino acid sequence shown for human SMDF in Fig. 8. Any combination of deletion, insertion, and substitution is made to arrive at the final construct, provided that the final construct possesses the desired characteristics. The amino acid changes also may alter post-translational processes of the native sequence SMDF, such as changing the number or position of O-linked glycosylation sites.

Additional variants include polypeptides in which the variant has an amino acid substitution at a selected residue corresponding to a residue of 645-amino acid native human heregulin-β1 selected from:
S177, H178, L179, V180, K181, E184, E186,
K187, T188, V191, N192, G193, G194, E195,
M198, V199, K200, D201, N204, P205, S206,
R207, Y208, L209, K211, P213, N214, E215,
T217, G218, D219, Q222, N223, Y224, S228, and F229.
In a variation of this embodiment, the amino acid substitution is not a replacement of the selected residue with an epidermal growth factor (EGF) residue corresponding to the selected residue.

Other heregulin-β1 variants include an amino acid substitution selected from:
S177W; H178S, E, R, or A; V180Q, I or E;
K181P or A; A183G; E184V, W, K, R, G, or N;
K185E, S, Q, or G; E186R; K187E or A; T188Q;
E195Q; F197Y; M198R or K; K200R; D201T or I;
P205T or Y; S206K. H, G, P, or R; R207Y:
Y208R or L; L209M or G; K211R; P213S, T, N, or K;
N214L, K, S, or E; F216M; N223H or W; and M226I.
In a variation of this embodiment, the heregulin variant includes sets of amino acid substitutions selected from this group. Some heregulin variants of the invention having sets of amino acid substitutions exhibit at least a 50-fold increase in HER3 receptor affinity, which is also accompanied by an increase in HER4 receptor affinity. Specific variants include:
A183G, E184W, K185D, E186R, K187E, T188G, M226I;
A183D, E184K, K185S, E186R, K187E, T188G, M226I;
F197Y, M198K, K200R, D201I, M226I;
P205Y, S206G, R207Y, Y208L, L209M;
P205Y, S206R, R207Y, Y208R, L209M, M226I;
P205T, S206H, R207Y, Y208R, L209M;
P205T. S206K, R207Y, Y208R, L209G;
N223W, M226I;
N223H, M226I;
S177W, H178E, K181P, A183G, E184W, K185D, E186R, K187E, T188G, M226I;
P205Y, S206G, R207Y, Y208L, L209M, M2261;
A183G, K185E, E186R, K187E, T188G, F197Y, M198R, D201T:
A183G, K185E, E186R, K187E, T188G, P205Y, S206G, R207Y, Y208L, L209M;
A183G, K185E, E186R, K187E, T188G, F197Y, M198R, D201T, P205Y, S206G, R207Y, Y208L, L209M;
A183G, K185E, E186R, K187E, T188G, M226I;
F197Y, M198R, D201T, P205Y, S206G, R207Y, Y208L. L209M;
F197Y, M198R, D201T, P205Y, S206G, R207Y, Y208L, L209M. M226I;
F197Y, M198R, D201T, M226I;
A183G, K185E, E186R, K187E, T188G, F197Y, M198R, D201T, M226I;
A183G, K185E, E186R, K187E, T188G, P205Y, S206G, R207Y, Y208L. L209M, M226I;
A183G, K185E, E186R, K187E, T188G, F197Y, M198R, D201T, P205Y, S206G, R207Y, Y208L, L209M, M226I;
F197Y, M198R, D201T, P205Y, S206G, R207Y, Y208L, L209M, N223H, M226I; and
A183G, K185E, E186R, K187E, T188G, F197Y, M198R, D201T, P205Y, S206G, R207Y, Y208L, L209M, N223H, M226I.

In addition to including one or more of the amino acid substitutions disclosed herein, the heregulin variant can have one or more other modifications, such as an amino acid substitution, an insertion of at least one amino acid, a deletion of at least one amino acid, or a chemical modification. For example, the invention provides a heregulin variant that is a fragment. In a variation of this embodiment, the fragment includes residues corresponding to a portion of human heregulin-β1 extending from about residue 175 to about residue 230 (i.e., the EGF-like domain). For example, the fragment can extend from residue 177 to residue 244 and may be prepared by recombinant techniques (rHRGβ1-177-244).

DNA encoding amino acid sequence variants of HRG is prepared by a variety of methods known in the art. These methods include, but are not limited to, isolation from a natural source (in the case of naturally occurring amino acid sequence variants) or preparation by oligonucleotide-mediated (or site-directed) mutagenesis. PCR mutagenesis, and cassette mutagenesis of an earlier prepared variant or a non-variant version of HRG. These techniques may utilize HRG nucleic acid (DNA or RNA), or nucleic acid complementary to HRG nucleic acid.

Oligonucleotide-mediated mutagenesis is a preferred method for preparing substitution, deletion, and insertion variants of HRG DNA. This technique is well known in the art as described by Adelman *et al*., *DNA*, **2**: 183 (1983). Briefly, HRG DNA is altered by hybridizing an oligonucleotide encoding the desired mutation to a DNA template, where the template is the single-stranded form of a plasmid or bacteriophage containing the unaltered or native DNA sequence of HRG. After hybridization, a DNA polymerase is used to synthesize an entire second complementary strand of the template that will thus incorporate the oligonucleotide primer, and will code for the selected alteration in HRG DNA.

Generally, oligonucleotides of at least 25 nucleotides in length are used. An optimal oligonucleotide will have 12 to 15 nucleotides that are completely complementary to the template on either side of the nucleotide(s) coding for the mutation. This ensures that the oligonucleotide will hybridize properly to the single-stranded DNA template molecule. The oligonucleotides are readily synthesized using techniques known in the art such as that described by Crea *et al*. *(Proc*. *Natl*. *Acad Sci*. *USA*, **75**: 5765,1978).

Single-stranded DNA template may also be generated by denaturing double-stranded plasmid (or other) DNA using standard techniques.

For alteration of the native DNA sequence (to generate amino acid sequence variants, for example), the oligonucleotide is hybridized to the single-stranded template under suitable hybridization conditions. A DNA polymerizing enzyme, usually the Klenow fragment of DNA polymerase I, is then added to synthesize the complementary strand of the template using the oligonucleotide as a primer for synthesis. A heteroduplex molecule is thus formed such that one strand of DNA encodes the mutated form of HRG, and the other strand (the original template) encodes the native, unaltered sequence of HRG. This heteroduplex molecule is then transformed into a suitable host cell, usually a prokaryote such as *E. coli* JM101. After the cells are grown, they are plated onto agarose plates and screened using the oligonucleotide primer radiolabeled with ³²P-phosphate to identify the bacterial colonies that contain the mutated DNA. The mutated region is then removed and placed in an appropriate vector for protein production, generally an expression vector of the type typically employed for transformation of an appropriate host.

The method described immediately above may be modified such that a homoduplex molecule is created wherein both strands of the plasmid contain the mutation(s). The modifications are as follows: the single-stranded oligonucleotide is annealed to the single-stranded template as described above. A mixture of three deoxyribonucleotides. deoxyriboadenosine (dATP), deoxyriboguanosine (dGTP), and deoxyribothymidine (dTTP), is combined with a modified thio-deoxyribocytosine called dCTP-(aS) (which can be obtained from Amersham Corporation). This mixture is added to the template-oligonucleotide complex. Upon addition of DNA polymerase to this mixture, a strand of DNA identical to the template except for the mutated bases is generated. In addition, this new strand of DNA will contain dCTP-(aS) instead of dCTP, which serves to protect it from restriction endonuclease digestion. After the template strand of the double-stranded heteroduplex is nicked with an appropriate restriction enzyme, the template strand can be digested with *Exo*III nuclease or another appropriate nuclease past the region that contains the site(s) to be mutagenized. The reaction is then stopped to leave a molecule that is only partially single-stranded. A complete double-stranded DNA homoduplex is then formed using DNA polymerase in the presence of all four deoxyribonucleotide triphosphates, ATP. and DNA ligase. This homoduplex molecule can then be transformed into a suitable host cell such as *E*. *coli* JM101, as described above.

DNA encoding HRG mutants with more than one amino acid to be substituted may be generated in one of several ways. If the amino acids are located close together in the polypeptide chain, they may be mutated simultaneously using one oligonucleotide that codes for all of the desired amino acid substitutions. If, however, the amino acids are located some distance from each other (separated by more than about ten amino acids), it is more difficult to generate a single oligonucleotide that encodes all of the desired changes. Instead, one of two alternative methods may be employed.

In the first method, a separate oligonucleotide is generated for each amino acid to be substituted. The oligonucleotides are then annealed to the single-stranded template DNA simultaneously, and the second strand of DNA that is synthesized from the template will encode all of the desired amino acid substitutions.

The alternative method involves two or more rounds of mutagenesis to produce the desired mutant. The first round is as described for the single mutants: wild-type DNA is used for the template, an oligonucleotide encoding the first desired amino acid substitution(s) is annealed to this template, and the heteroduplex DNA molecule is then generated. The second round of mutagenesis utilizes the mutated DNA produced in the first round of mutagenesis as the template. Thus, this template already contains one or more mutations. The oligonucleotide encoding the additional desired amino acid substitution(s) is then annealed to this template, and the resulting strand of DNA now encodes mutations from both the first and second rounds of mutagenesis. This resultant DNA can be used as a template in a third round of mutagenesis, and so on.

PCR mutagenesis is also suitable for making amino acid variants of HRG. While the following discussion refers to DNA, it is understood that the technique also finds application with RNA. The PCR technique generally refers to the following procedure (see Erlich, *supra*, the chapter by R. Higuchi, p. 61-70). When small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template. For introduction of a mutation into a plasmid DNA, one of the primers is designed to overlap the position of the mutation and to contain the mutation: the sequence of the other primer must be identical to a stretch of sequence of the opposite strand of the plasmid, but this sequence can be located anywhere along the plasmid DNA. It is preferred, however, that the sequence of the second primer is located within 200 nucleotides from that of the first, such that in the end the entire amplified region of DNA bounded by the primers can be easily sequenced. PCR amplification using a primer pair like the one just described results in a population of DNA fragments that differ at the position of the mutation specified by the primer, and possibly at other positions, as template copying is somewhat error-prone.

If the ratio of template to product material is extremely low, the vast majority of product DNA fragments incorporate the desired mutation(s). This product material is used to replace the corresponding region in the plasmid that served as PCR template using standard DNA technology. Mutations at separate positions can be introduced simultaneously by either using a mutant second primer, or performing a second PCR with different mutant primers and ligating the two resulting PCR fragments simultaneously to the vector fragment in a three (or more)-part ligation.

In a specific example of PCR mutagenesis, template plasmid DNA (1mg) is linearized by digestion with a restriction endonuclease that has a unique recognition site in the plasmid DNA outside of the region to be amplified. Of this material, 100 ng is added to a PCR mixture containing PCR buffer, which contains the four deoxynucleotide tri-phosphates and is included in the GENEAMP kits (obtained from Perkin-Elmer Cetus, Norwalk, CT and Emeryville, CA), and 25 pmole of each oligonucleotide primer, to a final volume of 50 ml. The reaction mixture is overlaid with 35 ml mineral oil. The reaction is denatured for 5 minutes at 100°C, placed briefly on ice, and then 1 ml *Thermus aquaticus (Taq)* DNA polymerase (5 units/ml, purchased from Perkin-Elmer Cetus, Norwalk, CT and Emeryville, CA) is added below the mineral oil layer. The reaction mixture is then inserted into a DNA Thermal Cycler (purchased from Perkin-Elmer Cetus) programmed as follows:
2 min. 55°C,
30 sec. 72°C, then 19 cycles of the following:
30 sec. 94°C,
30 sec. 55°C, and
30 sec. 72°C.

At the end of the program, the reaction vial is removed from the thermal cycler and the aqueous phase transferred to a new vial, extracted with phenol/chloroform (50:50:vol), and ethanol precipitated, and ' the DNA is recovered by standard procedures. This material is subsequently subjected to the appropriate treatments for insertion into a vector.

Another method for preparing variants, cassette mutagenesis, is based on the technique described by Wells *et al*. (*Gene*, **34**: 315,1985). The starting material is the plasmid (or other vector) comprising HRG DNA to be mutated. The codon(s) in HRG DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in HRG DNA. After the restriction sites have been introduced into the plasmid, the plasmid is cut at these sites to linearize it. A double-stranded oligonucleotide encoding the sequence of the DNA between the restriction sites but containing the desired mutation(s) is synthesized using standard procedures. The two strands are synthesized separately and then hybridized together using standard techniques. This double-stranded oligonucleotide is referred to as the cassette. This cassette is designed to have 3' and 5' ends that are compatible with the ends of the linearized plasmid, such that it can be directly ligated to the plasmid. This plasmid now contains the mutated HRG DNA sequence.

### C. Insertion of DNA into a Cloning Vehicle

The cDNA or genomic DNA encoding native or variant HRG is inserted into a replicable vector for further cloning (amplification of the DNA) or for expression. Many vectors are available. and selection of the appropriate vector will depend on 1) whether it is to be used for DNA amplification or for DNA expression, 2) the size of the DNA to be inserted into the vector, and 3) the host cell to be transformed with the vector. Each vector contains various components depending on its function (amplification of DNA or expression of DNA) and the host cell for which it is compatible. The vector components generally include, but are not limited to, one or more of the following: a signal sequence, an origin of replication, one or more marker genes, an enhancer element, a promoter, and a transcription termination sequence.

### (i) Signal Sequence Component

In general, the signal sequence may be a component of the vector, or it may be a part of HRG DNA that is inserted into the vector. The native HRG DNA is believed to encode a signal sequence at the amino terminus (5' end of the DNA encoding HRG) of the polypeptide that is cleaved during post-translational processing of the polypeptide to form the mature HRG polypeptide ligand that binds to the HER2/HER3 receptor, although a conventional signal structure is not apparent. Native HRG is, secreted from the cell but remains lodged in the membrane because it contains a transmembrane domain and a cytoplasmic region in the carboxyl terminal region of the polypeptide. Thus, in a secreted, soluble version of HRG the carboxyl terminal domain of the molecule, including the transmembrane domain, is ordinarily deleted. This truncated variant HRG polypeptide may be secreted from the cell, provided that the DNA encoding the truncated variant encodes a signal sequence recognized by the host.

HRG of this invention may be expressed not only directly, but also as a fusion with a heterologous polypeptide, preferably a signal sequence or other polypeptide having a specific cleavage site at the N-and/or C-terminus of the mature protein or polypeptide. In general, the signal sequence may be a component of the vector, or it may be a part of HRG DNA that is inserted into the vector. Included within the scope of this invention are HRG with the native signal sequence deleted and replaced with a heterologous signal sequence. The heterologous signal sequence selected should be one that is recognized and processed, i.e., cleaved by a signal peptidase, by the host cell. For prokaryotic host cells that do not recognize and process the native HRG signal sequence, the signal sequence is substituted by a prokaryotic signal sequence selected, for example, from the group of the alkaline phosphatase, penicillinase, Ipp, or heat-stable enterotoxin II leaders. For yeast secretion the native HRG signal sequence may be substituted by the yeast invertase, alpha factor, or acid phosphatase leaders. In mammalian cell expression the native signal sequence is satisfactory, although other mammalian signal sequences may be suitable.

### (ii) Origin of Replication Component

Both expression and cloning vectors generally contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2m plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

Most expression vectors are "shuttle" vectors, i.e., they are capable of replication in at least one class of organisms but can be transfected into another organism for expression. For example, a vector is cloned in *E*. *coli* and then the same vector is transfected into yeast or mammalian cells for expression even though it is not capable of replicating independently of the host cell chromosome.

DNA may also be amplified by insertion into the host genome. This is readily accomplished using *Bacillus* species as hosts, for example, by including in the vector a DNA sequence that is complementary to a sequence found in *Bacillus* genomic DNA. Transfection of *Bacillus* with this vector results in homologous recombination with the genome and insertion of HRG DNA. However, the recovery of genomic DNA encoding HRG is more complex than that of an exogenously replicated vector because restriction enzyme digestion is required to excise HRG DNA. DNA can be amplified by PCR and directly transfected into the host cells without any replication component.

### (iii) Selection Gene Component

Expression and cloning vectors should contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli*.

One example of a selection scheme utilizes a drug to arrest growth of a host cell. Those cells that are successfully transformed with a heterologous gene express a protein conferring drug resistance and thus survive the selection regimen. Examples of such dominant selection use the drugs neomycin (Southern *et al., J. Molec. Appl*. *Genet*. **1:** 327,1982), mycophenolic acid (Mulligan *et al., Science* **209:** 1422,1980) or hygromycin (Sugden *et al., Mol. Cell. Biol*. **5:** 410-413,1985). The three examples given above employ bacterial genes under eukaryotic control to convey resistance to the appropriate drug G418 or neomycin (geneticin), xgpt (mycophenolic acid), or hygromycin, respectively.

Another example of suitable selectable markers for mammalian cells are those that enable the identification of cells competent to take up HRG nucleic acid, such as dihydrofolate reductase (DHFR) or thymidine kinase. The mammalian cell transformants are placed under selection pressure which only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes HRG. Amplification is the process by which genes in greater demand for the production of a protein critical for growth are reiterated in tandem within the chromosomes of successive generations of recombinant cells. Increased quantities of HRG are synthesized from the amplified DNA.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity, prepared and propagated as described by Urlaub and Chasin, *Proc. Natl. Acad. Sci. USA*, **77**: 4216, 1980. The transformed cells are then exposed to increased levels of methotrexate. This leads to the synthesis of multiple copies of the DHFR gene, and, concomitantly, multiple copies of other DNA comprising the expression vectors, such as the DNA encoding HRG. This amplification technique can be used with any otherwise suitable host, e.g., ATCC No. CCL61 CHO-K1, notwithstanding the presence of endogenous DHFR if, for example, a mutant DHFR gene that is highly resistant to Mtx is employed (EP 117,060). Alternatively, host cells (particularly wild-type hosts that contain endogenous DHFR) transformed or co-transformed with DNA sequences encoding HRG, wild-type DHFR protein, and another selectable marker such as aminoglycoside 3' phosphotransferase (APH) can be selected by cell growth in medium containing a selection agent for the selectable marker such as an aminoglycosidic antibiotic, e.g., kanamycin, neomycin, or G418 (see U.S. Pat. No. 4,965,199).

A suitable selection gene for use in yeast is the *trp*1 gene present in the yeast plasmid YRp7 (Stinchcomb *et al., Nature*, **282**: 39, 1979; Kingsman *et al., Gene*, **7**: 141, 1979; or Tschemper *et al., Gene*, **10:** 157, 1980). The *trp*1 gene provides a selection marker for a mutant strain of yeast lacking the ability to grow in tryptophan, for example, ATCC No. 44076 or PEP4-1 (Jones, *Genetics*, **85:** 12, 1977). The presence of the *trp*1 lesion in the yeast host cell genome then provides an effective environment for detecting transformation by growth in the absence of tryptophan. Similarly, *Leu*2-deficient yeast strains (ATCC 20,622 or 38,626) are complemented by known plasmids bearing the *Leu*2 gene.

### (iv) Promoter Component

Expression and cloning vectors usually contain a promoter that is recognized by the host organism and is operably linked to HRG nucleic acid. Promoters are untranslated sequences located upstream (5') to the start codon of a structural gene (generally within about 100 to 1000 bp) that control the transcription and translation of a particular nucleic acid sequence, such as HRG to which they are operably linked. Such promoters typically fall into two classes, inducible and constitutive. Inducible promoters are promoters that initiate increased levels of transcription from DNA under their control in response to some change in culture conditions, e.g., the presence or absence of a nutrient or a change in temperature. At this time a large number of promoters recognized by a variety of potential host cells are well known. These promoters are operably linked to DNA encoding HRG by removing the promoter from the source DNA by restriction enzyme digestion and inserting the isolated promoter sequence into the vector. Both the native HRG promoter sequence and many heterologous promoters may be used to direct amplification and/or expression of HRG DNA. However, heterologous promoters are preferred, as they generally permit greater transcription and higher yields of expressed HRG as compared to the native HRG promoter.

Promoters suitable for use with prokaryotic hosts include the b-lactamase and lactose promoter systems (Chang *et al*., *Nature*, **275**: 615, 1978; and Goeddel *et al*., *Nature* **281**: 544, 1979), alkaline phosphatase, a tryptophan (trp) promoter system (Goeddel. *Nucleic Acids Res*., **8**: 4057, 1980 and EP 36,776), tPA (U.S. 5,641,655) and hybrid promoters such as the tac promoter (deBoer *et al*., *Proc*. *Natl*. *Acad Sci*. *USA* **80**: 21-25, 1983). However, other known bacterial promoters are suitable. Their nucleotide sequences have been published, thereby enabling a skilled worker operably to ligate them to DNA encoding HRG (Siebenlist *et al*., *Cell* **20**: 269, 1980) using linkers or adaptors to supply any required restriction sites. Promoters for use in bacterial systems also generally will contain a Shine-Dalgarno (S.D.) sequence operably linked to the DNA encoding HRG.

Suitable promoting sequences for use with yeast hosts include the promoters for 3-phosphoglycerate kinase (Hitzeman *et al*., *J*. *Biol*. *Chem*., **255**: 2073, 1980) or other glycolytic enzymes (Hess *et al*., *J*. *Adv*. *Enzyme Reg* 7: 149, 1968; and Holland, *Biochemistry* **17**: 4900, 1978), such as enolase, glyceraldehyde 3-phosphate dehydrogenase, hexokinase, pyruvate decarboxylase, phosphofructokinase, glucose-6-phosphate isomerase. 3-phosphoglycerate mutase, pyruvate kinase. triosephosphate isomerase, phosphoglucose isomerase. and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in Hitzeman *et al*., EP 73,657A. Yeast enhancers also are advantageously used with yeast promoters.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence that may be the signal for addition of the poly A tail to the 3' end of the coding sequence. All of these sequences are suitably inserted into mammalian expression vectors.

HRG gene transcription from vectors in mammalian host cells may be controlled by promoters obtained from the genomes of viruses such as polyoma virus, fowlpox virus (UK 2,211,504, published 5 July 1989), adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g., the actin promoter or an immunoglobulin promoter, from heat-shock promoters, and from the promoter normally associated with HRG sequence, provided such promoters are compatible with the host cell systems.

The early and late promoters of the SV40 virus are conveniently obtained as an SV40 restriction fragment that also contains the SV40 viral origin of replication (Fiers *et al*., *Nature*, **273**:113 (1978); Mulligan and Berg, *Science,* **209**: 1422-1427 (1980); Pavlakis *et al., Proc. Natl. Acad Sci. USA*, **78**: 7398-7402 (1981)). The immediate early promoter of the human cytomegalovirus is conveniently obtained as a *Hind*III E restriction fragment (Greenaway *et al., Gene*, **18:** 355-360 (1982)). A system for expressing DNA in mammalian hosts using the bovine papilloma virus as a vector is disclosed in U.S. 4,419,446. A modification of this system is described in U.S. 4,601,978. See also Gray *et al., Nature*, **295**: 503-508 (1982) on expressing cDNA encoding immune interferon in monkey cells; Reyes *et al., Nature*, **297**: 598-601 (1982) on expression of human b-interferon cDNA in mouse cells under the control of a thymidine kinase promoter from herpes simplex virus; Canaani and Berg, *Proc. Natl. Acad Sci. USA*, **79**: 5166-5170 (1982) on expression of the human interferon b1 gene in cultured mouse and rabbit cells; and Gorman *et al*., *Proc. Nail. Acad. Sci. USA*, **79**: 6777-6781 (1982) on expression of bacterial CAT sequences in CV-1 monkey kidney cells, chicken embryo fibroblasts, Chinese hamster ovary cells, HeLa cells, and mouse NIH-3T3 cells using the Rous sarcoma virus long terminal repeat as a promoter.

### (v) Enhancer Element Component

Transcription of a DNA encoding HRG of this invention by higher eukaryotes is often increased by inserting an enhancer sequence into the vector. Enhancers are cis-acting elements of DNA, usually about from 10-300 bp, that act on a promoter to increase its transcription. Enhancers are relatively orientation and position independent having been found 5' (Laimins *et al., Proc. Natl. Acad Sci. USA*, **78**: 993, 1981) and 3' (Lusky *et al*., *Mol. Cell Bio*., **3**: 1108, 1983) to the transcription unit, within an intron (Banerji *et al., Cell*, **33**: 729, 1983) as well as within the coding sequence itself (Osborne *et al., Mol*. *Cell Bio*., **4**: 1293, 1984). Many enhancer sequences are now known from mammalian genes (globin, elastase, albumin, a-fetoprotein and insulin). Typically, however. one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers (see also Yaniv, *Nature*, **297**: 17-18 (1982)) on enhancing elements for activation of eukaryotic promoters. The enhancer may be spliced into the vector at a position 5' or 3' to HRG DNA, but is preferably located at a site 5' from the promoter.

### (vi) Transcription Termination Component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3' untranslated regions of eukaryotic or viral DNAs or cDNAs. These regions contain nucleotide segments transcribed as polyadenylated fragments in the untranslated portion of the mRNA encoding HRG. The 3' untranslated regions also include transcription termination sites.

Construction of suitable vectors containing one or more of the above listed components the desired coding and control sequences employs standard ligation techniques. Isolated plasmids or DNA fragments are cleaved. tailored, and religated in the form desired to generate the plasmids required.

For analysis to confirm correct sequences in plasmids constructed, the ligation mixtures are used to transform *E. coli* K12 strain 294 (ATCC 31,446) and successful transformants selected by ampicillin or tetracycline resistance where appropriate. Plasmids from the transformants are prepared, analyzed by restriction endonuclease digestion, and/or sequenced by the method of Messing *et al., Nucleic Acids Res*. **9**: 309 (1981) or by the method of Maxam *et al., Methods in Enzymology* **65:** 499 (1980).

Particularly useful in the practice of this invention are expression vectors that provide for the transient expression in mammalian cells of DNA encoding HRG. In general, transient expression involves the use of an expression vector thai is able to replicate efficiently in a host cell, such that the host cell accumulates many copies of the expression vector and, in turn, synthesizes high levels of a desired polypeptide encoded by the expression vector. Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying analogs and variants of HRG that have HRG-like activity. Such a transient expression system is described in U.S. 5,024,939.

Other methods, vectors, and host cells suitable for adaptation to the synthesis of HRG in recombinant vertebrate cell culture are described in Gething *et al., Nature* **293:** 620-625, 1981; Mantei *et al., Nature*, **281**: 40-46, 1979; Levinson *et al*., EP 117,060 and EP 117,058. A particularly useful expression plasmid for mammalian cell culture expression of HRG is pRKS (EP pub. no. 307,247).

### D. Selection and Transformation of Host Cells

Suitable host cells for cloning or expressing the vectors herein are the prokaryote, yeast, or higher eukaryote cells described above. Suitable prokaryotes include eubacteria, such as Gram-negative or Gram-positive organisms, for example, *E. coli, Bacilli* such as *B. subtilis, Pseudomonas* species such as *P. aeruginosa, Salmonella typhimurium,* or *Serratia marcescans*. One preferred *E. coli* cloning host is *E. coli* 294 (ATCC 31,446), although other strains such as *E. coli B, E. coli* ₓ1776 (ATCC 31,537), and *E. coli* W3110 (ATCC 27,325) are suitable. These examples are illustrative rather than limiting. Preferably the host cell should secrete minimal amounts of proteolytic enzymes. Alternatively, *in vitro* methods of cloning, e.g., PCR or other nucleic acid polymerase reactions, are suitable.

In addition to prokaryotes, eukaryotic microbes such as filamentous fungi or yeast are suitable hosts for HRG-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe* (Beach and Nurse, *Nature,* **290**: 140 (1981); EP 139,383, published May 2, 1985), *Kluyveromyces* hosts (U.S.S.N. 4,943,529) such as, e.g., *K. lactis* (Louvencourt *et al., J. Bacteriol*., **737** (1983); *K. fragilis, K. bulgaricus, K. thermotolerans*, and *K. marxianus, yarrowia* (EP 402,226); *Pichia pastoris* (EP 183,070). Sreekrishna *et al., J. Basic Microbiol*., **28**: 265-278 (1988); *Candeda, Trichoderma reesia* (EP 244,234); *Neurospora crassa* (Case *et al., Proc. Natl. Acad Sci. USA*, **76:** 5259-5263 (1979), and filamentous fungi such as, e.g, *Neurospora, Penicillium, Tolypocladium* (WO 91/00357, published 10 January 1991), and *Aspergillus* hosts such as *A. nidulans* (Ballance *et al., Biochem*. *Biophys. Res. Commun*., **112:** 284-289 (1983); Tilbum *et al., Gene*, **26:** 205-221 (1983); Yelton *et al., Proc. Natl. Acad Sci. USA*, **81:** 1470-1474 (1984) and *A. niger* (Kelly and Hynes, *EMBO J*., **4:** 475-479 (1985)).

Suitable host cells for the expression of glycosylated HRG polypeptide are derived from multicellular organisms. Such host cells are capable of complex processing and glycosylation activities. In principle, any higher eukaryotic cell culture is workable, whether from vertebrate or invertebrate culture. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* host cells have been identified (see, e.g., Luckow *et al., Bio*/*Technology*, **6**: 47-55 (1988); Miller *et al*., in *Genetic Engineering*, Setlow, J.K. *et al*., eds., Vol. **8** (Plenum Publishing, 1986), pp. 277-279; and Maeda *et al., Nature*, **315**: 592-594 (1985)). A variety of such viral strains are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells. Plant cell cultures of cotton, com, potato, soybean, petunia, tomato, and tobacco can be utilized as hosts. Typically, plant cells are transfected by incubation with certain strains of the bacterium *Agrobacterium tumefaciens*. which has been previously manipulated to contain HRG DNA. During incubation of the plant cell culture with *A*. *tumefaciens*, the DNA encoding HRG is transferred to the plant cell host such that it is transfected, and will, under appropriate conditions, express HRG DNA. In addition, regulatory and signal sequences compatible with plant cells are available, such as the nopaline synthase promoter and polyadenylation signal sequences (Depicker *et al*., *J*. *Mol*. *Appl*. *Gen*., **1**: 561 (1982)). In addition, DNA segments isolated from the upstream region of the T-DNA *780* gene are capable of activating or increasing transcription levels of plant-expressible genes in recombinant DNA-containing plant tissue (see EP 321,196, published 21 June 1989).

However, interest has been greatest in vertebrate cells, and propagation of vertebrate cells in culture (tissue culture) has become a routine procedure in recent years (*Tissue Culture*, Academic Press, Kruse and Patterson, editors (1973)). Examples of useful mammalian host cell lines are monkey kidney CV1 line transformed by SV40 (COS-7, ATCC CRL 1651); human embryonic kidney line (293 or 293 cells subcloned for growth in suspension culture, Graham *et al*., *J*. *Gen Virol*., **36**: 59, 1977); baby hamster kidney cells (BHK, ATCC CCL 10); Chinese hamster ovary cells/-DHFR (CHO, Urlaub and Chasin, *Proc*. *Natl*. *Acad. Sci*. *USA*, 77: 4216 (1980)); mouse sertoli cells (TM4, Mather, *Biol*. *Reprod*., 23: 243-251 (1980)); monkey kidney cells (CV1 ATCC CCL 70); African green monkey kidney cells (VERO-76. ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al*., *Annals N*.*Y*. *Acad. Sci*., 383: 44-68 (1982)); MRC 5 cells; FS4 cells; and a human hepatoma cell line (Hep G2). Preferred host cells are human embryonic kidney 293 and Chinese hamster ovary cells.

Host cells are transfected and preferably transformed with the above-described expression or cloning vectors of this invention and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducing DNA into an organism so that the DNA is replicable, either as an extrachromosomal element or by chromosomal integration. Depending on the host cell used, transformation is done using standard techniques appropriate to such cells. The calcium treatment employing calcium chloride, as described in section 1.82 of Sambrook *et al*., *supra*, is generally used for prokaryotes or other cells that contain substantial cell-wall barriers. Infection with *Agrobacterium tumefaciens* is used for transformation of certain plant cells, as described by Shaw *et al*., *Gene*, 23: 315 (1983) and WO 89/05859, published 29 June 1989. For mammalian cells without such cell walls, the calcium phosphate precipitation method described in sections 16.30-16.37 of Sambrook *et al*, *supra*, is preferred. General aspects of mammalian cell host system transformations have been described by Axel in U.S. 4,399,216, issued 16 August 1983. Transformations into yeast are typically carried out according to the method of Van Solingen *et al*., *J*. *Bact*., 130: 946 (1977) and Hsiao *et al*., *Proc*. *Natl*. *Acad*. *Sci*. *(USA)*, 76: 3829 (1979). However, other methods for introducing DNA into cells such as by nuclear injection, electroporation, or protoplast fusion may also be used.

### E. Culturing the Host Cells

Prokaryotic cells used to produce HRG polypeptide of this invention are cultured in suitable media as described generally in Sambrook *et al*., *supra*.

The mammalian host cells used to produce HRG of this invention may be cultured in a variety of media. Commercially available media such as Ham's F10 (Sigma), Minimal Essential Medium ((MEM), Sigma), RPMI-1640 (Sigma), and Dulbecco's Modified Eagle's Medium ((DMEM), Sigma) are suitable for culturing the host cells. In addition, any of the media described in Ham and Wallace, *Meth. Ent*., 58: 44 (1979), Barnes and Sato, *Anal. Biochem.,* 102: 255 (1980), U.S. 4,767,704; 4,657,866; 4,927,762; or 4,560,655; WO 90/03430; WO 87/00195; U.S. Pat. Re. 30,985; or U.S. 5,122,469 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as GENTAMYCIN drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

The host cells referred to in this disclosure encompass cells in *in vitro* culture as well as cells that are within a host animal.

It is further envisioned that HRG of this invention may be produced by homologous recombination, or with recombinant production methods utilizing control elements introduced into cells already containing DNA encoding HRG currently in use in the field. For example, a powerful promoter/enhancer element, a suppresser, or an exogenous transcription modulatory element is inserted in the genome of the intended host cell in proximity and orientation sufficient to influence the transcription of DNA encoding the desired HRG. The control element does not encode HRG of this invention, but the DNA is present in the host cell genome. One next screens for cells making HRG of this invention, or increased or decreased levels of expression, as desired.

### F. Detecting Gene Amplification/Expression

Gene amplification and/or expression may be measured in a sample directly, for example, by conventional Southern blotting, Northern blotting to quantitate the transcription of mRNA (Thomas, *Proc*. *Natl*. *Acad. Sci*. *USA*, 77: 5201-5205 (1980)), dot blotting (DNA analysis), or *in situ* hybridization, using an appropriately labeled probe based on the sequences provided herein. Various labels may be employed, most commonly radioisotopes, particularly ³²P. However, other techniques may also be employed, such as using biotin-modified nucleotides for introduction into a polynucleotide. The biotin then serves as the site for binding to avidin or antibodies which may be labeled with a wide variety of labels, such as radionuclides, fluorescers, enzymes, or the like. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled where the labels are usually visually detectable such as enzymatic labels, fluorescent labels, luminescent labels, and the like. A particularly sensitive staining technique suitable for use in the present invention is described by Hsu *et al*., *Am*. *J*. *Clin*. *Path*., 75: 734-738 (1980).

Antibodies useful for immunohistochemical staining and/or assay of sample fluids may be either monoclonal or polyclonal, and may be prepared in any mammal. Conveniently, the antibodies may be prepared against a native HRG polypeptide or against a synthetic peptide based on the DNA sequences provided herein as described further below.

### G. Purification of The Heregulin Polypeptides

HRG is recovered from a cellular membrane fraction. Alternatively, a proteolytically cleaved or a truncated expressed soluble HRG fragment or subdomain are recovered from the culture medium as a soluble polypeptide. A HRG is recovered from host cell lysates when directly expressed without a secretory signal.

When HRG is expressed in a recombinant cell other than one of human origin, HRG is completely free of proteins or polypeptides of human origin. However, it is desirable to purify HRG from recombinant cell proteins or polypeptides to obtain preparations that are substantially homogeneous as to HRG. As a first step, the culture medium or lysate is centrifuged to remove particulate cell debris. The membrane and soluble protein fractions are then separated. HRG is then be purified from both the soluble protein fraction (requiring the presence of a protease) and from the membrane fraction of the culture lysate, depending on whether HRG is membrane bound. The following procedures are exemplary of suitable purification procedures: fractionation on immunoaffinity or ion-exchange columns; ethanol precipitation; reverse phase HPLC; chromatography on silica, heparin SEPHAROSE or on a cation exchange resin such as DEAE; chromatofocusing; SDS-PAGE; ammonium sulfate precipitation; and gel filtration using, for example, SEPHADEX G-75.

HRG variants in which residues have been deleted, inserted or substituted are recovered in the same fashion as the native HRG, taking account of any substantial changes in properties occasioned by the variation. For example, preparation of a HRG fusion with another protein or polypeptide, e.g., a bacterial or viral antigen, facilitates purification; an immunoaffinity column containing antibody to the antigen can be used to adsorb the fusion. Immunoaffinity columns such as a rabbit polyclonal anti-HRG column can be employed to absorb HRG variant by binding it to at least one remaining immune epitope. A protease inhibitor such as phenylmethylsulfonylfluoride (PMSF) also may be useful to inhibit proteolytic degradation during purification, and antibiotics may be included to prevent the growth of adventitious contaminants. One skilled in the art will appreciate that purification methods suitable for native HRG may require modification to account for changes in the character of HRG variants or upon expression in recombinant cell culture.

### H. Covalent Modifications of HRG

Covalent modifications of HRG polypeptides are included within the scope of this invention. Both native HRG and amino acid sequence variants of HRG optionally are covalently modified. One type of covalent modification included within the scope of this invention is a HRG polypeptide fragment. HRG fragments, such as HRG-GDF, having up to about 40 amino acid residues are conveniently prepared by chemical synthesis, or by enzymatic or chemical cleavage of the full-length HRG polypeptide or HRG variant polypeptide. Other types of covalent modifications of HRG or fragments thereof are introduced into the molecule by reacting targeted amino acid residues of HRG or fragments thereof with an organic derivatizing agent that is capable of reacting with selected side chains or the N- or C-terminal residues.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing a-amino-containing residues include imidoesters such as methyl picolinimidate; pyridoxal phosphate; pyridoxal; chloroborohydride; trinitrobenzenesulfonic acid; O-methylisourea; 2,4-pentanedione: and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method described above being suitable.

Carboxyl side groups (aspartyl or glutamyl) are selectively modified by reaction with carbodiimides (R'-N=C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for crosslinking HRG to a water-insoluble support matrix or surface for use in a method for purifying anti-HRG antibodies, and vice versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-((p-azidophenyl)dithio)propioimidate yield photoactivatable intermediates that are capable of forming crosslinks in the presence of light. Alternatively, reactive water-insoluble matrices such as cyanogen bromide-activated carbohydrates and the reactive substrates described in U.S. 3,969,287; 3,691,016; 4,195,128; 4,247,642; 4,229,537; and 4,330,440 are employed for protein immobilization.

Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the a-amino groups of lysine, arginine, and histidine side chains (T.E. Creighton, *Proteins: Structure and Molecular Properties*, W.H. Freeman & Co., San Francisco, pp. 79-86 (1983)), acetylation of the N-terminal amine, and amidation of any C-terminal carboxyl group.

HRG optionally is fused with a polypeptide heterologous to HRG. The heterologous polypeptide optionally is an anchor sequence such as that found in a phage coat protein such as M13 gene III or gene VIII proteins. These heterologous polypeptides can be covalently coupled to HRG polypeptide through side chains or through the terminal residues.

HRG may also be covalently modified by altering its native glycosylation pattern. One or more carbohydrate substituents in these embodiments, are modified by adding, removing or varying the monosaccharide components at a given site, or by modifying residues in HRG as that glycosylation sites are added or deleted.

Glycosylation of polypeptides is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in a polypeptide creates a potential giycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.

Glycosylation sites are added to HRG by altering its amino acid sequence to contain one or more of the above-described tri-peptide sequences (for N-linked glycosylation sites). The alteration may also be made by the addition of, or substitution by, one or more serine or threonine residues to HRG (for O-linked glycosylation sites). For ease, HRG is preferably altered through changes at the DNA level, particularly by mutating the DNA encoding HRG at preselected bases such that codons are generated that will translate into the desired amino acids.

Chemical or enzymatic coupling of glycosides to HRG increases the number of carbohydrate substituents. These procedures are advantageous in that they do not require production of the polypeptide in a host cell that is capable of N- and O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, published 11 September 1987, and in Aplin and Wriston (*CRC Crit*. *Rev*. *Biochem*., pp. 259-306 (1981)).

Carbohydrate moieties present on an HRG also are removed chemically or enzymatically. Chemical deglycosylation requires exposure of the polypeptide to the compound trifluoromethanesulfonic acid, or an equivalent compound. This treatment results in the cleavage of most or all sugars except the linking sugar (N-acetylglucosamine or N-acetylgalactosamine), while leaving the polypeptide intact. Chemical deglycosylation is described by Hakimuddin *et al*. *(Arch*. *Biochem*. *Biophys*., 259:52 (1987)) and by Edge *et al*. *(Anal. Biochem.,* 118:131 (1981)). Carbohydrate moieties are removed from HRG by a variety of endo- and exo- glycosidases as described by Thotakura *et al*. (*Meth*. *Enzymol*., 138:350 (1987)).

Glycosylation also is suppressed by tunicamycin as described by Duskin *et al*. (*J. Biol*. *Chem*., 257:3105 (1982)). Tunicamycin blocks the formation of protein-N-glycoside linkages.

HRG may also be modified by linking HRG to various nonproteinaceous polymers, e.g., polyethylene glycol, polypropylene glycol or polyoxyalkylenes, in the manner set forth in U.S. 4,640,835; 4,496,689; 4,301,144; 4,670,417; 4,791,192 or 4,179,337.

One preferred way to increase the *in vivo* circulating half life of non-membrane bound HRG is to conjugate it to a polymer that confers extended half-life, such as polyethylene glycol (PEG). (Maxfield, *et al*, Polymer 16,505-509 (1975); Bailey, F. E., *et al*, in Nonionic Surfactants (Schick, M. J., ed.) pp.794-821, 1967); (Abuchowski, A. *et al*., J. Biol. Chem. 252, 3582-3586, 1977; Abuchowski, A. *et al*., *Cancer Biochem. Biophys*. 7, 175-186, 1984); (Katre, N.V. *et al*., *Proc. Natl*. *Acad*. *Sci*., 84, 1487-1491, 1987; Goodson, R. *et al*. *Bio Technology,* 8, 343-346, 1990). Conjugation to PEG also has been reported to have reduced immunogenicity and toxicity (Abuchowski, A, *et al*., *J*. *Biol*. *Chem*., 252, 3578-3581, 1977).

HRG may also be entrapped in microcapsules prepared, for example, by coacervation techniques or by interfacial polymerization (for example, hydroxymethylcellulose or gelatin-microcapsules and poly(methylmethacylate) microcapsules, respectively), in colloidal drug delivery systems (for example, liposomes, albumin microspheres, microemulsions, nano-particles and nanocapsules), or in macroemulsions. Such techniques are disclosed in *Remington's Pharmaceutical Sciences,* 16th edition, Osol, A., Ed., (1980).

Those skilled in the art will be capable of screening variants in order to select the optimal variant for the purpose intended. For example, a change in the immunological character of HRG, such as a change in affinity for a given antigen or for the HER2 receptor, is measured by a competitive-type immunoassay using a standard or control such as a native HRG (in particular native HRG-GFD). Other potential modifications of protein or polypeptide properties such as redox or thermal stability, hydrophobicity, susceptibility to proteolytic degradation, stability in recombinant cell culture or in plasma, or the tendency to aggregate with carriers or into multimers are assayed by methods well known in the art.

### I. Heregulin Antibody Preparation

The antibodies of this invention are obtained by routine screening and include polyclonal antibodies. monoclonal antibodies and fragments thereof.

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, *e.g*., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e*.*g*., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies may be made using the hybridoma method first described by Kohler *et al., Nature*, 256:495 (1975), or may be made by recombinant DNA methods (U.S. Patent No. 4,816,567).

In the hybridoma method, a mouse or other appropriate host animal, such as a hamster or macaque monkey, is immunized as hereinabove described to elicit lymphocytes that produce or are capable of producing antibodies that will specifically bind to the protein used for immunization. Alternatively, lymphocytes may be immunized *in vitro*. Lymphocytes then are fused with myeloma cells using a suitable fusing agent, such as polyethylene glycol, to form a hybridoma cell (Goding, *Monoclonal Antibodies: Principles and Practice*, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOP-21 and M.C.-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, *J*. *Immunol*., 133:3001 (1984); Brodeur *et al*., *Monoclonal Antibody Production Techniques and Applications*, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the Scatchard analysis of Munson *et al*., *Anal*. *Biochem*., 107:220 (1980).

After hybridoma cells are identified that produce antibodies of the desired specificity, affinity, and/or activity, the clones may be subcloned by limiting dilution procedures and grown by standard methods (Goding, *Monoclonal Antibodies: Principles and Practice*, pp.59-103 (Academic Press, 1986)). Suitable culture media for this purpose include, for example, D-MEM or RPM1-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-SEPHAROSE, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies will be described in more detail below.

Hybridoma cell lines producing antibodies are identified by screening the culture supernatants for antibody which binds to HER2/HER3 receptors. This is routinely accomplished by conventional immunoassays using soluble receptor preparations or by FACS using cell-bound receptor and labeled candidate antibody. Agonist antibodies are preferably antibodies which stimulate autophosphorylation in the HRG tyrosine autophosphorylation assay described above.

The hybrid cell lines can be maintained in culture *in vitro* in cell culture media. The cell lines of this invention can be selected and/or maintained in a composition comprising the continuous cell line in hypoxanthine-aminopterin thymidine (HAT) medium. In fact, once the hybridoma cell line is established, it can be maintained on a variety of nutritionally adequate media. Moreover, the hybrid cell lines can be stored and preserved in any number of conventional ways, including freezing and storage under liquid nitrogen. Frozen cell lines can be revived and cultured indefinitely with resumed synthesis and secretion of monoclonal antibody. The secreted antibody is recovered from tissue culture supernatant by conventional methods such as precipitation, ion exchange chromatography, affinity chromatography, or the like. The antibodies described herein are also recovered from hybridoma cell cultures by conventional methods for purification of IgG or IgM as the case may be that heretofore have been used to purify these immunoglobulins from pooled plasma, e.g., ethanol or polyethylene glycol precipitation procedures.

Human antibodies may be used and are preferable. Such antibodies can be obtained by using human hybridomas (Cote *et al*., *Monoclonal Antibodies and Cancer Therapy*, Alan R. Liss, p. 77 (1985)). Chimeric antibodies, Cabilly et al., U.S. 4,816,567, (Morrison *et al*., *Proc*. *Natl*. *Acad. Sci*., 81:6851 (1984); Neuberger *et al*., *Nature* 312:604 (1984); Takeda *et al*., *Nature* 314:452 (1985)) containing a murine anti-HER2/HER3 variable region and a human constant region of appropriate biological activity (such as ability to activate human complement and mediate ADCC) are within the scope of this invention, as are humanized antibodies produced by conventional CDR-grafting methods(Riechmann et al., Nature 332:333-327(1988); EP 0328404 A1; EP 02394000 A2).

Techniques for creating recombinant DNA versions of the antigen-binding regions of antibody molecules (Fab or variable regions fragments) which bypass the generation of monoclonal antibodies are also encompassed within the practice of this invention. One extracts antibody-specific messenger RNA molecules from immune system cells taken from an immunized subject, transcribes these into complementary DNA (cDNA), and clones the cDNA into a bacterial expression system and selects for the desired binding characteristic. The Scripps/Stratagene method uses a bacteriophage lambda vector system containing a leader sequence that causes the expressed Fab protein to migrate to the periplasmic space (between the bacterial cell membrane and the cell wall) or to be secreted. One can rapidly generate and screen great numbers of functional Fab fragments to identify those which bind the receptors with the desired characteristics. Alternatively, the antibodies can be prepared by the phage display techniques described in Hoogenboom, *Tibtech* February 1997 (vol 15); Neri *et al., Cell Biophysics* **27:**47-61 (1995); Winter *et al., Annu. Rev. Immunol*. **12**:433-55 (1994); and Soderlind *et al., Immunol. Rev*. **130:**109-124 (1992) and the references described therein as well as the monovalent phage display technique described in Lowman *et al., Biochem*. **30:**10832-10838 (1991).

### 2. Therapeutic compositions, administration and use of Heregulins and Agonist Antibodies

The HRG are used in the present invention to induce epithelial cell growth, for example lung epithelial cell growth, proliferation and differentiation , and to increase the production of surfactant protein A by lung cells. These effects allow treatment of disease states associated with tissue damage, for example, chronic obstructive pulmonary disease(COPD) including subtypes thereof such as chronic bronchitis, emphysema, asthma, etc., neonatal pulmonary diseases including neonatal respiratory distress syndrome, meconium aspiration syndrome, chronic lung disease of the neonate, congenital diaphragmatic hernia, etc., acute lung injuries including smoke or chemical inhalation, pneumonitis due to aspiration, radiation, etc., near drowning, cystic fibrosis and other epithelial cell trauma diseases, including injuries associated with surgical wounds and resections, ulcers, lesions, and tissue tears, with the medicament prepared in accordance with the invention.

A preferred indication for treatment with the medicament prepared in accordance with the invention is the treatment of COPD. COPD is a spectrum of chronic inflammatory respiratory diseases characterized by cough, sputum, dyspnea, airflow limitation and impaired gas exchange. COPD is common in older populations and presents a pattern of gradually declining lung function. Typically, a patient will exhibit a chronic cough with clear sputum which worsens to a cough with thick sputum and accompanying poor air exchange. These conditions frequently lead to heart disease and death. Many persons with COPD will have chronic bronchitis together with emphysema. The present invention is particularly important because it halts, slows and/or reverses the lung destruction process in COPD patients. In this action, the medicament prepared in accordance with the invention is very different from typical treatments for COPD in which the symptoms are treated, but not the underlying destruction of lung cell tissue and function.

The medicament prepared in accordance with the invention may, however, be combined with or administered together with other therapies for treatment of lung disease such as COPD. For example, the medicament prepared in accordance with this invention can be used together with the administration of an anticholinergic bronchodilator such as ipratropium bromide (ATROVENT available from Boehringer Ingleheim) or tiotropium, a beta adrenergic receptor agonist such as albuterol (PROVENTIL available from Schering) or salmeterol, steroids such as prednisone, retinoic acid, phosphodiesterase inhibitors, endothelin antagonists, metalloproteinase inhibitors, elastase inhibitors, free radical inhibitors, serine proteinase inhibitors, neutrophil elastase inhibitors, pulmonary surfactant compositions such as beractant (SURVANTA available from Ross Labs.) , PDGF, FGF, EGF, growth hormone or other protein growth factors, etc. or combinations thereof. The relative amount of the HRG and the additional compound(s) can be readily determined by a physician with regard to the individual symptoms of the patient. It is anticipated that these compositions and the relative amounts of the components therein will be varied as necessary to address the specific needs of a patient and will be monitored and adjusted using conventional physiochemical and medical tests for lung function.

Therapeutic formulations of HRG or agonist antibody are prepared for storage by mixing the HRG protein having the desired degree of purity with optional physiologically acceptable carriers, excipients, or stabilizers (*Remington's Pharmaceutical Sciences, supra*), in the form of lyophilized cake or aqueous solutions. Acceptable carriers, excipients or stabilizers are nontoxic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN, PLURONICS or polyethylene glycol (PEG).

HRG or agonist antibody to be used for *in vivo* administration must be sterile. This is readily accomplished by filtration through sterile filtration membranes, prior to or following lyophilization and reconstitution. The HRG or antibody ordinarily will be stored in lyophilized form or in solution.

Therapeutic HRG or antibody compositions generally are placed into a container having a sterile access port, for example, an intravenous solution bag or vial having a stopper pierceable by a hypodermic injection needle.

The route of HRG or antibody administration is in accord with known methods, e.g., injection or infusion by intravenous, intraperitoneal, intracerebral, intramuscular, intraocular, intraarterial, powder or liquid aerosol administration to the nose or lung or intralesional routes, or by sustained release systems as noted below. The HRG ligand may be administered continuously by infusion or by bolus injection. An agonist antibody is preferably administered in the same fashion, or by administration into the blood stream or lymph.

The HRG, HRG variant or fragment and agonist antibodies may be spray dried or spray freeze dried using known techniques (Yeo et al, Biotech. and Bioeng., 41:341-346 (1993); Gombotz et al, PCT/US90/02421).

Suitable examples of sustained-release preparations include semipermeable matrices of solid hydrophobic polymers containing the protein, which matrices are in the form of shaped articles, e.g. films, or microcapsules. Examples of sustained-release matrices include polyesters, hydrogels (e.g., poly(2-hydroxyethyl-methacrylate) as described by Langer *et al*., *J*. *Biomed. Mater*. *Res*., 15: 167-277 (1981) and Langer, *Chem*. *Tech*., 12: 98-105 (1982) or poly(vinylalcohol)), polylactides (U.S. 3,773,919, EP 58,481), copolymers of L-glutamic acid and gamma ethyl-L-glutamate (Sidman *et al*., *Biopolymers*, 22: 547-556 (1983)), non-degradable ethylene-vinyl acetate (Langer *et al*., *supra*), degradable lactic acid-glycolic acid copolymers such as the LUPRON DEPOT (injectable microspheres composed of lactic acid-glycolic acid copolymer and leuprolide acetate), and poly-D-(-)-3-hydroxybutyric acid (EP 133,988). While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C, resulting in a loss of biological activity and possible changes in immunogenicity. Rational strategies can be devised for protein stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Sustained-release HRG or antibody compositions also include liposomally entrapped HRG or antibody. Liposomes containing HRG or antibody are prepared by methods known *per se*: DE 3,218,121; Epstein *et al., Proc. Natl. Acad. Sci. USA*. 82: 3688-3692 (1985): Hwang *et al., Proc. Natl. Acad. Sci. USA*, 77: 4030-4034 (1980); EP 52,322; EP 36,676; EP 88.046; EP 143,949; EP 142,641; Japanese patent application 83-118008; U.S. 4,485,045 and 4,544,545; and EP 102,324. Ordinarily the liposomes are of the small (about 200-800 Angstroms) unilamelar type in which the lipid content is greater than about 30 mol. % cholesterol, the selected proportion being adjusted for the optimal HRG therapy. Liposomes with enhanced circulation time are disclosed in U.S. patent 5,013,556.

An effective amount of HRG or antibody to be employed therapeutically will depend, for example, upon the therapeutic objectives, the route of administration, and the condition of the patient. Also, the amount of HRG polypeptide will generally be less than the amount of an agonist antibody. Accordingly, it will be necessary for the therapist to titer the dosage and modify the route of administration as required to obtain the optimal therapeutic effect. A typical daily dosage might range from about 1 µg/kg to about 1 mg/kg and up to 100 mg/kg or more, depending on the factors mentioned above. Typically, the clinician will administer HRG or antibody until a dosage is reached that achieves the desired effect. The progress of this therapy is easily monitored by conventional assays, for example, surfactant protein A production.

In a further embodiment, epithelial cells may be obtained or isolated from a mammalian tissue to obtain a normal epithelial cell sample using techniques well known in the art (biopsy, etc.). This sample may then be treated with a heregulin protein in order to induce epithelial cell growth and/or proliferation in the sample thereby expanding the population of primary epithelial cells. Typically, heregulin will be added to the *in vitro* epithelial cell culture at a concentration of about 0.1 to about 100 nM preferably 1-50 nM. If desired, the primary epithelial cells may be cultured *in vitro* for several generations in order to sufficiently expand the epithelial cell population. The epithelial cells are cultured under conditions suitable for mammalian cell culture as discussed above. After expansion, the expanded sample is reintroduced into the mammal for the purpose of re-epithelializing the mammalian tissue. For example, lung epithelial cells isolated from a patient having emphysema or chronic obstructive pulmonary disease may be obtained, expanded and reintroduced into the lung in order to more quickly re-epithelialize the damaged lung tissue thereby reestablishing lung function. The expanded cells may be reintroduced into the lung by aspiration or intubation using methods well known in the art.

The methods and procedures described herein with respect to HRG-α or HRG in general may be applied similarly to other HRG such as HRG-β1, HRG-β2 and HRG-β3 and to variants thereof, as well as to the antibodies. The following examples are offered by way of illustration and not by way of limitation.

### EXAMPLES

### Example 1 - Preparation of Heregulins

(a) Heregulins HRG-α, HRG-β1, HRG-β2, HRG-β2-like, and HRG-β3 were isolated, cloned, expressed and isolated from the cell culture medium as described in U.S. 5,367,060.
(b) SMDF polypeptides are prepared as described in WO 96/15244.
(c) γ-HRG polypeptide was prepared and characterized as described below.

*Reagents*: The EGF-like domain of HRGβ1₍₁₇₇₋₂₄₄₎ was expressed in *E. coli*, purified and radioiodinated as described previously (Sliwkowski *et al. J. Biol. Chem*. 269:14661-14665 (1994)). The anti-HER2 monoclonal antibodies 2C4 and 4D5 have been described elsewhere (Fendly *et al. Cancer Research* 50:1550-1558 (1990)).

*HER3 and HER4-immunoadhesins*: A unique M1 I site was engineered into a plasmid expressing human IgG heavy chain at the region encoding the hinge domain of the immunoglobulin. M1 I sites were also engineered into a set of HER expression plasmids at the region encoding the ECD/TM junctions of these receptors. All mutageneses were done using the Kunkel method (Kunkel, T., *Proc. Natl. Acad. Sci. U.S.A.* 82:488 (1985)). The M1 I sites were utilized to make the appropriate HER-IgG fusion constructs. The fusion junctions of the various HER-IgG chimeras were: for HER2, E⁶⁴⁶_{HER2}-(TR)-DKTH²²⁴_{VH}; for HER3, L⁶³⁶_{HER3}-(TR)-DKTH²²⁴_{VH}; for HER4, G⁶⁴⁰_{HER4}-(TR)-DKTH²²⁴_{VH}. The conserved TR sequence is derived from the M1 I site. The final expression constructs were in a pRK-type plasmid backbone wherein eukaryotic expression is driven by a CMV promoter (Gorman *et al*., *DNA Prot. Eng. Tech*. 2:3-10 (1990)).

To obtain protein for *in vitro* experiments, adherent HEK-293 cells were transfected with the appropriate expression plasmids using standard calcium phosphate methods (Gorman *et al., supra* and Huang *et al., Nucleic Acids Res*. 18:937-947 (1990)). Serum-containing media was replaced with serum-free media 15 hours post-transfection and the transfected cells incubated for 5-7 days. The resulting conditioned media was harvested and passed through Protein A columns (1 mL Pharmacia HiTrap™). Purified IgG fusions were eluted with 0. 1 M citric acid (pH 4.2) into tubes containing 1 M Tris pH 9.0. The eluted proteins were subsequently dialyzed against PBS and concentrated using Centri-prep-30 filters (Amicon). Glycerol was added to a final concentration of 25% and the material stored at -20 C. Concentrations of material were determined via a Fc-ELISA.

*Cell Culture*: Human breast cancer cell lines MDA-MB-175, MDA-MB-231, SK-BR-3 and MCF7 were obtained from the American Type Culture Collection and maintained in a 50:50 mixture of F12 Ham's and Dulbecco's modified Eagle medium (DMEM), supplemented with 10% heat inactivated FBS, 2 mM glutamine and 10% penicillin-streptomycin.

*Generation and Characterization of cDNA Library*: Total RNA was purified from MDA-MB-175 cells using the guanidinium isothiocyanate-cesium chloride procedure (Sambrook *et al., Molecular Cloning: A Laboratory Manual* (New York: Cold Spring Harbor Laboratory Press, (1989)). Poly (A)⁺ RNA was isolated using oligo (dT) Dynabeads (DYNAL) as recommended by the supplier. First and second strand syntheses were performed using a Gibco BRL cDNA synthesis kit. λgt10 cDNA recombinants were generated when a cDNA cloning system from Amersham was used. *In vitro* packaging was performed using Gigapack II packaging extract (Stratagene). PstI-XhoI HRGβ3 cDNA fragment (nt 144-618) was labeled by random priming and 1 x 10⁶ plaques were screened. Positive clones were confirmed and purified by secondary and tertiary screening. Phage DNA was isolated as a BamHI fragment and subcloned into the corresponding site of pBluescript SK. Clone 5 was completely sequenced using the Sequenase version 2.0 DNA sequencing kit (United States Biochemicals, Inc.). Both strands were sequenced.

*Bacterial Expression System*: A cDNA fragment of clone 5 (nt 1690- 2722) was subcloned into the pET-32 TRX fusion vector (Novagen). This BglII-BglII fragment was inserted into the BamHI site of the pET32a plasmid. The trxγ-HRG (amino acids 455-768) protein expression in *E.coli* was induced as recommended by the supplier.

*Purification of Recombinant γ-HRG: E.coli* cells expressing trxγ-HRG were collected and suspended at 9 ml/g in 50 mM Tris HCL pH 8. Lysozyme was added to a final concentration of 0.2 mg/ml and the solution was stirred on ice for 1 hr. Dnase I (10 µg/ml) and MgCl₂ (4 mM) were added. The solution was then sonicated for 30 min and cell pellets collected afterwards. The pellet fraction was dissolved at 250 ml/g in 6 M Gdn HCL, 0.1 M Tris HCL, pH 8.8. Solubilized proteins were sulfitolyzed by adding 1/10 volume of 1 M Na₂SO₃ and 1/10 volume of 0.2 M Na₂S₄O₆. The reaction was allowed to proceed for 1.5 hours at room temperature and protein was purified by gel filtration chromatography using a High Load Superdex™ 75 prep grade column (Pharmacia). Refolding was initiated by the addition of 1 mM cysteine, and 10 mM methionine was added as an antioxidant and incubated overnight at room temperature. Protein concentration was determined by quantitative amino acid analysis.

*Northern and Southern Hybridization*: Total RNA was isolated by the method of Chomczynski *et al. Anal Biochem*. 162:156-159 (1987). Poly (A)⁺ was isolated using oligo d(T) cellulose columns (Qiagen) as recommended by the supplier. RNA was denatured and size fractioned in a 0.8% formaldehyde/ 1% agarose gel and transferred onto nylon membrane (Hybond. Amersham). RNA was UV crosslinked (UV Stratalinker, Stratagene). Prehybridization was carried out at 42 C in 50 %formamide/ 1% SDS/ 1 M NaCl, 10 % dextransulfate and 100 µg/ml herring sperm DNA for at least 2 hours. cDNA probes using either a restriction fragment with complementary sequence to the EGF-like domain of HRGβ3 or a KpnI-AvaII cDNA fragment encoding the unique sequence of γ-HRG (nt 1238-1868) were radiolabeled by random priming (Prime-It II, Stratagene). Hybridiziation was done in equal solution at 42°C containing the ³²P labeled fragments for 16 hr. Blots were washed several times with 2 x SSC/ 1% SDS at room temperature, washed with the same solution at 65°C for 20 min and finally washed with 0.2 x SSC/ 0.1% SDS at room temperature for 15 min. The blots were air dried and exposed to Du Pont Reflection™ film with intensifying screens at -80°C for 7-40 hours. Human multiple tissue Northern blots (Clontech) containing 2 µg poly (A)⁺ from spleen, thymus, prostate, testis, ovary, small intestine, colon, peripheral blood leukocytes, heart, brain, placenta, lung, liver, skeletal muscle, kidney and pancreas were hybridized with a radiolabeled γ-HRG cDNA probe (nt 841-1447) as recommended by the supplier.

MDA-MB-175 and MDA-MB-231 genomic DNA was isolated as described in Sambrook *et al*, *supra*. DNA was digested with different restriction enzymes, prior to transfer treated with 0.25 N HCl and transferred onto nylon membrane (Hybond, Amersham). BglII-Ndel cDNA fragment of γ-HRG (nt 1690-2351) was also radiolabeled by random priming and used as a hybridization probe. Prehybridization was carried out in 6 x SSC/ 5 x Denhardt's/ 0.75% SDS, 10% Dextransulfate and 100 µg/ml herring sperm DNA at 68 C for 4 hours and hybridization with radiolabeled probe was done overnight. The same wash conditions as for Northern blots were used except a wash step with 0.2 x SSC/ 0.1 % SDS at 68°C was added and detection was pursued as described above.

^{*125*}*I-HRG Binding Assay*: Binding assays were performed in Nunc breakapart strip wells. Plates were coated at 4°C overnight with 100 µl of 5 µg/ml goat-anti-human antibody (Boehringer Mannheim) in 50 mM carbonate buffer (pH 9.6). Plates were rinsed twice with wash buffer (PBS/ 0.05% Tween-20 ) and blocked with 100 µl 1% BSA/ PBS for 30 min. Buffer has removed and each well was incubated with 15 ng IgG fusion protein in 1% BSA/ PBS under vigorous shaking for 1.5 hours. Plates were rinsed three times with wash buffer and competitive binding was carried out by adding various amounts of γ-HRG and ¹²⁵I-HRGβ1 under vigorous shaking. After incubation for 1.5-2 hours, wells were rinsed three times with wash buffer, drained and individual wells were counted using a 100 Series Iso Data γ-counter.

*Tyrosine Phosphorylation Assay*: MCF7 cells were plated in 24 well plates at 1 x 10⁵ cells/ well in F12/DMEM containing 10% FBS. After 48 hours, cells were washed with serum free F12/DMEM and serum starved for 6 hours. Various concentrations of bacterial expressed truncated γ-HRG (*i*.*e*., 0 pM, 22 pM, 66 pM, 200 pM and 600 pM trxγ-HRG) or unpurified conditioned medium of MDA-MB-175 cells were prepared in binding buffer (0.1% BSA in F12/DMEM) and added to each well. After 8 min incubation at room temperature. media was carefully aspirated and reactions were stopped by adding 100 µl of sample buffer (5% SDS, 0.25% 2-mercaptoethanol, 25 mM Tris-HCL pH 6.8). 20 µl of each sample was size fractionated in a 4-12% gradient gel (Novex) and then electrophoretically transferred onto nitrocellulose membrane. Antiphosphotyrosine (4G10, from UBI, used at 1 µg/ml) immunoblots were developed and the predominant reactive band at Mᵣ∼180 kDa was quantified by reflectance densitometry.

*Production and characterization of conditioned medium from MDA-MB-175 cells*. Cells were seeded in T175 flasks and grown until reaching 70-80% confluency ( ∼ 2.5 x 10⁷ cells/ flask). Subsequently, cells were washed with PBS and grown in serum free F12/ DMEM medium for 3-4 days. Medium was then collected, filtered and concentrated using an ultrafiltration cell with YM10 Diaflo ultafiltration membranes (Amicon). γ-HRG was visualized in conditioned medium of MDA-MB-175 cells by Western blot analysis under non reducing conditions. γ-HRG was partially purified by HPLC using a C4 reverse phase column. CHO expressed full length HRGβ1 (lane 1) and semi pure γ-HRG (lane 2) were electrophoresed, blot was probed with HER2/HER4 IgG heterodimers and Western blot was developed. A ∼ 64 kDa band could be seen in the lane containing partial purified supernatant whereas CHO expressed full length HRGβ1 migrated as a 45 kDa protein.

*Cell Proliferation Assay with Crystal Violet*: Tumor cell lines were plated in 96 well plates at following densities: 2 x 10⁴ cells/well for MDA-MB-175 and 1 x 10⁴ cells/well for SK-BR-3. The media contained 1% FBS and cells were allowed to adhere for 2 hours. Monoclonal antibodies, immunoadhesions (10 µg/ml) or media alone were added and the cells were incubated for 2 hours at 37°C. rHRGβ1₁₇₇₋₂₄₄ was added at a final concentration of 1 nM, or 100 nM for neutralising the immunoadhesion, and the cells were incubated for 4 days. Monolayers were washed with PBS and stained/fixed with 0.5% crystal violet. Plates were air dried, the dye was eluted with 0.1 M sodium citrate (pH 4.2) in ethanol (50:50) and the absorbance was measured at 540 nm.

*Isolation and sequence analysis of γ-HRG:* To characterize the heregulin transcript in MDA-MB-175 cells, a λgt 10 cDNA library was constructed with mRNA derived from this ceil line. The library was screened with a cDNA probe corresponding to the EGF-like domain and part of the N-terminal sequence of HRGβ3. Various clones were identified. One of the clones which appeared to contain the full length cDNA sequence was isolated and sequenced. Fig.7A-7D shows the nucleotide sequence and the predicted amino acid sequence of γ-HRG. The single open reading frame of 2303 bp starts with an ATG codon at nt 334. This start codon lies in a nucleotide sequence context, which is known to be a potential translation initiation site (Kozak, *Nucleic Acid Research* 15:8125-8148 (1987)). Several termination codons were found upstream of the initiation codon. The stop codon TAG at nt 2637 is followed by the 3' noncoding sequence, which is identical to other HRG isoform sequences and includes a polyadenylation signal followed by an A-rich region. The open reading frame encodes a protein of 768 amino acid residues with a calculated molecular mass of 84.2 kDa.

(d) The selection of HRG-β1 variants containing residues corresponding to the minimal EGF-like domain (HRG-β1 177-228) was conducted using monovalent phage display. For these variants, residue numbers also are expressed, in parentheses, in terms of the position of the residue in the minimal EGF-like domain (i.e., HRG-β1 EGF 1-52).

Variants of HRG-β1 EGF were prepared and selected for binding to HER-3-Ig using monovalent phage display, according to the method of Bass *et al*., *Proteins* 8:309-314 (1990). As discussed in detail below, an HRG-β1 EGF phagemid vector was prepared, in which HRG-β1 EGF was fused to a C-terminal fragment of the M13 coat protein pIII. Kunkel mutagenesis was performed to introduce stop codons into this vector at sites selected for randomization. This step ensures that the starting vector is incapable of expressing the wild-type polypeptide. Stretches of four to six residues per library were randomized in a linear fashion, except for the six cysteines, Phe189 (HRG-β1 EGF Phe13) and the two most C-terminal residues. Phe189 was not altered because this residue is conserved as an aromatic residue in EGF and TGF-α and forms a stacking interaction with Tyr208 (HRG-β1 EGF Tyr32) Jacobsen *et al*., *Biochemistry* 35:3402-17 (1996). HRG-β1 EGF was thus covered in eight libraries, designated A-E, G, H and I.

Library E, covering HRG-β1 202-209 (HRG-β1 EGF 26-33), contained a three-residue deletion. The deleted region corresponds to a disordered turn between the second and third β-sheet of HRG-β1 EGF, and the equivalent amino acids are absent in EGF and TGF-α. An HRG-β1 EGF control variant in which HRG-β1 202-204 (HRG-β1 EGF 26-28) of HRG8 are deleted (HRG63) bound HER-3-Ig with an affinity similar to that of wild-type.

An additional library (F) was created to randomize a surface patch composed of side chains from the first and second β-sheets, which included HRG-β1 178, 180, 198, and 200 (HRG-β1 EGF 2, 4, 22, and 24).

The selected sites in the starting vectors were randomized by Kunkel mutagenesis to produce HRG-β1 EGF libraries. Phage displaying mutated HRG-β1 EGFs were produced from the libraries under conditions such that, statistically, each phage particle displayed no more than one copy of the mutated HRG-β1 EGF. See Bass *et al., supra*. These phage were then selected for binding to (sorted against) HER-3-Ig immobilized on an ELISA plate. Bound phage were eluted and used to reinfect host cells, which were used to produce new phage for another round of sorting. This process was repeated six to seven times for each library. Twelve clones from the phage selected from each library were then sequenced.

**Table 2:**

| Library A Variants | | | | | |
|---|---|---|---|---|---|
| | Position in HRG-β1 | | | | |
| Variant No. | 177 | 178 | 179 | 180 | 181 |
| Wild-type | S | H | L | V | K |
| 1 | W | R | . | . | P |
| 2 | W | S | . | Q | P |
| 3, 5, 10 | W | E | . | . | P |
| 4 | W | S | . | . | . |
| 6 | W | S | . | I | P |
| 7 | W | R | . | . | A |
| 8 | W | A | . | . | P |
| 9 | W | S | . | Q | . |
| 11 | W | E | . | . | A |
| 12 | W | S | . | E | P |

**Table 3:**

| Library B Variants | | | | | | |
|---|---|---|---|---|---|---|
| | Position in HRG-β1 | | | | | |
| Variant No. | 183 | 184 | 185 | 186 | 187 | 188 |
| Wild-type | A | E | K | E | K | T |
| 1* | G | V | G | R | D | G |
| 2* | G | G | E | R | E | G |
| 3 | G | . | E | R | E | G |
| 4*, 5* | G | W | D | R | E | G |
| 6* | G | V | Q | R | E | G |
| 7 | G | . | E | R | A | G |
| 8 | G | K | E | R | E | G |
| 9* | T | N | S | R | E | G |
| 10* | D | K | S | R | E | G |
| 11* | G | . | D | R | . | Q |
| 12 | G | R | E | R | E | G |

| | | | | | | |
|---|---|---|---|---|---|---|
| *Variant also contained Met226Ile. | | | | | | |

**Table 4:**

| Library C Variants | | | | | |
|---|---|---|---|---|---|
| | Position in HRG-β1 | | | | |
| Variant No. | 191 | 192 | 193 | 194 | 195 |
| Wild-type | V | N | G | G | E |
| 1,2,4,5,7-12 | . | . | . | . | . |
| 3 | . | . | . | . | V |
| 6 | . | . | . | . | Q |

**Table 5:**

| Library D Variants | | | | | |
|---|---|---|---|---|---|
| | Position in HRG-β1 | | | | |
| Variant No. | 197 | 198 | 199 | 200 | 201 |
| Wild-type | F | M | V | K | D |
| 1*,2*,8*,12* | Y | K | . | R | I |
| 3 | . | R | . | . | T |
| 4,5,7,9 | Y | R | . | . | T |
| 6 | Y | . | I | . | Y |
| 10 | Y | . | . | . | T |
| 11 | M | R | . | R | T |

| | | | | | |
|---|---|---|---|---|---|
| *Variant also contained Met226Ile. | | | | | |

**Table 6:**

| Library E Variants | | | | | |
|---|---|---|---|---|---|
| | Position in HRG-β1 | | | | |
| Variant No. | 205 | 206 | 207 | 208 | 209 |
| Wild-type | P | S | R | Y | L |
| 1 | T | P | Y | L | M |
| 2,4 | Y | G | Y | L | M |
| 3* | Y | R | Y | R | M |
| 5,12 | T | H | Y | R | G |
| 6 | T | H | Y | R | M |
| 7* | Y | K | Y | R | M |
| 8,9 | T | K | Y | R | G |
| 10 | Y | K | Y | R | . |

| | | | | | |
|---|---|---|---|---|---|
| *Variant also contained Met226Ile. | | | | | |

**Table 7:**

| Library G Variants | | | | | | |
|---|---|---|---|---|---|---|
| | Position in HRG-β1 | | | | | |
| Variant No. | 211 | 212 | 213 | 214 | 215 | 216 |
| Wild-type | K | C | P | N | E | F |
| 1, 5, 6, 10, 12 | R | . | S | L | . | . |
| 2 | R | . | S | E | . | . |
| 3 | . | . | . | K | . | M |
| 4 | R | . | T | V | . | Y |
| 7,8 | R | . | T | V | . | Y |
| 9 | . | . | N | S | . | . |
| 11 | R | . | K | K | . | . |

### Example 2 - HER2/HER3 expression in embryonic rat lung

Rat lungs were microdissected from rat embryos on embryologic day (E) 16, 18, 20, and post-natal (P) days 7, 14, and adult. Isolated lung tissue was homogenized in a standard protease inhibitor buffer, and equal protein amounts subjected to SDS-PAGE (4-20%), biotted to nitrocellulose and identified with specific antibodies (HER2, HER3, HER4-Santa Cruz Biological, San Jose, CA. and HRG, 3G11, Genentech, Inc.) using chemiluminescent techniques.

Analysis of the blot indicates that HER2 is expressed at high levels throughout development in utero, appears to peak on E18 and declines after birth to lower adult levels. HER3 expression peaks at E18, and then declines after birth to lower adult levels. HER4 is not identified at any time during lung development. A preform of HRG may be present (75 kDa protein) on E16, peaking on E18 and then declining to low levels in the adult. These data suggest that the HER/HRG system is developmentally modulated during lung development and the active receptor may be a HER2/HER3 heterodimer. The time period during which the receptors peak in expression (E18) represents the pseudoglandular stage of rat lung development (days 13-18), bordering on the canalicular stage (days 19-20). During the pseudoglandular stage, pulmonary epithelial cell proliferation is higher than at any other time. During the canalicular stage, differentiation begins with the appearance of type I pneymocyte and type II (surfactant producing) pneymocytes. This indicates a role for HRG/HER interaction in either or both processes.

### Example 3 - HER2/HER3 expression in fetal human lung

Fetal human lung was obtained from mid trimester embryos (17-22 weeks). Lung tissue was cultured in serum free Weymouth's media at an air fluid interface at 37°C in a humidified 5% CO₂ atmosphere. Tissue was harvested from the culture on days 0 (day tissue was received) and after 1-4 days (D) in culture (D1-D4), homogenized in a standard protease inhibitor buffer, equal protein amounts subjected to SDS-PAGE (4-20%), blotted to nitrocellulose and identified with specific antibodies.

HER2 is expressed on D0 and increases in expression level during *in vitro* culture. HER3 is present at low to undetectable levels at D0 and increases in expression level during *in vitro* culture. HER4 was not identified at any time during lung development. HRG was not identified on D0, however, it was identified as a 75,000 Da protein on D1-D2 and continued to rise in expression level throughout time in culture. This human explant model recapitulates part of the normal lung developmental program over the 5 days in culture. Development occurs rapidly with both epithelial cell proliferation, and differentiation occurring, along with air space formation. These data indicate that the HER/HRG system is also modulated during *in vitro* human lung development, and the active receptor may be a HER2/HER3 heterodimer. The third trimester represents late pseudoglandular (days 42-112) and canalicular stage (days 112-196) in human lung development. As in the rat, during the pseudoglandular stage epithelial cell proliferation and formation of the prospective airways occurs. During the canalicular stage differentiation of the epithelium occurs.

### Example 4 - Expression of HER2 and HER3 in human lung

HER2 and HER3 are expressed exclusively in the pulmonary epithelium during lung development. Mid-trimester human lung was cultured *in vitro* as outlined above. Tissue was harvested daily, snap frozen, and 5 micron sections cut. The sections were mounted on glass slides, and immunohistochemistry performed using standard ABC procedures. HER2, HER3, and HER4 were identified using specific antibodies as described above.

As a control, lung tissue was stained at D0 and D5 with an irrelevant antibody and no staining was detected. At D0, the lung is relatively unformed. The majority of the tissue is mesenchymal cells. Early air spaces are being developed. On D5, air spaces are clearly identifiable, with thinning of the mesenchymal tissue and proliferation of the lung epithelium required to cover the enlarging air spaces.

Using HER2 staining at D0 and D5, it was established that HER 2 is uniformly present on D0 lung tissue throughout the lung epithelial tissue. No expression was present in the mesenchymal tissue. By D5, HER2 remains uniform and restricted to the pulmonary epithelium.

Using HER3 staining control at D0 and D5, HER3 was identifiable in D0 lung tissue. The staining was relatively less than HER2, and was not homogenous, suggesting that there are specific epithelial areas expressing the HER3 receptor. By D5, expression had become more homogenous throughout the lung epithelium, but clearly not uniform. Expression remained epithelium specific.

### Example 5 - Preparation of rHRGβ1₁₇₇₋₂₄₄

The EGF-like domain fragment HRG-β1 177-244 was amplified from vector pHL89 (which is described in Holmes et al., *Science* 256:1205-1210 (1992)) by PCR with primers having NsiI/Xbal- containing overhangs. The fragment was inserted into phagemid display vector pam-g3 by restriction digest-ligation at the same sites to generate construct pHRG2-g3 (177-244). pam-g3 was a derivative of phGHam-g3, which was designed for phage display of human growth hormone (hGH) and was described in Lowman et al., *Biochemistry* 30:10832-10838 (1991), pam g3 was produced by removing the hGH gene present in phGHam-g3 and replacing this gene with a stuffer fragment, which provides space for cleavage at the restriction sites used for cloning. The HRG-β1 fragment was attached to residue 247 of pIII.

The HRG-β1 EGF-like domain expressed from the above-described construct is designated by removing the "p" and the "-g3" that appear in the name of the construct. Thus, the HRG-β1 EGF-like domain expressed from the pHRG2-g3 construct is designated "HRG2."

The domain was displayed monovalently on phage as a pIII fusion protein, as described by Bass et al., *Proteins* 8:309-314 (1990).

Similarly, variants HRG-β1₁₄₇₋₂₂₇, HRG-β1₁₄₇₋₂₄₄, and HRG-β1₁₇₇₋₂₂₇ were prepared and expressed as described above.

### Example 6 - rHRGβ1₁₇₇₋₂₄₄ causes accelerated lung development

Exogenous rHRGβ1₁₇₇₋₂₄₄ caused accelerated lung development *in vitro*. To determine if the expressed HER2/HER3 receptors were functional and the role of HRG stimulation during lung development *in vitro*, rHRGβ1₁₇₇₋₂₄₄ was added to the *in vitro* culture at 10 nM. Tissue was harvested at D5, snap frozen, and 5 micron sections were cut for analysis.

The morphology, in comparison to the untreated control specimens was grossly different. There was marked proliferation of the epithelium. Air spaces which are typically lined with a single cell layer now had a 2-3 cell thickness. The changes were dose dependent with more epithelial cell response with higher concentrations. HER2 and HER3 were still identifiable in the epithelium only.

### Example 7 - Human lung differentiation

Differentiation of human lung epithelial cells occurs after HRG treatment. Differentiation was measured by Surfactant Protein A (SPA) production. All sections were stained for SPA. Human lung explant stained for SPA with stain localizing in epithelial cells of the prealveolar ducts. Human lung explant exposed to 10 nM HRG showed an effect on SPA production. As a differentiation control, a lung explant was exposed to 1mM dibutyryl cAMP. A negative control was also run.

## Claims

1. Use of an isolated HER2, HER3 and/or HER4 activating ligand which is a heregulin (HRG) polypeptide, HRG variant, HRG agonist antibody or fragment thereof capable of binding to the HER2, HER3 and/or HER4 receptor for manufacture of a medicament (a) for treating chronic obstructive pulmonary disease, or (b) for treating respiratory distress or emphysema, or (c) for treating cystic fibrosis.

2. The use according to Claim 1, wherein the activating ligand is capable of binding to human HER2, HER3 and/or HER4 receptor.

3. The use according to Claim 1, wherein the activating ligand is human HRG or a fragment thereof.

4. The use according to Claim 1, wherein the activating ligand is selected from the group consisting of HRG-α, -β1, -β2, -β2-like, and -β3 and fragments thereof.

5. The use according to Claim 1, wherein the activating ligand is γ-HRG or a fragment thereof.

6. The use according to Claim 1, wherein the activating ligand is a recombinant human HRG or a fragment thereof.

7. The use according to Claim 6, wherein the HRG is rHRG-β1-177-244.

8. The use according to Claim 1, wherein the activating ligand is an agonist antibody.

9. The use according to any one of the previous claims, wherein the epithelial cell is a lung cell.

10. The use according to any one of the previous claims, wherein the epithelial cell expresses HER2/HER3, HER2/HER4, HER3/HER4, HER3 or HER4.

11. The use according to any of the previous claims, wherein the activating ligand is to be administered to a patient in need thereof at a daily dose of about 1µg/kg to 100 mg/kg.

12. The use according to claim 1, wherein the activating ligand is a HRG variant.

13. The use according to claim 12, wherein the HRG variant has at least one amino acid substitution at a residue corresponding to a residue of 645-amino acid native human heregulin-β1, selected from: S177, H178, L179, V180, K181, E184, E186, K187, T188, V191, N192, G193, G194, E195, M198, V199, K200, D201, N204, P205, S206, R207, Y208, L209, K211, P213, N214, E215, T217, G218, D219, Q222, N223, Y224, S228, and F229.

14. The use according to claim 12, wherein the HRG variant is a heregulin-β1 variant, which includes one or more amino acid substitutions selected from:
a) S177W;
b) H178S, E, R, or A;
c) V180Q, I, or E;
d) K181P or A;
e) A183G;
f) E184V, W, K, R, G, or N;
g) K185E, S, Q, or G;
h) E186R;
i) K187E or A;
j) T188Q;
k) E195Q;
l) F197Y;
m) M198R or K;
n) K200R;
o) D201T or I;
p) P205T or Y;
q) S206K, H, G, P, or R;
r) R207Y;
s) Y208R or L;
t) L209M or G;
u) K211R;
v) P213S, T, N, or K;
w) N214L, K, S, or E;
x) F216M;
y) N223H or W; and
z) M226I.

15. The use according to claim 14, wherein the HRG variant comprises a set of amino acid substitutions, and wherein the set of amino acid substitutions is selected from the group consisting of:
a) A183G, E184W, K185D, E186R, K187E, T188G, and M226I;
b) A183D, E184K, K185S, E186R, K187E, T188G, and M226I;
c) F197Y, M198K, K200R, D201I, and M226I;
d) P205Y, S206G, R207Y, Y208L, and L209M;
e) P205Y, S206R, R207Y, Y208R, L209M, and M226I;
f) P205T, S206H, R207Y, Y208R, and L209M;
g) P205T, S206K, R207Y, Y208R, and L209G;
h) N223W and M226I;
i) N223H and M226I;
j) S177W, H178E, K181P, A183G, E184W, K185D, E186R, K187E, T188G, and M226I;
k) P205Y, S206G, R207Y, Y208L, L209M, and M226I;
l) A183G, K185E, E186R, K187E, T188G, F197Y, M198R, D201T;
m) A183G, K185E, E186R, K187E, T188G, P205Y, S206G, R207Y, Y208L, and L209M;
n) A183G, K185E, E186R, K187E, T188G, F197Y, M198R, D201T, P205Y, S206G, R207Y, Y208L, and L209M;
o) A183G, K185E, E186R, K187E, T188G, and M226I;
p) F197Y, M198R, D201T, P205Y, S206G, R207Y, Y208L, and L209M;
q) F197Y, M198R, D201T, P205Y, S206G, R207Y, Y208L, L209M, and M226I;
r) F197Y, M198R, D201T, and M226I;
s) A183G, K185E, E186R, K187E, T188G, F197Y, M198R, D201T, and M226I;
t) A183G, K185E, E186R, K187E, T188G, P205Y, S206G, R207Y, Y208L, L209M, and M226I;
u) A183G, K185E, E186R, K187E, T188G, F197Y, M198R, D201T, P205Y, S206G, R207Y, Y208L, L209M, and M226I;
v) F197Y, M198R, D201T, P205Y, S206G, R207Y, Y208L, L209M, N223H, and M226I; and
w) A183G, K185E, E186R, K187E, T188G, F197Y, M198R, D201T, P205Y, S206G, R207Y, Y208L, L209M, N223H, and M226I.

16. The use according to claim 1, wherein the activating ligand is sensory and motor neuron derived factor.

17. A method for producing an expanded sample of epithelial cells for re-introduction into a mammalian patient in need thereof, the method comprising the steps of:
(a) providing a normal epithelial cell sample, which has been obtained from said mammalian patient, and
(b) contacting the sample with an isolated ligand which is an HRG variant capable of binding to the HER2, HER3 and/or HER4 receptor and which activates HER2, HER3 or HER4 receptors or a combination thereof, to induce growth and/or proliferation of epithelial cells in the sample and thereby to obtain an expanded sample for re-introduction into the mammalian patient.

## Patentansprüche

1. Verwendung eines isolierten HER2-, HER3- und/oder HER4-Aktivierungsliganden, der ein Heregulin- (HRG-) Polypeptid, eine HRG-Variante, ein HRG-Agonist-Antikörper oder ein Fragment davon ist, der fähig ist, an den HER2-, HER3- und/oder HER4-Rezeptor zu binden, um ein Medikament (a) zur Behandlung chronischer obstruktiver Lungenleiden oder (b) zur Behandlung von Atemnot oder Emphysem oder (c) zur Behandlung von Mukoviszidose herzustellen.

2. Verwendung nach Anspruch 1, worin der Aktivierungsligand zur Bindung eines menschlichen HER2-, HER3- und/oder HER4-Rezeptors fähig ist.

3. Verwendung nach Anspruch 1, worin der Aktivierungsligand menschliches HRG oder ein Fragment davon ist.

4. Verwendung nach Anspruch 1, worin der Aktivierungsligand aus der aus HRG-α, -β1, -β2, -β2-Ähnlichen und -β3 sowie Fragmenten davon bestehenden Gruppe ausgewählt ist.

5. Verwendung nach Anspruch 1, worin der Aktivierungsligand γ-HRG oder ein Fragment davon ist.

6. Verwendung nach Anspruch 1, worin der Aktivierungsligand rekombinantes menschliches HRG oder ein Fragment davon ist.

7. Verwendung nach Anspruch 6, worin das HRG rHRG-β1-177-244 ist.

8. Verwendung nach Anspruch 1, worin der Aktivierungsligand ein Agonist-Antikörper ist.

9. Verwendung nach einem der vorangegangenen Ansprüche, worin die Epithelzelle eine Lungenzelle ist.

10. Verwendung nach einem der vorangegangenen Ansprüche, worin die Epithelzelle HER2/HER3, HER2/HER4, HER3/HER4, HER3 oder HER4 exprimiert.

11. Verwendung nach einem der vorangegangenen Ansprüche, worin der Aktivierungsligand einem Patienten, der diesen benötigt, in einer täglichen Dosis von etwa 1 µg/kg bis 100 mg/kg verabreicht werden soll.

12. Verwendung nach Anspruch 1, worin der Aktivierungsligand eine HRG-Variante ist.

13. Verwendung nach Anspruch 12, worin die HRG-Variante zumindest eine Aminosäuresubstitution an einem Rest aufweist, der einem Rest des 645 Aminosäuren langen nativen menschlichen Heregulin-β1 entspricht, ausgewählt aus:
S177, H178, L179, V180, K181, E184, E186, K187,
T188, V191, N192, G193, G194, E195, M198, V199,
K200, D201, N204, P205, S206, R207, Y208, L209,
K211, P213, N214, E215, T217, G218, D219, Q222,
N223, Y224, S228, und F229.

14. Verwendung nach Anspruch 12, worin die HRG-Variante eine Heregulin-β1-Variante ist, die eine oder mehrere Aminosäuresubstitutionen umfasst, die aus Folgenden ausgewählt sind:
a) S177W;
b) H178S, E, R, oder A;
c) V180Q, I, oder E;
d) K181P oder A;
e) A183G;
f) E184V, W, K, R, G, oder N;
g) K185E, S, Q,oder G;
h) E186R;
i) K187E oder A;
j) T188Q;
k) E195Q;
l) F197Y;
m) M198R oder K;
n) K200R;
o) D201T oder I;
p) P205T oder Y;
q) S206K, H, G, P, oder R;
r) R207Y;
s) Y208R oder L;
t) L209M oder G;
u) K211R;
v) P213S, T, N, oder K;
w) N214L, K, S, oder E;
x) F216M;
y) N223H oder W; und
z) M226I.

15. Verwendung nach Anspruch 14, worin die HRG-Variante eine Gruppe von Aminosäuresubstitutionen umfasst und worin die Aminosäuresubstitutionen aus der aus Folgenden bestehenden Gruppe ausgewählt sind:
a) A183G, E184W, K185D, E186R, K187E, T188G, und M226I;
b) A183D, E184K, K185S, E186R, K187E, T188G, und M226I;
c) F197Y, M198K, K200R, D201I, und M226I;
d) P205Y, S206G, R207Y, Y208L, und L209M;
e) P205Y, S206R, R207Y, Y208R, L209M, und M226I;
f) P205T, S206H, R207Y, Y208R, und L209M;
g) P205T, S206K, R207Y, Y208R, und L209G;
h) N223W und M226I;
i) N223H und M226I;
j) S177W, H178E, K181P, A183G, E184W, K185D, E186R, K187E, T188G, und M226I;
k) P205Y, S206G, R207Y, Y208L, L209M, und M226I;
l) A183G, K185E, E186R, K187E, T188G, F197Y, M198R, D201T;
m) A183G, K185E, E186R, K187E, T188G, P205Y, S206G, R207Y, Y208L, und L209M;
n) A183G, K185E, E186R, K187E, T188G, F197Y, M198R, D201T, P205Y, S206G, R207Y, Y208L, und L209M;
o) A183G, K185E, E186R, K187E, T188G, und M226I;
p) F197Y, M198R, D201T, P205Y, S206G, R207Y, Y208L, und L209M;
q) F197Y, M198R, D201T, P205Y, S206G, R207Y, Y208L, L209M, und M226I;
r) F197Y, M198R, D201T, und M226I;
s) A183G, K185E, E186R, K187E, T188G, F197Y, M198R, D201T, und M226I;
t) A183G, K185E, E186R, K187E, T188G, P205Y, S206G, R207Y, Y208L, L209M, und M226I;
u) A183G, K185E, E186R, K187E, T188G, F197Y, M198R, D201T, P205Y, S206G, R207Y, Y208L, L209M, und M226I;
v) F197Y, M198R, D201T, P205Y, S206G, R207Y, Y208L, L209M, N223H, und M226I; und
w) A183G, K185E, E186R, K187E, T188G, F197Y, M198R, D201T, P205Y, S206G, R207Y, Y208L, L209M, N223H, und M226I.

16. Verwendung nach Anspruch 1, worin der Aktivierungsligand ein von einem sensorischen und motorischen Neuron stammender Faktor ist.

17. Verfahren zur Herstellung einer erweiterten Probe von Epithelzellen zur Wiedereinbringung in einen Säugetierpatienten, der diese benötigt, wobei das Verfahren die folgenden Schritte umfasst:
(a) das Bereitstellen einer normalen Epithelzellenprobe, die von einem Säugetierpatienten erhalten wurde, und
(b) das Kontaktieren der Probe mit einem isolierten Liganden, der eine HRG-Variante ist, die zur Bindung an den HER2-, HER3- und/oder HER4-Rezeptor fähig ist und HER2-, HER3- und/oder HER4-Rezeptoren oder eine Kombination davon aktiviert, um Wachstum und/oder Proliferation von Epithelzellen in der Probe auszulösen und so eine erweiterte Probe zur Wiedereinbringung in den Säugetierpatienten zu erhalten.

## Revendications

1. Utilisation d'un ligand activant isolé de HER2, HER3 et/ou HER4 qui est un polypeptide d'héréguline (HRG), un variant de HRG, un anticorps agoniste de HRG ou son fragment capable de se lier au récepteur de HER2, HER3 et/ou HER4 pour la fabrication d'un médicament (a) pour le traitement d'une maladie pulmonaire obstructive chronique, ou (b) pour le traitement de la détresse respiratoire ou de l'emphysème, ou (c) pour le traitement de la fibrose kystique.

2. Utilisation selon la revendic ation 1, où le ligand activant est capable de se lier au récepteur de HER2, HER3 et/ou HER4 de l'humain.

3. Utilisation selon la revendication 1, où le ligand activant est une HRG humaine ou son fragment.

4. Utilisation selon la revendication 1, où le ligand activant est sélectionné dans le groupe consistant en HRG-α, -β1, -β2, ressemblant à -β2, et -β3 et leurs fragments.

5. Utilisation selon la revendication 1, où le ligand activant est γ-HRG ou un fragment.

6. Utilisation selon la revendication 1 , où le ligand activant est une HRG humaine recombinante ou son fragment.

7. Utilisation selon la revendication 6, où HRG est rHRG-β1-177-244.

8. Utilisation selon la revendication 1, où le ligand activant est un anticorps agoniste.

9. Utilisation sel on l'une quelconque des revendications précédentes, où la cellule épithéliale est une cellule de poumon.

10. Utilisation selon l'une quelconque des revendications précédentes, où la cellule épithéliale exprime HER2/HER3, HER2/HER4, HER3/HER4, HER3 ou HER4.

11. Utilisation selon l'une quelconque des revendications précédentes, où le ligand activant doit être administré à un patient le nécessitant à une dose quotidienne d'environ 1µg/kg à 100 mg/kg.

12. Utilisation selon la revendication 1, où le ligand activant est un variant de HRG.

13. Utilisation selon la revendication 12, où le variant de HRG a au moins une substitution d'acide aminé à un résidu correspondant à un résidu de l'acide aminé 645 de l'héréguline β1 native humaine sélectionné parmi :
S177, H178, L179, V180, K181, E184, E186, K187,
T188, V191, N192, G193, G194, E195, M198, V199,
K200, D201, N204, P205, S206, R207, Y208, L209,
K211, P213, N214, E215, T217, G218, D219, Q222,
N223, Y224, S228, and F229.

14. Utilisation selon la revendicat ion 12, où le variant de HRG est un variant d'héréguline-β1 qui contient une ou plusieurs substitutions d'acide aminé sélectionnées parmi :
a) S177W ;
b) H178S, E, R, ou A ;
c) V180Q, I ou E ;
d) K181P ou A ;
e) A183G ;
f) E184V, W, K, R, G, ou N ;
g) K185E, S, Q, ou G ;
h) E186R ;
i) K187E ou A ;
j) T188Q ;
k) E195Q ;
l) F197Y ;
m) M198R ou K ;
n) K200R ;
o) D201T ou I ;
p) P205T ou Y ;
q) S206K, H, G, P, ou R ;
r) R207Y ;
s) Y208R ou L ;
t) L209M ou G ;
u) K211R ;
v) P213S, T, N, ou K ;
w) N214L, K, S, ou E ;
x) F216M ;
y) N223H ou W ; et
z) M226I.

15. Utilisation selon la revendication 14, où le variant de HRG comprend un groupe de substitutions d'acides aminés et où le groupe de substitutions d'acides aminés est sélectionné dans le groupe consistant en :
a) A183G, E184W, K185D, E186R, K187E, T188G, et M226I ;
b) A183D, E184K, K185S, E186R, K187E, T188G, et M226I ;
c) F197Y, M198K, K200R, D201I, et M226I ;
d) P205Y, S206G, R207Y, Y208L, et L209M ;
e) P205Y, S206R, R207Y, Y208R, L209M, et M226I ;
f) P205T, S206H, R207Y, Y208R, et L209M ;
g) P205T, S206K, R207Y, Y208R, et L209G ;
h) N223W et M226I ;
i) N223H et M226I ;
j) S177W, H178E, K181P, A183G, E184W, K185D, E186R, K187E, T188G, et M226I ;
k) P205Y, S206G, R207Y, Y208L, L209M, et M226I ;
l) A183G, K185E, E186R, K187E, T188G, F197Y, M198R, D201T ;
m) A183G, K185E, E186R, K187E, T188G, P205Y, S206G, R207Y, Y208L, et L209M ;
n) A183G, K185E, E186R, K187E, T188G, F197Y, M198R, D201T, P205Y, S206G, R207Y, Y208L, et L209M ;
o) A183G, K185E, E186R, K187E, T188G, et M226I ;
p) F197Y, M198R, D201T, P205Y, S206G, R207Y, Y208L, et L209M ;
q) F197Y, M198R, D201T, P205Y, S206G, R207Y, Y208L, L209M, et M226I ;
r) F197Y, M198R, D201T, et M226I ;
s) A183G, K185E, E186R, K187E, T188G, F197Y, M198R, D201T, et M226I ;
t) A183G, K185E, E186R, K187E, T188G, P205Y, S206G, R207Y, Y208L, L209M, et M226I ;
u) A183G, K185E, E186R, K187E, T188G, F197Y, M198R, D201T, P205Y, S206G, R207Y, Y208L, L209M, et M226I ;
v) F197Y, M198R, D201T, P205Y, S206G, R207Y, Y208L, L209M, N223H, et M226I ; et
w) A183G, K185E, E186R, K187E, T188G, F197Y, M198R, D201T, P205Y, S206G, R207Y, Y208L, L209M, N223H, et M226I.

16. Utilisation selon la revendication 1, où le ligand activant est un facteur dérivé d'un neurone sensoriel et moteur.

17. Méthode pour la production d'un échantillon propagé de cellules épithéliales pour une réintroduction chez un patient ma mmalien le nécessitant, la méthode comprenant les étapes de :
(a) prévoir un échantillon de cellules épithéliales normales, qui a été obtenu dudit patient mammalien, et
(b) mettre l'échantillon en contact avec un ligand isolé qui est un variant de HRG capable de se lier au récepteur de HER2, HER3 et/ou HER4 et qui active les récepteurs de HER2, HER3 ou HER4 ou une combinaison pour induire la croissance et/ou la prolifération des cellules épithéliales dans l'échantillon et ainsi obtenir un échantillon propagé pour réintroduction chez le patient mammalien.


### Sequence Listing

<110> Genentech, Inc.
<120> Use of Heregulin as a Growth Factor
<130> P1145PCT
<141> 1999-02-03
<150> US 09/020,598
   <151> 1998-02-04
<160> 14
   <210> 1
   <211> 669
   <212> PRT
   <213> Homo sapiens
<400> 1
   Ala Arg Ala Pro Gln Arg Gly Arg Ser Leu Ser Pro Ser Arg Asp
    1               5                  10                  15
   Lys Leu Phe Pro Asn Pro Ile Arg Ala Leu Gly Pro Asn Ser Pro
                   20                  25                  30
   Ala Pro Arg Ala Val Arg Val Glu Arg Ser Val Ser Gly Glu Met
                   35                  40                  45
   Ser Glu Arg Lys Glu Gly Arg Gly Lys Gly Lys Gly Lys Lys Lys
                   50                  55                  60
   Glu Arg Gly Ser Gly Lys Lys Pro Glu Ser Ala Ala Gly Ser Gln
                   65                  70                  75
   Ser Pro Ala Leu Pro Pro Arg Leu Lys Glu Met Lys Ser Gln Glu
                   80                  85                  90
   Ser Ala Ala Gly Ser Lys Leu Val Leu Arg Cys Glu Thr Ser Ser
                   95                  100                 105
   Glu Tyr Ser Ser Leu Arg Phe Lys Trp Phe Lys Asn Gly Asn Glu
                   110                 115                 120
   Leu Asn Arg Lys Asn Lys Pro Gln Asn Ile Lys Ile Gln Lys Lys
                   125                 130                 135
   Pro Gly Lys Ser Glu Leu Arg Ile Asn Lys Ala Ser Leu Ala Asp
                   140                 145                 150
   Ser Gly Glu Tyr Met Cys Lys Val Ile Ser Lys Leu Gly Asn Asp
                   155                 160                 165
   Ser Ala Ser Ala Asn Ile Thr Ile Val Glu Ser Asn Glu Ile Ile
                       170                 175                 180
   Thr Gly Met Pro Ala Ser Thr Glu Gly Ala Tyr Val Ser Ser Glu
                   185                 190                 195
   Ser Pro Ile Arg Ile Ser Val Ser Thr Glu Gly Ala Asn Thr Ser
                   200                 205                 210
   Ser Ser Thr Ser Thr Ser Thr Thr Gly Thr Ser His Leu Val Lys
                   215                 220                 225
   Cys Ala Glu Lys Glu Lys Thr Phe Cys Val Asn Gly Gly Glu Cys
                   230                 235                 240
   Phe Met Val Lys Asp Leu Ser Asn Pro Ser Arg Tyr Leu Cys Lys
                   245                 250                 255
   Cys Gln Pro Gly Phe Thr Gly Ala Arg Cys Thr Glu Asn Val Pro
                   260                 265                 270
   Met Lys Val Gln Asn Gln Glu Lys Ala Glu Glu Leu Tyr Gln Lys
                   275                 280                 285
   Arg Val Leu Thr Ile Thr Gly Ile Cys Ile Ala Leu Leu Val Val
                   290                 295                 300
   Gly Ile Met Cys Val Val Ala Tyr Cys Lys Thr Lys Lys Gln Arg
                   305                 310                 315
   Lys Lys Leu His Asp Arg Leu Arg Gln Ser Leu Arg Ser Glu Arg
                   320                 325                 330
   Asn Asn Met Met Asn Ile Ala Asn Gly Pro His His Pro Asn Pro
                   335                 340                 345
   Pro Pro Glu Asn Val Gln Leu Val Asn Gln Tyr Val Ser Lys Asn
                   350                 355                 360
   Val Ile Ser Ser Glu His Ile Val Glu Arg Glu Ala Glu Thr Ser
                   365                 370                 375
   Phe Ser Thr Ser His Tyr Thr Ser Thr Ala His His Ser Thr Thr
                   380                 385                 390
   Val Thr Gln Thr Pro Ser His Ser Trp Ser Asn Gly His Thr Glu
                   395                 400                 405
   Ser Ile Leu Ser Glu Ser His Ser Val Ile Val Met Ser Ser Val
                   410                 415                 420
   Glu Asn Ser Arg His Ser Ser Pro Thr Gly Gly Pro Arg Gly Arg
                   425                 430                 435
   Leu Asn Gly Thr Gly Gly Pro Arg Glu Cys Asn Ser Phe Leu Arg
                   440                 445                 450
   His Ala Arg Glu Thr Pro Asp Ser Tyr Arg Asp Ser Pro His Ser
                   455                 460                 465
   Glu Arg Tyr Val Ser Ala Met Thr Thr Pro Ala Arg Met Ser Pro
                   470                 475                 480
   Val Asp Phe His Thr Pro Ser Ser Pro Lys Ser Pro Pro Ser Glu
                   485                 490                 495
   Met Ser Pro Pro Val Ser Ser Met Thr Val Ser Met Pro Ser Met
                   500                 505                 510
   Ala Val Ser Pro Phe Met Glu Glu Glu Arg Pro Leu Leu Leu Val
                   515                 520                 525
   Thr Pro Pro Arg Leu Arg Glu Lys Lys Phe Asp His His Pro Gln
                   530                 535                 540
   Gln Phe Ser Ser Phe His His Asn Pro Ala His Asp Ser Asn Ser
                   545                 550                 555
   Leu Pro Ala Ser Pro Leu Arg Ile Val Glu Asp Glu Glu Tyr Glu
                   560                 565                 570
   Thr Thr Gln Glu Tyr Glu Pro Ala Gln Glu Pro Val Lys Lys Leu
                   575                 580                 585
   Ala Asn Ser Arg Arg Ala Lys Arg Thr Lys Pro Asn Gly His Ile
                   590                 595                 600
   Ala Asn Arg Leu Glu Val Asp Ser Asn Thr Ser Ser Gln Ser Ser
                   605                 610                 615
   Asn Ser Glu Ser Glu Thr Glu Asp Glu Arg Val Gly Glu Asp Thr
                   620                 625                 630
   Pro Phe Leu Gly Ile Gln Asn Pro Leu Ala Ala Ser Leu Glu Ala
                   635                 640                 645
   Thr Pro Ala Phe Arg Leu Ala Asp Ser Arg Thr Asn Pro Ala Gly
                   650                 655                 660
   Arg Phe Ser Thr Gln Glu Glu Ile Gln
                   665             669
<210> 2
   <211> 2226
   <212> DNA
   <213> Homo sapiens
<400> 2
   gg  gcg cga gcg cct cag cgc ggc cgc tcg ctc tcc ccc 38
       Ala Arg Ala Pro Gln Arg Gly Arg Ser Leu Ser Pro
        1               5                  10
   tcg agg gac aaa ctt ttc cca aac ccg atc cga gcc ctt 77
   Ser Arg Asp Lys Leu Phe Pro Asn Pro Ile Arg Ala Leu
           15                  20                  25
   gga cca aac tcg cct gcg ccg aga gcc gtc cgc gta gag 116
   Gly Pro Asn Ser Pro Ala Pro Arg Ala Val Arg Val Glu
                   30                  35
   cgc tcc gtc tcc ggc gag atg tcc gag cgc aaa gaa ggc 155
   Arg Ser Val Ser Gly Glu Met Ser Glu Arg Lys Glu Gly
       40                  45                  50
   aga ggc aaa ggg aag ggc aag aag aag gag cga ggc tcc 194
   Arg Gly Lys Gly Lys Gly Lys Lys Lys Glu Arg Gly Ser
               55                  60
   ggc aag aag ccg gag tcc gcg gcg ggc agc cag agc cca 233
   Gly Lys Lys Pro Glu Ser Ala Ala Gly Ser Gln Ser Pro
   65                  70                  75
   gcc ttg cct ccc cga ttg aaa gag atg aaa agc cag gaa 272
   Ala Leu Pro Pro Arg Leu Lys Glu Met Lys Ser Gln Glu
           80                  85                  90
   tcg gct gca ggt tcc aaa cta gtc ctt cgg tgt gaa acc 311
   Ser Ala Ala Gly Ser Lys Leu Val Leu Arg Cys Glu Thr
                   95                  100
   agt tct gaa tac tcc tct ctc aga ttc aag tgg ttc aag 350
   Ser Ser Glu Tyr Ser Ser Leu Arg Phe Lys Trp Phe Lys
       105                 110                 115
   aat ggg aat gaa ttg aat cga aaa aac aaa cca caa aat 389
   Asn Gly Asn Glu Leu Asn Arg Lys Asn Lys Pro Gln Asn
               120                 125
   atc aag ata caa aaa aag cca ggg aag tca gaa ctt cgc 428
   Ile Lys Ile Gln Lys Lys Pro Gly Lys Ser Glu Leu Arg
   130                 135                 140
   att aac aaa gca tca ctg gct gat tct gga gag tat atg 467
   Ile Asn Lys Ala Ser Leu Ala Asp Ser Gly Glu Tyr Met
           145                 150                 155
   tgc aaa gtg atc agc aaa tta gga aat gac agt gcc tct 506
   Cys Lys Val Ile Ser Lys Leu Gly Asn Asp Ser Ala Ser
               160                 165
   gcc aat atc acc atc gtg gaa tca aac gag atc atc act 545
   Ala Asn Ile Thr Ile Val Glu Ser Asn Glu Ile Ile Thr
       170                 175                 180
   ggt atg cca gcc tca act gaa gga gca tat gtg tct tca 584
   Gly Met Pro Ala Ser Thr Glu Gly Ala Tyr Val Ser Ser
               185                 190
   gag tct ccc att aga ata tca gta tcc aca gaa gga gca 623
   Glu Ser Pro Ile Arg Ile Ser Val Ser Thr Glu Gly Ala
   195                 200                 205
   aat act tct tca tct aca tct aca tcc acc act ggg aca 662
   Asn Thr Ser Ser Ser Thr Ser Thr Ser Thr Thr Gly Thr
           210                 215                 220
   agc cat ctt gta aaa tgt gcg gag aag gag aaa act ttc 701
   Ser His Leu Val Lys Cys Ala Glu Lys Glu Lys Thr Phe
                       225                 230
   tgt gtg aat gga ggg gag tgc ttc atg gtg aaa gac ctt 740
   Cys Val Asn Gly Gly Glu Cys Phe Met Val Lys Asp Leu
       235                 240                 245
   tca aac ccc tcg aga tac ttg tgc aag tgc caa cct gga 779
   Ser Asn Pro Ser Arg Tyr Leu Cys Lys Cys Gln Pro Gly
               250                 255
   ttc act gga gca aga tgt act gag aat gtg ccc atg aaa 818
   Phe Thr Gly Ala Arg Cys Thr Glu Asn Val Pro Met Lys
   260                 265                 270
   gtc caa aac caa gaa aag gcg gag gag ctg tac cag aag 857
   Val Gln Asn Gln Glu Lys Ala Glu Glu Leu Tyr Gln Lys
           275                 280                 285
   aga gtg ctg acc ata acc ggc atc tgc atc gcc ctc ctt 896
   Arg Val Leu Thr Ile Thr Gly Ile Cys Ile Ala Leu Leu
                   290                 295
   gtg gtc ggc atc atg tgt gtg gtg gcc tac tgc aaa acc 935
   Val Val Gly Ile Met Cys Val Val Ala Tyr Cys Lys Thr
       300                 305                 310
   aag aaa cag cgg aaa aag ctg cat gac cgt ctt cgg cag 974
   Lys Lys Gln Arg Lys Lys Leu His Asp Arg Leu Arg Gln
               315                 320
   agc ctt cgg tct gaa cga aac aat atg atg aac att gcc 1013
   Ser Leu Arg Ser Glu Arg Asn Asn Met Met Asn Ile Ala
   325                 330                 335
   aat ggg cct cac cat cct aac cca ccc ccc gag aat gtc 1052
   Asn Gly Pro His His Pro Asn Pro Pro Pro Glu Asn Val
           340                 345                 350
   cag ctg gtg aat caa tac gta tct aaa aac gtc atc tcc 1091
   Gln Leu Val Asn Gln Tyr Val Ser Lys Asn Val Ile Ser
                   355                 360
   agt gag cat att gtt gag aga gaa gca gag aca tcc ttt 1130
   Ser Glu His Ile Val Glu Arg Glu Ala Glu Thr Ser Phe
       365                 370                 375
   tcc acc agt cac tat act tcc aca gcc cat cac tcc act 1169
   Ser Thr Ser His Tyr Thr Ser Thr Ala His His Ser Thr
               380                 385
   act gtc acc cag act cct agc cac agc tgg agc aac gga 1208
   Thr Val Thr Gln Thr Pro Ser His Ser Trp Ser Asn Gly
   390                 395                 400
   cac act gaa agc atc ctt tcc gaa agc cac tct gta atc 1247
   His Thr Glu Ser Ile Leu Ser Glu Ser His Ser Val Ile
           405                 410                 415
   gtg atg tca tcc gta gaa aac agt agg cac agc agc cca 1286
   Val Met Ser Ser Val Glu Asn Ser Arg His Ser Ser Pro
                   420                 425
   act ggg ggc cca aga gga cgt ctt aat ggc aca gga ggc 1325
   Thr Gly Gly Pro Arg Gly Arg Leu Asn Gly Thr Gly Gly
       430                 435                 440
   cct cgt gaa tgt aac agc ttc ctc agg cat gcc aga gaa 1364
   Pro Arg Glu Cys Asn Ser Phe Leu Arg His Ala Arg Glu
               445                 450
   acc cct gat tcc tac cga gac tct cct cat agt gaa agg 1403
   Thr Pro Asp Ser Tyr Arg Asp Ser Pro His Ser Glu Arg
   455                 460                 465
   tat gtg tca gcc atg acc acc ccg gct cgt atg tca cct 1442
   Tyr Val Ser Ala Met Thr Thr Pro Ala Arg Met Ser Pro
           470                 475                 480
   gta gat ttc cac acg cca agc tcc ccc aaa tcg ccc cct 1481
   Val Asp Phe His Thr Pro Ser Ser Pro Lys Ser Pro Pro
                   485                 490
   tcg gaa atg tct cca ccc gtg tcc agc atg acg gtg tcc 1520
   Ser Glu Met Ser Pro Pro Val Ser Ser Met Thr Val Ser
       495                 500                 505
   atg cct tcc atg gcg gtc agc ccc ttc atg gaa gaa gag 1559
   Met Pro Ser Met Ala Val Ser Pro Phe Met Glu Glu Glu
               510                 515
   aga cct cta ctt ctc gtg aca cca cca agg ctg cgg gag 1598
   Arg Pro Leu Leu Leu Val Thr Pro Pro Arg Leu Arg Glu
   520                 525                 530
   aag aag ttt gac cat cac cct cag cag ttc agc tcc ttc 1637
   Lys Lys Phe Asp His His Pro Gln Gln Phe Ser Ser Phe
           535                 540                 545
   cac cac aac ccc gcg cat gac agt aac agc ctc cct gct 1676
   His His Asn Pro Ala His Asp Ser Asn Ser Leu Pro Ala
                   550                 555
   agc ccc ttg agg ata gtg gag gat gag gag tat gaa acg 1715
   Ser Pro Leu Arg Ile Val Glu Asp Glu Glu Tyr Glu Thr
       560                 565                 570
   acc caa gag tac gag cca gcc caa gag cct gtt aag aaa 1754
   Thr Gln Glu Tyr Glu Pro Ala Gln Glu Pro Val Lys Lys
               575                 580
   ctc gcc aat agc cgg cgg gcc aaa aga acc aag ccc aat 1793
   Leu Ala Asn Ser Arg Arg Ala Lys Arg Thr Lys Pro Asn
   585                 590                 595
   ggc cac att gct aac aga ttg gaa gtg gac agc aac aca 1832
   Gly His Ile Ala Asn Arg Leu Glu Val Asp Ser Asn Thr
           600                 605                 610
   agc tcc cag agc agt aac tca gag agt gaa aca gaa gat 1871
   Ser Ser Gln Ser Ser Asn Ser Glu Ser Glu Thr Glu Asp
                   615                 620
   gaa aga gta ggt gaa gat acg cct ttc ctg ggc ata cag 1910
   Glu Arg Val Gly Glu Asp Thr Pro Phe Leu Gly Ile Gln
       625                 630                 635
   aac ccc ctg gca gcc agt ctt gag gca aca cct gcc ttc 1949
   Asn Pro Leu Ala Ala Ser Leu Glu Ala Thr Pro Ala Phe
               640                 645
   cgc ctg gct gac agc agg act aac cca gca ggc cgc ttc 1988
   Arg Leu Ala Asp Ser Arg Thr Asn Pro Ala Gly Arg Phe
   650                 655                 660
   tcg aca cag gaa gaa atc cag g ccaggctgtc tagtgtaatt 2030
   Ser Thr Gln Glu Glu Ile Gln
           665             669
   gctaaccaag accctattgc tgtataaaac ctaaataaac acatagattc 2080
   acctgtaaaa ctttatttta tataataaag tattccacct taaattaaac 2130
   aatttatttt attttagcag ttctgcaaat agaaaacagg aaaaaaactt 2180
   ttataaatta aatatatgta tgtaaaaatg aaaaaaaaaa aaaaaa 2226
<210> 3
   <211> 675
   <212> PRT
   <213> Homo sapiens
<400> 3
   Asp Lys Leu Phe Pro Asn Pro Ile Arg Ala Leu Gly Pro Asn Ser
   1               5                   10                  15
   Pro Ala Pro Arg Ala Val Arg Val Glu Arg Ser Val Ser Gly Glu
                   20                  25                  30
   Met Ser Glu Arg Lys Glu Gly Arg Gly Lys Gly Lys Gly Lys Lys
                       35                  40                  45
   Lys Glu Arg Gly Ser Gly Lys Lys Pro Glu Ser Ala Ala Gly Ser
                   50                  55                  60
   Gln Ser Pro Ala Leu Pro Pro Gln Leu Lys Glu Met Lys Ser Gln
                   65                  70                  75
   Glu Ser Ala Ala Gly Ser Lys Leu Val Leu Arg Cys Glu Thr Ser
                   80                  85                  90
   Ser Glu Tyr Ser Ser Leu Arg Phe Lys Trp Phe Lys Asn Gly Asn
                   95                  100                 105
   Glu Leu Asn Arg Lys Asn Lys Pro Gln Asn Ile Lys Ile Gln Lys
                   110                 115                 120
   Lys Pro Gly Lys Ser Glu Leu Arg Ile Asn Lys Ala Ser Leu Ala
                   125                 130                 135
   Asp Ser Gly Glu Tyr Met Cys Lys Val Ile Ser Lys Leu Gly Asn
                   140                 145                 150
   Asp Ser Ala Ser Ala Asn Ile Thr Ile Val Glu Ser Asn Glu Ile
                   155                 160                 165
   Ile Thr Gly Met Pro Ala Ser Thr Glu Gly Ala Tyr Val Ser Ser
                   170                 175                 180
   Glu Ser Pro Ile Arg Ile Ser Val Ser Thr Glu Gly Ala Asn Thr
                   185                 190                 195
   Ser Ser Ser Thr Ser Thr Ser Thr Thr Gly Thr Ser His Leu Val
                   200                 205                 210
   Lys Cys Ala Glu Lys Glu Lys Thr Phe Cys Val Asn Gly Gly Glu
                   215                 220                 225
   Cys Phe Met Val Lys Asp Leu Ser Asn Pro Ser Arg Tyr Leu Cys
                   230                 235                 240
   Lys Cys Pro Asn Glu Phe Thr Gly Asp Arg Cys Gln Asn Tyr Val
                   245                 250                 255
   Met Ala Ser Phe Tyr Lys His Leu Gly Ile Glu Phe Met Glu Ala
                   260                 265                 270
   Glu Glu Leu Tyr Gln Lys Arg Val Leu Thr Ile Thr Gly Ile Cys
                   275                 280                 285
   Ile Ala Leu Leu Val Val Gly Ile Met Cys Val Val Ala Tyr Cys
                   290                 295                 300
   Lys Thr Lys Lys Gln Arg Lys Lys Leu His Asp Arg Leu Arg Gln
                   305                 310                 315
   Ser Leu Arg Ser Glu Arg Asn Asn Met Met Asn Ile Ala Asn Gly
   320 325 330
   Pro His His Pro Asn Pro Pro Pro Glu Asn Val Gln Leu Val Asn
   335 340 345
   Gln Tyr Val Ser Lys Asn Val Ile Ser Ser Glu His Ile Val Glu
   350 355 360
   Arg Glu Ala Glu Thr Ser Phe Ser Thr Ser His Tyr Thr Ser Thr
   365 370 375
   Ala His His Ser Thr Thr Val Thr Gln Thr Pro Ser His Ser Trp
   380 385 390
   Ser Asn Gly His Thr Glu Ser Ile Leu Ser Glu Ser His Ser Val
   395 400 405
   Ile Val Met Ser Ser Val Glu Asn Ser Arg His Ser Ser Pro Thr
   410 415 420
   Gly Gly Pro Arg Gly Arg Leu Asn Gly Thr Gly Gly Pro Arg Glu
   425 430 435
   Cys Asn Ser Phe Leu Arg His Ala Arg Glu Thr Pro Asp Ser Tyr
   440 445 450
   Arg Asp Ser Pro His Ser Glu Arg Tyr Val Ser Ala Met Thr Thr
   455 460 465
   Pro Ala Arg Met Ser Pro Val Asp Phe His Thr Pro Ser Ser Pro
   470 475 480
   Lys Ser Pro Pro Ser Glu Met Ser Pro Pro Val Ser Ser Met Thr
   485 490 495
   Val Ser Met Pro Ser Met Ala Val Ser Pro Phe Met Glu Glu Glu
   500 505 510
   Arg Pro Leu Leu Leu Val Thr Pro Pro Arg Leu Arg Glu Lys Lys
   515 520 525
   Phe Asp His His Pro Gln Gln Phe Ser Ser Phe His His Asn Pro
   530 535 540
   Ala His Asp Ser Asn Ser Leu Pro Ala Ser Pro Leu Arg Ile Val
   545 550 555
   Glu Asp Glu Glu Tyr Glu Thr Thr Gln Glu Tyr Glu Pro Ala Gln
   560 565 570
   Glu Pro Val Lys Lys Leu Ala Asn Ser Arg Arg Ala Lys Arg Thr
   575 580 585
   Lys Pro Asn Gly His Ile Ala Asn Arg Leu Glu Val Asp Ser Asn
   590 595 600
   Thr Ser Ser Gln Ser Ser Asn Ser Glu Ser Glu Thr Glu Asp Glu
                   605                 610                 615
   Arg Val Gly Glu Asp Thr Pro Phe Leu Gly Ile Gln Asn Pro Leu
                   620                 625                 630
   Ala Ala Ser Leu Glu Ala Thr Pro Ala Phe Arg Leu Ala Asp Ser
                   635                 640                 645
   Arg Thr Asn Pro Ala Gly Arg Phe Ser Thr Gln Glu Glu Ile Gln
                   650                 655                 660
   Ala Arg Leu Ser Ser Val Ile Ala Asn Gln Asp Pro Ile Ala Val
                   665                 670                 675
<210> 4
   <211> 2199
   <212> DNA
   <213> Homo sapiens
<400> 4
   gg  gac aaa ctt ttc cca aac ccg atc cga gcc ctt gga 38
       Asp Lys Leu Phe Pro Asn Pro Ile Arg Ala Leu Gly
        1               5                  10
   cca aac tcg cct gcg ccg aga gcc gtc cgc gta gag cgc 77
   Pro Asn Ser Pro Ala Pro Arg Ala Val Arg Val Glu Arg
           15                  20                  25
   tcc gtc tcc ggc gag atg tcc gag cgc aaa gaa ggc aga 116
   Ser Val Ser Gly Glu Met Ser Glu Arg Lys Glu Gly Arg
                   30                  35
   ggc aaa ggg aag ggc aag aag aag gag cga ggc tcc ggc 155
   Gly Lys Gly Lys Gly Lys Lys Lys Glu Arg Gly Ser Gly
       40                  45                  50
   aag aag ccg gag tcc gcg gcg ggc agc cag agc cca gcc 194
   Lys Lys Pro Glu Ser Ala Ala Gly Ser Gln Ser Pro Ala
               55                  60
   ttg cct ccc caa ttg aaa gag atg aaa agc cag gaa tcg 233
   Leu Pro Pro Gln Leu Lys Glu Met Lys Ser Gln Glu Ser
    65                 70                  75
   gct gca ggt tcc aaa cta gtc ctt cgg tgt gaa acc agt 272
   Ala Ala Gly Ser Lys Leu Val Leu Arg Cys Glu Thr Ser
           80                  85                  90
   tct gaa tac tcc tct ctc aga ttc aag tgg ttc aag aat 311
   Ser Glu Tyr Ser Ser Leu Arg Phe Lys Trp Phe Lys Asn
                   95                 100
   ggg aat gaa ttg aat cga aaa aac aaa cca caa aat atc 350
   Gly Asn Glu Leu Asn Arg Lys Asn Lys Pro Gln Asn Ile
       105                 110                 115
   aag ata caa aaa aag cca ggg aag tca gaa ctt cgc att 389
   Lys Ile Gln Lys Lys Pro Gly Lys Ser Glu Leu Arg Ile
               120                 125
   aac aaa gca tca ctg gct gat tct gga gag tat atg tgc 428
   Asn Lys Ala Ser Leu Ala Asp Ser Gly Glu Tyr Met Cys
   130                 135                 140
   aaa gtg atc agc aaa tta gga aat gac agt gcc tct gcc 467
   Lys Val Ile Ser Lys Leu Gly Asn Asp Ser Ala Ser Ala
           145                 150                 155
   aat atc acc atc gtg gaa tca aac gag atc atc act ggt 506
   Asn Ile Thr Ile Val Glu Ser Asn Glu Ile Ile Thr Gly
               160                 165
   atg cca gcc tca act gaa gga gca tat gtg tct tca gag 545
   Met Pro Ala Ser Thr Glu Gly Ala Tyr Val Ser Ser Glu
       170                 175                 180
   tct ccc att aga ata tca gta tcc aca gaa gga gca aat 584
   Ser Pro Ile Arg Ile Ser Val Ser Thr Glu Gly Ala Asn
               185                 190
   act tct tca tct aca tct aca tcc acc act ggg aca agc 623
   Thr Ser Ser Ser Thr Ser Thr Ser Thr Thr Gly Thr Ser
   195                 200                 205
   cat ctt gta aaa tgt gcg gag aag gag aaa act ttc tgt 662
   His Leu Val Lys Cys Ala Glu Lys Glu Lys Thr Phe Cys
           210                 215                 220
   gtg aat gga ggg gag tgc ttc atg gtg aaa gac ctt tca 701
   Val Asn Gly Gly Glu Cys Phe Met Val Lys Asp Leu Ser
               225                 230
   aac ccc tcg aga tac ttg tgc aag tgc cca aat gag ttt 740
   Asn Pro Ser Arg Tyr Leu Cys Lys Cys Pro Asn Glu Phe
       235                 240                 245
   act ggt gat cgc tgc caa aac tac gta atg gcc agc ttc 779
   Thr Gly Asp Arg Cys Gln Asn Tyr Val Met Ala Ser Phe
               250                 255
   tac aag cat ctt ggg att gaa ttt atg gag gcg gag gag 818
   Tyr Lys His Leu Gly Ile Glu Phe Met Glu Ala Glu Glu
   260                 265                 270
   ctg tac cag aag aga gtg ctg acc ata acc ggc atc tgc 857
   Leu Tyr Gln Lys Arg Val Leu Thr Ile Thr Gly Ile Cys
           275                 280                 285
   atc gcc ctc ctt gtg gtc ggc atc atg tgt gtg gtg gcc 896
   Ile Ala Leu Leu Val Val Gly Ile Met Cys Val Val Ala
               290                 295
   tac tgc aaa acc aag aaa cag cgg aaa aag ctg cat gac 935
   Tyr Cys Lys Thr Lys Lys Gln Arg Lys Lys Leu His Asp
       300                 305                 310
   cgt ctt cgg cag agc ctt cgg tct gaa cga aac aat atg 974
   Arg Leu Arg Gln Ser Leu Arg Ser Glu Arg Asn Asn Met
               315                 320
   atg aac att gcc aat ggg cct cac cat cct aac cca ccc 1013
   Met Asn Ile Ala Asn Gly Pro His His Pro Asn Pro Pro
   325                 330                 335
   ccc gag aat gtc cag ctg gtg aat caa tac gta tct aaa 1052
   Pro Glu Asn Val Gln Leu Val Asn Gln Tyr Val Ser Lys
           340                 345                 350
   aac gtc atc tcc agt gag cat att gtt gag aga gaa gca 1091
   Asn Val Ile Ser Ser Glu His Ile Val Glu Arg Glu Ala
                   355                 360
   gag aca tcc ttt tcc acc agt cac tat act tcc aca gcc 1130
   Glu Thr Ser Phe Ser Thr Ser His Tyr Thr Ser Thr Ala
       365                 370                 375
   cat cac tcc act act gtc acc cag act cct agc cac agc 1169
   His His Ser Thr Thr Val Thr Gln Thr Pro Ser His Ser
               380                 385
   tgg agc aac gga cac act gaa agc atc ctt tcc gaa agc 1208
   Trp Ser Asn Gly His Thr Glu Ser Ile Leu Ser Glu Ser
   390                 395                 400
   cac tct gta atc gtg atg tca tcc gta gaa aac agt agg 1247
   His Ser Val Ile Val Met Ser Ser Val Glu Asn Ser Arg
           405                 410                 415
   cac agc agc cca act ggg ggc cca aga gga cgt ctt aat 1286
   His Ser Ser Pro Thr Gly Gly Pro Arg Gly Arg Leu Asn
                   420                 425
   ggc aca gga ggc cct cgt gaa tgt aac agc ttc ctc agg 1325
   Gly Thr Gly Gly Pro Arg Glu Cys Asn Ser Phe Leu Arg
       430                 435                 440
   cat gcc aga gaa acc cct gat tcc tac cga gac tct cct 1364
   His Ala Arg Glu Thr Pro Asp Ser Tyr Arg Asp Ser Pro
               445                 450
   cat agt gaa agg tat gtg tca gcc atg acc acc ccg gct 1403
   His Ser Glu Arg Tyr Val Ser Ala Met Thr Thr Pro Ala
   455                 460                 465
   cgt atg tca cct gta gat ttc cac acg cca agc tcc ccc 1442
   Arg Met Ser Pro Val Asp Phe His Thr Pro Ser Ser Pro
           470                 475                 480
   aaa tcg ccc cct tcg gaa atg tct cca ccc gtg tcc agc 1481
   Lys Ser Pro Pro Ser Glu Met Ser Pro Pro Val Ser Ser
                   485                     490
   atg acg gtg tcc atg cct tcc atg gcg gtc agc ccc ttc 1520
   Met Thr Val Ser Met Pro Ser Met Ala Val Ser Pro Phe
       495                 500                 505
   atg gaa gaa gag aga cct cta ctt ctc gtg aca cca cca 1559
   Met Glu Glu Glu Arg Pro Leu Leu Leu Val Thr Pro Pro
               510                 515
   agg ctg cgg gag aag aag ttt gac cat cac cct cag cag 1598
   Arg Leu Arg Glu Lys Lys Phe Asp His His Pro Gln Gln
   520                 525                 530
   ttc agc tcc ttc cac cac aac ccc gcg cat gac agt aac 1637
   Phe Ser Ser Phe His His Asn Pro Ala His Asp Ser Asn
           535                 540                 545
   agc ctc cct gct agc ccc ttg agg ata gtg gag gat gag 1676
   Ser Leu Pro Ala Ser Pro Leu Arg Ile Val Glu Asp Glu
                   550                 555
   gag tat gaa acg acc caa gag tac gag cca gcc caa gag 1715
   Glu Tyr Glu Thr Thr Gln Glu Tyr Glu Pro Ala Gln Glu
       560                 565                 570
   cct gtt aag aaa ctc gcc aat agc cgg cgg gcc aaa aga 1754
   Pro Val Lys Lys Leu Ala Asn Ser Arg Arg Ala Lys Arg
               575                 580
   acc aag ccc aat ggc cac att gct aac aga ttg gaa gtg 1793
   Thr Lys Pro Asn Gly His Ile Ala Asn Arg Leu Glu Val
   585                 590                 595
   gac agc aac aca agc tcc cag agc agt aac tca gag agt 1832
   Asp Ser Asn Thr Ser Ser Gln Ser Ser Asn Ser Glu Ser
           600                 605                 610
   gaa aca gaa gat gaa aga gta ggt gaa gat acg cct ttc 1871
   Glu Thr Glu Asp Glu Arg Val Gly Glu Asp Thr Pro Phe
                   615                 620
   ctg ggc ata cag aac ccc ctg gca gcc agt ctt gag gca 1910
   Leu Gly Ile Gln Asn Pro Leu Ala Ala Ser Leu Glu Ala
       625                 630                 635
   aca cct gcc ttc cgc ctg gct gac agc agg act aac cca 1949
   Thr Pro Ala Phe Arg Leu Ala Asp Ser Arg Thr Asn Pro
               640                 645
   gca ggc cgc ttc tcg aca cag gaa gaa atc cag gcc agg 1988
   Ala Gly Arg Phe Ser Thr Gln Glu Glu Ile Gln Ala Arg
   650                 655                 660
   ctg tct agt gta att gct aac caa gac cct att gct gta ta 2029
   Leu Ser Ser Val Ile Ala Asn Gln Asp Pro Ile Ala Val
           665                 670                 675
   a aacctaaata aacacataga ttcacctgta aaactttatt 2070
   ttatataata aagtattcca ccttaaatta aacaatttat tttattttag 2120
   cagttctgca aatagaaaac aggaaaaaaa cttttataaa ttaaatatat 2170
   gtatgtaaaa atgaaaaaaa aaaaaaaaa 2199
<210> 5
   <211> 637
   <212> PRT
   <213> Homo sapiens
<400> 5
   Met Ser Glu Arg Lys Glu Gly Arg Gly Lys Gly Lys Gly Lys Lys
    1               5                  10                  15
   Lys Glu Arg Gly Ser Gly Lys Lys Pro Glu Ser Ala Ala Gly Ser
                   20                  25                  30
   Gln Ser Pro Ala Leu Pro Pro Gln Leu Lys Glu Met Lys Ser Gln
                   35                  40                  45
   Glu Ser Ala Ala Gly Ser Lys Leu Val Leu Arg Cys Glu Thr Ser
                   50                  55                  60
   Ser Glu Tyr Ser Ser Leu Arg Phe Lys Trp Phe Lys Asn Gly Asn
                   65                  70                  75
   Glu Leu Asn Arg Lys Asn Lys Pro Gln Asn Ile Lys Ile Gln Lys
                   80                  85                  90
   Lys Pro Gly Lys Ser Glu Leu Arg Ile Asn Lys Ala Ser Leu Ala
                   95                 100                 105
   Asp Ser Gly Glu Tyr Met Cys Lys Val Ile Ser Lys Leu Gly Asn
                  110                 115                 120
   Asp Ser Ala Ser Ala Asn Ile Thr Ile Val Glu Ser Asn Glu Ile
                  125                 130                 135
   Ile Thr Gly Met Pro Ala Ser Thr Glu Gly Ala Tyr Val Ser Ser
                  140                 145                 150
   Glu Ser Pro Ile Arg Ile Ser Val Ser Thr Glu Gly Ala Asn Thr
                  155                 160                 165
   Ser Ser Ser Thr Ser Thr Ser Thr Thr Gly Thr Ser His Leu Val
                  170                 175                 180
   Lys Cys Ala Glu Lys Glu Lys Thr Phe Cys Val Asn Gly Gly Glu
                  185                 190                 195
   Cys Phe Met Val Lys Asp Leu Ser Asn Pro Ser Arg Tyr Leu Cys
   200 205 210
   Lys Cys Pro Asn Glu Phe Thr Gly Asp Arg Cys Gln Asn Tyr Val
   215 220 225
   Met Ala Ser Phe Tyr Lys Ala Glu Glu Leu Tyr Gln Lys Arg Val
   230 235 240
   Leu Thr Ile Thr Gly Ile Cys Ile Ala Leu Leu Val Val Gly Ile
   245 250 255
   Met Cys Val Val Ala Tyr Cys Lys Thr Lys Lys Gln Arg Lys Lys
   260 265 270
   Leu His Asp Arg Leu Arg Gln Ser Leu Arg Ser Glu Arg Asn Asn
   275 280 285
   Met Met Asn Ile Ala Asn Gly Pro His His Pro Asn Pro Pro Pro
   290 295 300
   Glu Asn Val Gln Leu Val Asn Gln Tyr Val Ser Lys Asn Val Ile
   305 310 315
   Ser Ser Glu His Ile Val Glu Arg Glu Ala Glu Thr Ser Phe Ser
   320 325 330
   Thr Ser His Tyr Thr Ser Thr Ala His His Ser Thr Thr Val Thr
   335 340 345
   Gln Thr Pro Ser His Ser Trp Ser Asn Gly His Thr Glu Ser Ile
   350 355 360
   Leu Ser Glu Ser His Ser Val Ile Val Met Ser Ser Val Glu Asn
   365 370 375
   Ser Arg His Ser Ser Pro Thr Gly Gly Pro Arg Gly Arg Leu Asn
   380 385 390
   Gly Thr Gly Gly Pro Arg Glu Cys Asn Ser Phe Leu Arg His Ala
   395 400 405
   Arg Glu Thr Pro Asp Ser Tyr Arg Asp Ser Pro His Ser Glu Arg
   410 415 420
   Tyr Val Ser Ala Met Thr Thr Pro Ala Arg Met Ser Pro Val Asp
   425 430 435
   Phe His Thr Pro Ser Ser Pro Lys Ser Pro Pro Ser Glu Met Ser
   440 445 450
   Pro Pro Val Ser Ser Met Thr Val Ser Lys Pro Ser Met Ala Val
   455 460 465
   Ser Pro Phe Met Glu Glu Glu Arg Pro Leu Leu Leu Val Thr Pro
   470 475 480
   Pro Arg Leu Arg Glu Lys Lys Phe Asp His His Pro Gln Gln Phe
   485 490 495
   Ser Ser Phe His His Asn Pro Ala His Asp Ser Asn Ser Leu Pro
   500 505 510
   Ala Ser Pro Leu Arg Ile Val Glu Asp Glu Glu Tyr Glu Thr Thr
   515 520 525
   Gln Glu Tyr Glu Pro Ala Gln Glu Pro Val Lys Lys Leu Ala Asn
   530 535 540
   Ser Arg Arg Ala Lys Arg Thr Lys Pro Asn Gly His Ile Ala Asn
   545 550 555
   Arg Leu Glu Val Asp Ser Asn Thr Ser Ser Gln Ser Ser Asn Ser
   560 565 570
   Glu Ser Glu Thr Glu Asp Glu Arg Val Gly Glu Asp Thr Pro Phe
   575 580 585
   Leu Gly Ile Gln Asn Pro Leu Ala Ala Ser Leu Glu Ala Thr Pro
   590 595 600
   Ala Phe Arg Leu Ala Asp Ser Arg Thr Asn Pro Ala Gly Arg Phe
   605 610 615
   Ser Thr Gln Glu Glu Ile Gln Ala Arg Leu Ser Ser Val Ile Ala
   620 625 630
   Asn Gln Asp Pro Ile Ala Val
   635 637
<210> 6
   <211> 2490
   <212> DNA
   <213> Home sapiens
<400> 6
   gtggctgcgg ggcaattgaa aaagagccgg cgaggagttc cccgaaactt 50
   gttggaactc cgggctcgcg cggaggccag gagctgagcg gcggcggctg 100
   ccggacgatg ggagcgtgag caggacggtg ataacctctc cccgatcggg 150
   ttgcgagggc gccgggcaga ggccaggacg cgagccgcca gcggcgggac 200
   ccatcgacga cttcccgggg cgacaggagc agccccgaga gccagggcga 250
   gcgcccgttc caggtggccg gaccgcccgc cgcgtccgcg ccgcgctccc 300
   tgcaggcaac gggagacgcc cccgcgcagc gcgagcgcct cagcgcggcc 350
   gctcgctctc cccatcgagg gacaaacttt tcccaaaccc gatccgagcc 400
   cttggaccaa actcgcctgc gccgagagcc gtccgcgtag agcgctccgt 450
   ctccggcgag  atg tcc gag cgc aaa gaa ggc aga ggc aaa 490
               Met Ser Glu Arg Lys Glu Gly Arg Gly Lys
                1               5                  10
   ggg aag ggc aag aag aag gag cga ggc tcc ggc aag aag 529
   Gly Lys Gly Lys Lys Lys Glu Arg Gly Ser Gly Lys Lys
                   15                  20
   ccg gag tcc gcg gcg ggc agc cag agc cca gcc ttg cct 568
   Pro Glu Ser Ala Ala Gly Ser Gln Ser Pro Ala Leu Pro
       25                  30                  35
   ccc caa ttg aaa gag atg aaa agc cag gaa tcg gct gca 607
   Pro Gln Leu Lys Glu Met Lys Ser Gln Glu Ser Ala Ala
               40                  45
   ggt tcc aaa cta gtc ctt cgg tgt gaa acc agt tct gaa 646
   Gly Ser Lys Leu Val Leu Arg Cys Glu Thr Ser Ser Glu
    50                  55                  60
   tac tcc tct ctc aga ttc aag tgg ttc aag aat ggg aat 685
   Tyr Ser Ser Leu Arg Phe Lys Trp Phe Lys Asn Gly Asn
           65                  70                  75
   gaa ttg aat cga aaa aac aaa cca caa aat atc aag ata 724
   Glu Leu Asn Arg Lys Asn Lys Pro Gln Asn Ile Lys Ile
                   80                  85
   caa aaa aag cca ggg aag tca gaa ctt cgc att aac aaa 763
   Gln Lys Lys Pro Gly Lys Ser Glu Leu Arg Ile Asn Lys
       90                  95                  100
   gca tca ctg gct gat tct gga gag tat atg tgc aaa gtg 802
   Ala Ser Leu Ala Asp Ser Gly Glu Tyr Met Cys Lys Val
               105                 110
   atc agc aaa tta gga aat gac agt gcc tct gcc aat atc 841
   Ile Ser Lys Leu Gly Asn Asp Ser Ala Ser Ala Asn Ile
   115                 120                 125
   acc atc gtg gaa tca aac gag atc atc act ggt atg cca 880
   Thr Ile Val Glu Ser Asn Glu Ile Ile Thr Gly Met Pro
           130                 135                 140
   gcc tca act gaa gga gca tat gtg tct tca gag tct ccc 919
   Ala Ser Thr Glu Gly Ala Tyr Val Ser Ser Glu Ser Pro
                   145                 150
   att aga ata tca gta tcc aca gaa gga gca aat act tct 958
   Ile Arg Ile Ser Val Ser Thr Glu Gly Ala Asn Thr Ser
       155                 160                 165
   tca tct aca tct aca tcc acc act ggg aca agc cat ctt 997
   Ser Ser Thr Ser Thr Ser Thr Thr Gly Thr Ser His Leu
               170                 175
   gta aaa tgt gcg gag aag gag aaa act ttc tgt gtg aat 1036
   Val Lys Cys Ala Glu Lys Glu Lys Thr Phe Cys Val Asn
   180                 185                 190
   gga ggg gag tgc ttc atg gtg aaa gac ctt tca aac ccc 1075
   Gly Gly Glu Cys Phe Met Val Lys Asp Leu Ser Asn Pro
           195                 200                 205
   tcg aga tac ttg tgc aag tgc cca aat gag ttt act ggt 1114
   Ser Arg Tyr Leu Cys Lys Cys Pro Asn Glu Phe Thr Gly
                   210                 215
   gat cgc tgc caa aac tac gta atg gcc agc ttc tac aag 1153
   Asp Arg Cys Gln Asn Tyr Val Met Ala Ser Phe Tyr Lys
       220                 225                 230
   gcg gag gag ctg tac cag aag aga gtg ctg acc ata acc 1192
   Ala Glu Glu Leu Tyr Gln Lys Arg Val Leu Thr Ile Thr
               235                 240
   ggc atc tgc atc gcc ctc ctt gtg gtc ggc atc atg tgt 1231
   Gly Ile Cys Ile Ala Leu Leu Val Val Gly Ile Met Cys
   245                 250                 255
   gtg gtg gcc tac tgc aaa acc aag aaa cag cgg aaa aag 1270
   Val Val Ala Tyr Cys Lys Thr Lys Lys Gln Arg Lys Lys
           260                 265                 270
   ctg cat gac cgt ctt cgg cag agc ctt cgg tct gaa cga 1309
   Leu His Asp Arg Leu Arg Gln Ser Leu Arg Ser Glu Arg
                   275                 280
   aac aat atg atg aac att gcc aat ggg cct cac cat cct 1348
   Asn Asn Met Met Asn Ile Ala Asn Gly Pro His His Pro
       285                 290                 295
   aac cca ccc ccc gag aat gtc cag ctg gtg aat caa tac 1387
   Asn Pro Pro Pro Glu Asn Val Gln Leu Val Asn Gln Tyr
               300                 305
   gta tct aaa aac gtc atc tcc agt gag cat att gtt gag 1426
   Val Ser Lys Asn Val Ile Ser Ser Glu His Ile Val Glu
   310                 315                 320
   aga gaa gca gag aca tcc ttt tcc acc agt cac tat act 1465
   Arg Glu Ala Glu Thr Ser Phe Ser Thr Ser His Tyr Thr
           325                 330                 335
   tcc aca gcc cat cac tcc act act gtc acc cag act cct 1504
   Ser Thr Ala His His Ser Thr Thr Val Thr Gln Thr Pro
                   340                 345
   agc cac agc tgg agc aac gga cac act gaa agc atc ctt 1543
   Ser His Ser Trp Ser Asn Gly His Thr Glu Ser Ile Leu
       350                 355                 360
   tcc gaa agc cac tct gta atc gtg atg tca tcc gta gaa 1582
   Ser Glu Ser His Ser Val Ile Val Met Ser Ser Val Glu
               365                 370
   aac agt agg cac agc agc cca act ggg ggc cca aga gga 1621
   Asn Ser Arg His Ser Ser Pro Thr Gly Gly Pro Arg Gly
   375                 380                 385
   cgt ctt aat ggc aca gga ggc cct cgt gaa tgt aac agc 1660
   Arg Leu Asn Gly Thr Gly Gly Pro Arg Glu Cys Asn Ser
           390                 395                 400
   ttc ctc agg cat gcc aga gaa acc cct gat tcc tac cga 1699
   Phe Leu Arg His Ala Arg Glu Thr Pro Asp Ser Tyr Arg
                   405                 410
   gac tct cct cat agt gaa agg tat gtg tca gcc atg acc 1738
   Asp Ser Pro His Ser Glu Arg Tyr Val Ser Ala Met Thr
       415                 420                 425
   acc ccg gct cgt atg tca cct gta gat ttc cac acg cca 1777
   Thr Pro Ala Arg Met Ser Pro Val Asp Phe His Thr Pro
               430                 435
   agc tcc ccc aaa tcg ccc cct tcg gaa atg tct cca ccc 1816
   Ser Ser Pro Lys Ser Pro Pro Ser Glu Met Ser Pro Pro
   440                 445                 450
   gtg tcc agc atg acg gtg tcc aag cct tcc atg gcg gtc 1855
   Val Ser Ser Met Thr Val Ser Lys Pro Ser Met Ala Val
           455                 460                 465
   agc ccc ttc atg gaa gaa gag aga cct cta ctt ctc gtg 1894
   Ser Pro Phe Met Glu Glu Glu Arg Pro Leu Leu Leu Val
                   470                 475
   aca cca cca agg ctg cgg gag aag aag ttt gac cat cac 1933
   Thr Pro Pro Arg Leu Arg Glu Lys Lys Phe Asp His His
       480                 485                 490
   cct cag cag ttc agc tcc ttc cac cac aac ccc gcg cat 1972
   Pro Gln Gln Phe Ser Ser Phe His His Asn Pro Ala His
               495                 500
   gac agt aac agc ctc cct gct agc ccc ttg agg ata gtg 2011
   Asp Ser Asn Ser Leu Pro Ala Ser Pro Leu Arg Ile Val
   505                 510                 515
   gag gat gag gag tat gaa acg acc caa gag tac gag cca 2050
   Glu Asp Glu Glu Tyr Glu Thr Thr Gln Glu Tyr Glu Pro
           520                 525                 530
   gcc caa gag cct gtt aag aaa ctc gcc aat agc cgg cgg 2089
   Ala Gln Glu Pro Val Lys Lys Leu Ala Asn Ser Arg Arg
                   535                 540
   gcc aaa aga acc aag ccc aat ggc cac att gct aac aga 2128
   Ala Lys Arg Thr Lys Pro Asn Gly His Ile Ala Asn Arg
       545                 550                 555
   ttg gaa gtg gac agc aac aca agc tcc cag agc agt aac 2167
   Leu Glu Val Asp Ser Asn Thr Ser Ser Gln Ser Ser Asn
               560                 565
   tca gag agt gaa aca gaa gat gaa aga gta ggt gaa gat 2206
   Ser Glu Ser Glu Thr Glu Asp Glu Arg Val Gly Glu Asp
   570                 575                 580
   acg cct ttc ctg ggc ata cag aac ccc ctg gca gcc agt 2245
   Thr Pro Phe Leu Gly Ile Gln Asn Pro Leu Ala Ala Ser
           585                 590                 595
   ctt gag gca aca cct gcc ttc cgc ctg gct gac agc agg 2284
   Leu Glu Ala Thr Pro Ala Phe Arg Leu Ala Asp Ser Arg
                   600                 605
   act aac cca gca ggc cgc ttc tcg aca cag gaa gaa atc 2323
   Thr Asn Pro Ala Gly Arg Phe Ser Thr Gln Glu Glu Ile
       610                 615                 620
   cag gcc agg ctg tct agt gta att gct aac caa gac cct 2362
   Gln Ala Arg Leu Ser Ser Val Ile Ala Asn Gln Asp Pro
               625                 630
   att gct gta taaaaccta aataaacaca tagattcacc tgtaaaactt 2410
   Ile Ala Val
   635     637
   tattttatat aataaagtat tccaccttaa attaaacaat ttattttatt 2460
   ttagcagttc tgcaaataaa aaaaaaaaaa 2490
<210> 7
   <211> 241
   <212> PRT
   <213> Homo sapiens
<400> 7
   Met Ser Glu Arg Lys Glu Gly Arg Gly Lys Gly Lys Gly Lys Lys
     1               5                  10                  15
   Lys Glu Arg Gly Ser Gly Lys Lys Pro Glu Ser Ala Ala Gly Ser
                    20                  25                  30
   Gln Ser Pro Ala Leu Pro Pro Gln Leu Lys Glu Met Lys Ser Gln
                    35                  40                  45
   Glu Ser Ala Ala Gly Ser Lys Leu Val Leu Arg Cys Glu Thr Ser
                    50                  55                  60
   Ser Glu Tyr Ser Ser Leu Arg Phe Lys Trp Phe Lys Asn Gly Asn
                    65                  70                  75
   Glu Leu Asn Arg Lys Asn Lys Pro Gln Asn Ile Lys Ile Gln Lys
                    80                  85                  90
   Lys Pro Gly Lys Ser Glu Leu Arg Ile Asn Lys Ala Ser Leu Ala
                    95                 100                 105
   Asp Ser Gly Glu Tyr Met Cys Lys Val Ile Ser Lys Leu Gly Asn
                   110                 115                 120
   Asp Ser Ala Ser Ala Asn Ile Thr Ile Val Glu Ser Asn Glu Ile
                   125                 130                 135
   Ile Thr Gly Met Pro Ala Ser Thr Glu Gly Ala Tyr Val Ser Ser
                   140                 145                 150
   Glu Ser Pro Ile Arg Ile Ser Val Ser Thr Glu Gly Ala Asn Thr
                   155                 160                 165
   Ser Ser Ser Thr Ser Thr Ser Thr Thr Gly Thr Ser His Leu Val
                   170                 175                 180
   Lys Cys Ala Glu Lys Glu Lys Thr Phe Cys Val Asn Gly Gly Glu
                   185                 190                 195
   Cys Phe Met Val Lys Asp Leu Ser Asn Pro Ser Arg Tyr Leu Cys
                   200                 205                 210
   Lys Cys Pro Asn Glu Phe Thr Gly Asp Arg Cys Gln Asn Tyr Val
                   215                 220                 225
   Met Ala Ser Phe Tyr Ser Thr Ser Thr Pro Phe Leu Ser Leu Pro
                   230                 235                 240
   Glu
   241
<210> 8
   <211> 1715
   <212> DNA
   <213> Homo sapiens
<400> 8
   gcgcctgcct ccaacctgcg ggcgggaggt gggtggctgc ggggcaattg 50
   aaaaagagcc ggcgaggagt tccccgaaac ttgttggaac tccgggctcg 100
   cgcggaggcc aggagctgag cggcggcggc tgccggacga tgggagcgtg 150
   agcaggacgg tgataacctc tccccgatcg ggttgcgagg gcgccgggca 200
   gaggccagga cgcgagccgc cagcggcggg acccatcgac gacttcccgg 250
   ggcgacagga gcagccccga gagccagggc gagcgcccgt tccaggtggc 300
   cggaccgccc gccgcgtccg cgccgcgctc cctgcaggca acgggagacg 350
   cccccgcgca gcgcgagcgc ctcagcgcgg ccgctcgctc tccccatcga 400
   gggacaaact tttcccaaac ccgatccgag cccttggacc aaactcgcct 450
   gcgccgagag ccgtccgcgt agagcgctcc gtctccggcg ag   atg 495
                                                     Met
                                                      1
   tcc gag cgc aaa gaa ggc aga ggc aaa ggg aag ggc aag 534
   Ser Glu Arg Lys Glu Gly Arg Gly Lys Gly Lys Gly Lys
             5                  10
   aag aag gag cga ggc tcc ggc aag aag ccg gag tcc gcg 573
   Lys Lys Glu Arg Gly Ser Gly Lys Lys Pro Glu Ser Ala
    15                  20                  25
   gcg ggc agc cag agc cca gcc ttg cct ccc caa ttg aaa 612
   Ala Gly Ser Gln Ser Pro Ala Leu Pro Pro Gln Leu Lys
            30                  35                  40
   gag atg aaa agc cag gaa tcg gct gca ggt tcc aaa cta 651
   Glu Met Lys Ser Gln Glu Ser Ala Ala Gly Ser Lys Leu
                    45                  50
   gtc ctt cgg tgt gaa acc agt tct gaa tac tcc tct ctc 690
   Val Leu Arg Cys Glu Thr Ser Ser Glu Tyr Ser Ser Leu
        55                  60                  65
   aga ttc aag tgg ttc aag aat ggg aat gaa ttg aat cga 729
   Arg Phe Lys Trp Phe Lys Asn Gly Asn Glu Leu Asn Arg
                70                  75
   aaa aac aaa cca caa aat atc aag ata caa aaa aag cca 768
   Lys Asn Lys Pro Gln Asn Ile Lys Ile Gln Lys Lys Pro
    80                  85                  90
   ggg aag tca gaa ctt cgc att aac aaa gca tca ctg gct 807
   Gly Lys Ser Glu Leu Arg Ile Asn Lys Ala Ser Leu Ala
            95                 100                 105
   gat tct gga gag tat atg tgc aaa gtg atc agc aaa tta 846
   Asp Ser Gly Glu Tyr Met Cys Lys Val Ile Ser Lys Leu
                   110                 115
   gga aat gac agt gcc tct gcc aat atc acc atc gtg gaa 885
   Gly Asn Asp Ser Ala Ser Ala Asn Ile Thr Ile Val Glu
       120                 125                 130
   tca aac gag atc atc act ggt atg cca gcc tca act gaa 924
   Ser Asn Glu Ile Ile Thr Gly Met Pro Ala Ser Thr Glu
               135                 140
   gga gca tat gtg tct tca gag tct ccc att aga ata tca 963
   Gly Ala Tyr Val Ser Ser Glu Ser Pro Ile Arg Ile Ser
   145                 150                 155
   gta tcc aca gaa gga gca aat act tct tca tct aca tct 1002
   Val Ser Thr Glu Gly Ala Asn Thr Ser Ser Ser Thr Ser
           160                     165                     170
   aca tcc acc act ggg aca agc cat ctt gta aaa tgt gcg 1041
   Thr Ser Thr Thr Gly Thr Ser His Leu Val Lys Cys Ala
                   175                     180
   gag aag gag aaa act ttc tgt gtg aat gga ggg gag tgc 1080
   Glu Lys Glu Lys Thr Phe Cys Val Asn Gly Gly Glu Cys
       185                     190                     195
   ttc atg gtg aaa gac ctt tca aac ccc tcg aga tac ttg 1119
   Phe Met Val Lys Asp Leu Ser Asn Pro Ser Arg Tyr Leu
               200                     205
   tgc aag tgc cca aat gag ttt act ggt gat cgc tgc caa 1158
   Cys Lys Cys Pro Asn Glu Phe Thr Gly Asp Arg Cys Gln
   210                     215                     220
   aac tac gta atg gcc agc ttc tac agt acg tcc act ccc 1197
   Asn Tyr Val Met Ala Ser Phe Tyr Ser Thr Ser Thr Pro
           225                     230                     235
   ttt ctg tct ctg cct gaa tagga gcatgctcag ttggtgctgc 1240
   Phe Leu Ser Leu Pro Glu
                   240 241
   tttcttgttg ctgcatctcc cctcagattc cacctagagc tagatgtgtc 1290
   ttaccagatc taatattgac tgcctctgcc tgtcgcatga gaacattaac 1340
   aaaagcaatt gtattacttc ctctgttcgc gactagttgg ctctgagata 1390
   ctaataggtg tgtgaggctc cggatgtttc tggaattgat attgaatgat 1440
   gtgatacaaa ttgatagtca atatcaagca gtgaaatatg ataataaagg 1490
   catttcaaag tctcactttt attgataaaa taaaaatcat tctactgaac 1540
   agtccatctt ctttatacaa tgaccacatc ctgaaaaggg tgttgctaag 1590
   ctgtaaccga tatgcacttg aaatgatggt aagttaattt tgattcagaa 1640
   tgtgttattt gtcacaaata aacataataa aaggagttca gatgtttttc 1690
   ttcattaacc aaaaaaaaaa aaaaa 1715
<210> 9
   <211> 420
   <212> PRT
   <213> Homo sapiens
<400> 9
   Met Ser Glu Arg Lys Glu Gly Arg Gly Lys Gly Lys Gly Lys Lys
   1 5 10 15
   Lys Glu Arg Gly Ser Gly Lys Lys Pro Glu Ser Ala Ala Gly Ser
   20 25 30
   Gln Ser Pro Ala Leu Pro Pro Gln Leu Lys Glu Met Lys Ser Gln
   35 40 45
   Glu Ser Ala Ala Gly Ser Lys Leu Val Leu Arg Cys Glu Thr Ser
   50 55 60
   Ser Glu Tyr Ser Ser Leu Arg Phe Lys Trp Phe Lys Asn Gly Asn
   65 70 75
   Glu Leu Asn Arg Lys Asn Lys Pro Gln Asn Ile Lys Ile Gln Lys
   80 85 90
   Lys Pro Gly Lys Ser Glu Leu Arg Ile Asn Lys Ala Ser Leu Ala
   95 100 105
   Asp Ser Gly Glu Tyr Met Cys Lys Val Ile Ser Lys Leu Gly Asn
   110 115 120
   Asp Ser Ala Ser Ala Asn Ile Thr Ile Val Glu Ser Asn Glu Ile
   125 130 135
   Ile Thr Gly Met Pro Ala Ser Thr Glu Gly Ala Tyr Val Ser Ser
   140 145 150
   Glu Ser Pro Ile Arg Ile Ser Val Ser Thr Glu Gly Ala Asn Thr
   155 160 165
   Ser Ser Ser Thr Ser Thr Ser Thr Thr Gly Thr Ser His Leu Val
   170 175 180
   Lys Cys Ala Glu Lys Glu Lys Thr Phe Cys Val Asn Gly Gly Glu
   185 190 195
   Cys Phe Met Val Lys Asp Leu Ser Asn Pro Ser Arg Tyr Leu Cys
   200 205 210
   Lys Cys Pro Asn Glu Phe Thr Gly Asp Arg Cys Gln Asn Tyr Val
   215 220 225
   Met Ala Ser Phe Tyr Lys Ala Glu Glu Leu Tyr Gln Lys Arg Val
   230 235 240
   Leu Thr Ile Thr Gly Ile Cys Ile Ala Leu Leu Val Val Gly Ile
   245 250 255
   Met Cys Val Val Ala Tyr Cys Lys Thr Lys Lys Gln Arg Lys Lys
   260 265 270
   Leu His Asp Arg Leu Arg Gln Ser Leu Arg Ser Glu Arg Asn Asn
   275 280 285
   Met Met Asn Ile Ala Asn Gly Pro His His Pro Asn Pro Pro Pro
   290 295 300
   Glu Asn Val Gln Leu Val Asn Gln Tyr Val Ser Lys Asn Val Ile
   305 310 315
   Ser Ser Glu His Ile Val Glu Arg Glu Ala Glu Thr Ser Phe Ser
   320 325 330
   Thr Ser His Tyr Thr Ser Thr Ala His His Ser Thr Thr Val Thr
   335 340 345
   Gln Thr Pro Ser His Ser Trp Ser Asn Gly His Thr Glu Ser Ile
   350 355 360
   Leu Ser Glu Ser His Ser Val Ile Val Met Ser Ser Val Glu Asn
   365 370 375
   Ser Arg His Ser Ser Pro Thr Gly Gly Pro Arg Gly Arg Leu Asn
   380 385 390
   Gly Thr Gly Gly Pro Arg Glu Cys Asn Ser Phe Leu Arg His Ala
   395 400 405
   Arg Glu Thr Pro Asp Ser Tyr Arg Asp Ser Pro His Ser Glu Arg
   410 415 420
<210> 10
   <211> 2431
   <212> DNA
   <213> Homo sapiens
<400> 10
   gaggcgcctg cctccaacct gcgggcggga ggtgggtggc tgcggggcaa 50
   ttgaaaaaga gccggcgagg agttccccga aacttgttgg aactccgggc 100
   tcgcgcggag gccaggagct gagcggcggc ggctgccgga cgatgggagc 150
   gtgagcagga cggtgataac ctctccccga tcgggttgcg agggcgccgg 200
   gcagaggcca ggacgcgagc cgccagcggc gggacccatc gacgacttcc 250
   cggggcgaca ggagcagccc cgagagccag ggcgagcgcc cgttccaggt 300
   ggccggaccg cccgccgcgt ccgcgccgcg ctccctgcag gcaacgggag 350
   acgcccccgc gcagcgcgag cgcctcagcg cggccgctcg ctctccccat 400
   cgagggacaa acttttccca aacccgatcc gagcccttgg accaaactcg 450
   cctgcgccga gagccgtccg cgtagagcgc tccgtctccg gcgag  atg 498
                                                      Met
                                                       1
   tcc gag cgc aaa gaa ggc aga ggc aaa ggg aag ggc aag 537
   Ser Glu Arg Lys Glu Gly Arg Gly Lys Gly Lys Gly Lys
                   5                   10
   aag aag gag cga ggc tcc ggc aag aag ccg gag tcc gcg 576
   Lys Lys Glu Arg Gly Ser Gly Lys Lys Pro Glu Ser Ala
    15                 20                  25
   gcg ggc agc cag agc cca gcc ttg cct ccc caa ttg aaa 615
   Ala Gly Ser Gln Ser Pro Ala Leu Pro Pro Gln Leu Lys
           30                  35                  40
   gag atg aaa agc cag gaa tcg gct gca ggt tcc aaa cta 654
   Glu Met Lys Ser Gln Glu Ser Ala Ala Gly Ser Lys Leu
                   45                  50
   gtc ctt cgg tgt gaa acc agt tct gaa tac tcc tct ctc 693
   Val Leu Arg Cys Glu Thr Ser Ser Glu Tyr Ser Ser Leu
       55                  60                  65
   aga ttc aag tgg ttc aag aat ggg aat gaa ttg aat cga 732
   Arg Phe Lys Trp Phe Lys Asn Gly Asn Glu Leu Asn Arg
               70                  75
   aaa aac aaa cca caa aat atc aag ata caa aaa aag cca 771
   Lys Asn Lys Pro Gln Asn Ile Lys Ile Gln Lys Lys Pro
    80                 85                  90
   ggg aag tca gaa ctt cgc att aac aaa gca tca ctg gct 810
   Gly Lys Ser Glu Leu Arg Ile Asn Lys Ala Ser Leu Ala
           95                  100                 105
   gat tct gga gag tat atg tgc aaa gtg atc agc aaa tta 849
   Asp Ser Gly Glu Tyr Met Cys Lys Val Ile Ser Lys Leu
                   110                 115
   gga aat gac agt gcc tct gcc aat atc acc atc gtg gaa 888
   Gly Asn Asp Ser Ala Ser Ala Asn Ile Thr Ile Val Glu
       120                 125                 130
   tca aac gag atc atc act ggt atg cca gcc tca act gaa 927
   Ser Asn Glu Ile Ile Thr Gly Met Pro Ala Ser Thr Glu
               135                 140
   gga gca tat gtg tct tca gag tct ccc att aga ata tca 966
   Gly Ala Tyr Val Ser Ser Glu Ser Pro Ile Arg Ile Ser
   145                 150                 155
   gta tcc aca gaa gga gca aat act tct tca tct aca tct 1005
   Val Ser Thr Glu Gly Ala Asn Thr Ser Ser Ser Thr Ser
           160                 165                 170
   aca tcc acc act ggg aca agc cat ctt gta aaa tgt gcg 1044
   Thr Ser Thr Thr Gly Thr Ser His Leu Val Lys Cys Ala
                   175                 180
   gag aag gag aaa act ttc tgt gtg aat gga ggg gag tgc 1083
   Glu Lys Glu Lys Thr Phe Cys Val Asn Gly Gly Glu Cys
       185                 190                 195
   ttc atg gtg aaa gac ctt tca aac ccc tcg aga tac ttg 1122
   Phe Met Val Lys Asp Leu Ser Asn Pro Ser Arg Tyr Leu
               200                 205
   tgc aag tgt cca aat gag ttt act ggt gat cgc tgc caa 1161
   Cys Lys Cys Pro Asn Glu Phe Thr Gly Asp Arg Cys Gln
   210                 215                 220
   aac tac gta atg gcc agc ttc tac aag gcg gag gag ctg 1200
   Asn Tyr Val Met Ala Ser Phe Tyr Lys Ala Glu Glu Leu
           225                 230                 235
   tac cag aag aga gtg ctg acc ata acc ggc atc tgc atc 1239
   Tyr Gln Lys Arg Val Leu Thr Ile Thr Gly Ile Cys Ile
                   240                 245
   gcc ctc ctt gtg gtc ggc atc atg tgt gtg gtg gcc tac 1278
   Ala Leu Leu Val Val Gly Ile Met Cys Val Val Ala Tyr
       250                 255                 260
   tgc aaa acc aag aaa cag cgg aaa aag ctg cat gac cgt 1317
   Cys Lys Thr Lys Lys Gln Arg Lys Lys Leu His Asp Arg
               265                 270
   ctt cgg cag agc ctt cgg tct gaa cga aac aat atg atg 1356
   Leu Arg Gln Ser Leu Arg Ser Glu Arg Asn Asn Met Met
   275                 280                 285
   aac att gcc aat ggg cct cac cat cct aac cca ccc ccc 1395
   Asn Ile Ala Asn Gly Pro His His Pro Asn Pro Pro Pro
           290                 295                 300
   gag aat gtc cag ctg gtg aat caa tac gta tct aaa aac 1434
   Glu Asn Val Gln Leu Val Asn Gln Tyr Val Ser Lys Asn
                   305                 310
   gtc atc tcc agt gag cat att gtt gag aga gaa gca gag 1473
   Val Ile Ser Ser Glu His Ile Val Glu Arg Glu Ala Glu
       315                 320                 325
   aca tcc ttt tcc acc agt cac tat act tcc aca gcc cat 1512
   Thr Ser Phe Ser Thr Ser His Tyr Thr Ser Thr Ala His
               330                 335
   cac tcc act act gtc acc cag act cct agc cac agc tgg 1551
   His Ser Thr Thr Val Thr Gln Thr Pro Ser His Ser Trp
   340                 345                 350
   agc aac gga cac act gaa agc atc ctt tcc gaa agc cac 1590
   Ser Asn Gly His Thr Glu Ser Ile Leu Ser Glu Ser His
           355                 360                 365
   tct gta atc gtg atg tca tcc gta gaa aac agt agg cac 1629
   Ser Val Ile Val Met Ser Ser Val Glu Asn Ser Arg His
                   370                 375
   agc agc cca act ggg ggc cca aga gga cgt ctt aat ggc 1668
   Ser Ser Pro Thr Gly Gly Pro Arg Gly Arg Leu Asn Gly
       380                 385                 390
   aca gga ggc cct cgt gaa tgt aac agc ttc ctc agg cat 1707
   Thr Gly Gly Pro Arg Glu Cys Asn Ser Phe Leu Arg His
               395                 400
   gcc aga gaa acc cct gat tcc tac cga gac tct cct cat 1746
   Ala Arg Glu Thr Pro Asp Ser Tyr Arg Asp Ser Pro His
   405                 410                 415
   agt gaa agg taaaa ccgaaggcaa agctactgca gaggagaaac 1790
   Ser Glu Arg
           420
   tcagtcagag aatccctgtg agcacctgcg gtctcacctc aggaaatcta 1840
   ctctaatcag aataaggggc ggcagttacc tgttctagga gtgctcctag 1890
   ttgatgaagt catctctttg tttgacggaa cttatttctt ctgagcttct 1940
   ctcgtcgtcc cagtgactga caggcaacag actcttaaag agctgggatg 1990
   ctttgatgcg gaaggtgcag cacatggagt ttccagctct ggccatgggc 2040
   tcagacccac tcggggtctc agtgtcctca gttgtaacat tagagagatg 2090
   gcatcaatgc ttgataagga cccttctata attccaattg ccagttatcc 2140
   aaactctgat tcggtggtcg agctggcctc gtgttcttat ctgctaaccc 2190
   tgtcttacct tccagcctca gttaagtcaa atcaagggct atgtcattgc 2240
   tgaatgtcat ggggggcaac tgcttgccct ccaccctata gtatctattt 2290
   tatgaaattc caagaaggga tgaataaata aatctcttgg atgctgcgtc 2340
   tggcagtctt cacgggtggt tttcaaagca gaaaaaaaaa aaaaaaaaaa 2390
   aaaaaaaaaa aaaaaaaaaa aaaaaaaaaa aaaaaaaaaa a 2431
<210> 11
   <211> 768
   <212> PRT
   <213> Homo sapiens
<400> 11
   Met Asp Val Lys Glu Arg Lys Pro Tyr Arg Ser Leu Thr Arg Arg
    1               5                  10                  15
   Arg Asp Ala Glu Arg Arg Tyr Thr Ser Ser Ser Ala Asp Ser Glu
                   20                  25                  30
   Glu Gly Lys Ala Pro Gln Lys Ser Tyr Ser Ser Ser Glu Thr Leu
                   35                  40                  45
   Lys Ala Tyr Asp Gln Asp Ala Arg Leu Ala Tyr Gly Ser Arg Val
                   50                  55                  60
   Lys Asp Ile Val Pro Gln Glu Ala Glu Glu Phe Cys Arg Thr Gly
                   65                  70                  75
   Ala Asn Phe Thr Leu Arg Glu Leu Gly Leu Glu Glu Val Thr Pro
                   80                  85                  90
   Pro His Gly Thr Leu Tyr Arg Thr Asp Ile Gly Leu Pro His Cys
                   95                  100                 105
   Gly Tyr Ser Met Gly Ala Gly Ser Asp Ala Asp Met Glu Ala Asp
                   110                 115                 120
   Thr Val Leu Ser Pro Glu His Pro Val Arg Leu Trp Gly Arg Ser
                   125                 130                 135
   Thr Arg Ser Gly Arg Ser Ser Cys Leu Ser Ser Arg Ala Asn Ser
                   140                 145                 150
   Asn Leu Thr Leu Thr Asp Thr Glu His Glu Asn Thr Glu Thr Asp
                   155                 160                 165
   His Pro Gly Gly Leu Gln Asn His Ala Arg Leu Arg Thr Pro Pro
                   170                 175                 180
   Pro Pro Leu Ser His Ala His Thr Pro Asn Gln His His Ala Ala
                   185                 190                 195
   Ser Ile Asn Ser Leu Asn Arg Gly Asn Phe Thr Pro Arg Ser Asn
                   200                 205                 210
   Pro Ser Pro Ala Pro Thr Asp His Ser Leu Ser Gly Glu Pro Pro
                   215                 220                 225
   Ala Gly Gly Ala Gln Glu Pro Ala His Ala Gln Glu Asn Trp Leu
                   230                 235                 240
   Leu Asn Ser Asn Ile Pro Leu Glu Thr Arg Asn Leu Gly Lys Gln
                   245                 250                 255
   Pro Phe Leu Gly Thr Leu Gln Asp Asn Leu Ile Glu Met Asp Ile
                   260                 265                 270
   Leu Gly Ala Ser Arg His Asp Gly Ala Tyr Ser Asp Gly His Phe
                   275                 280                 285
   Leu Phe Lys Pro Gly Gly Thr Ser Pro Leu Phe Cys Thr Thr Ser
                   290                 295                 300
   Pro Gly Tyr Pro Leu Thr Ser Ser Thr Val Tyr Ser Pro Pro Pro
                   305                 310                 315
   Arg Pro Leu Pro Arg Ser Thr Phe Ala Arg Pro Ala Phe Asn Leu
                   320                 325                 330
   Lys Lys Pro Ser Lys Tyr Cys Asn Trp Lys Cys Ala Ala Leu Ser
   335 340 345
   Ala Ile Val Ile Ser Ala Thr Leu Val Ile Leu Leu Ala Tyr Phe
   350 355 360
   Val Ala Met His Leu Phe Gly Leu Asn Trp His Leu Gln Pro Met
   365 370 375
   Glu Gly Gln Met Tyr Glu Ile Thr Glu Asp Thr Ala Ser Ser Trp
   380 385 390
   Pro Val Pro Thr Asp Val Ser Leu Tyr Pro Ser Gly Gly Thr Gly
   395 400 405
   Leu Glu Thr Pro Asp Arg Lys Gly Lys Gly Thr Thr Glu Gly Lys
   410 415 420
   Pro Ser Ser Phe Phe Pro Glu Asp Ser Phe Ile Asp Ser Gly Glu
   425 430 435
   Ile Asp Val Gly Arg Arg Ala Ser Gln Lys Ile Pro Pro Gly Thr
   440 445 450
   Phe Trp Arg Ser Gln Val Phe Ile Asp His Pro Val His Leu Lys
   455 460 465
   Phe Asn Val Ser Leu Gly Lys Ala Ala Leu Val Gly Ile Tyr Gly
   470 475 480
   Arg Lys Gly Leu Pro Pro Ser His Thr Gln Phe Asp Phe Val Glu
   485 490 495
   Leu Leu Asp Gly Arg Arg Leu Leu Thr Gln Glu Ala Arg Ser Leu
   500 505 510
   Glu Gly Thr Pro Arg Gln Ser Arg Gly Thr Val Pro Pro Ser Ser
   515 520 525
   His Glu Thr Gly Phe Ile Gln Tyr Leu Asp Ser Gly Ile Trp His
   530 535 540
   Leu Ala Phe Tyr Asn Asp Gly Lys Glu Ser Glu Val Val Ser Phe
   545 550 555
   Leu Thr Thr Ala Ile Ala Leu Pro Pro Arg Leu Lys Glu Met Lys
   560 565 570
   Ser Gln Glu Ser Ala Ala Gly Ser Lys Leu Val Leu Arg Cys Glu
   575 580 585
   Thr Ser Ser Glu Tyr Ser Ser Leu Arg Phe Lys Trp Phe Lys Asn
   590 595 600
   Gly Asn Glu Leu Asn Arg Lys Asn Lys Pro Gln Asn Ile Lys Ile
   605 610 615
   Gln Lys Lys Pro Gly Lys Ser Glu Leu Arg Ile Asn Lys Ala Ser
                   620                 625                 630
   Leu Ala Asp Ser Gly Glu Tyr Met Cys Lys Val Ile Ser Lys Leu
                   635                 640                 645
   Gly Asn Asp Ser Ala Ser Ala Asn Ile Thr Ile Val Glu Ser Asn
                   650                 655                 660
   Glu Ile Ile Thr Gly Met Pro Ala Ser Thr Glu Gly Ala Tyr Val
                   665                 670                 675
   Ser Ser Glu Ser Pro Ile Arg Ile Ser Val Ser Thr Glu Gly Ala
                   680                 685                 690
   Asn Thr Ser Ser Ser Thr Ser Thr Ser Thr Thr Gly Thr Ser His
                   695                 700                 705
   Leu Val Lys Cys Ala Glu Lys Glu Lys Thr Phe Cys Val Asn Gly
                   710                 715                 720
   Gly Glu Cys Phe Met Val Lys Asp Leu Ser Asn Pro Ser Arg Tyr
                   725                 730                 735
   Leu Cys Lys Cys Pro Asn Glu Phe Thr Gly Asp Arg Cys Gln Asn
                   740                 745                 750
   Tyr Val Met Ala Ser Phe Tyr Ser Thr Ser Thr Pro Phe Leu Ser
                   755                 760                 765
   Leu Pro Glu
           768
<210> 12
   <211> 3111
   <212> DNA
   <213> Homo sapiens
<400> 12
   gggtaccatg ggtcggtgag cgcgtttccc gcctgagcgc aactagcggc 50
   gggtcgtggg cacctccaga aaagatcccg caccatcctc caggatccaa 100
   tggccttgga gagagggctg cagggcccac ggacattgct gactcttcag 150
   aacgtgctga catggagcca ggtagactga aattatcatg tgtccaaatt 200
   aaaattgcat acttcaagga ttatttgaag gactattctt agaccctttt 250
   aagaagattt aaagaaaaac cactcggccc tgagtgcggc gaggaccctg 300
   tttgtggatg tggaggagcg cgggccggag gcc    atg gac gtg 342
                                           Met Asp Val
                                            1
   aag gag agg aag cct tac cgc tcg ctg acc cgg cgc cgc 381
   Lys Glu Arg Lys Pro Tyr Arg Ser Leu Thr Arg Arg Arg
         5                   10                  15
   gac gcc gag cgc cgc tac acc agc tcg tcc gcg gac agc 420
   Asp Ala Glu Arg Arg Tyr Thr Ser Ser Ser Ala Asp Ser
                20                  25
   gag gag ggc aaa gcc ccg cag aaa tcg tac agc tcc agc 459
   Glu Glu Gly Lys Ala Pro Gln Lys Ser Tyr Ser Ser Ser
    30                  35                  40
   gag acc ctg aag gcc tac gac cag gac gcc cgc cta gcc 498
   Glu Thr Leu Lys Ala Tyr Asp Gln Asp Ala Arg Leu Ala
            45                  50                  55
   tat ggc agc cgc gtc aag gac att gtg ccg cag gag gcc 537
   Tyr Gly Ser Arg Val Lys Asp Ile Val Pro Gln Glu Ala
                    60                  65
   gag gaa ttc tgc cgc aca ggt gcc aac ttc acc ctg cgg 576
   Glu Glu Phe Cys Arg Thr Gly Ala Asn Phe Thr Leu Arg
        70                  75                  80
   gag ctg ggg ctg gaa gaa gta acg ccc cct cac ggg acc 615
   Glu Leu Gly Leu Glu Glu Val Thr Pro Pro His Gly Thr
                85                  90
   ctg tac cgg aca gac att ggc ctc ccc cac tgc ggc tac 654
   Leu Tyr Arg Thr Asp Ile Gly Leu Pro His Cys Gly Tyr
    95                 100                 105
   tcc atg ggg gct ggc tct gat gcc gac atg gag gct gac 693
   Ser Met Gly Ala Gly Ser Asp Ala Asp Met Glu Ala Asp
           110                 115                 120
   acg gtg ctg tcc cct gag cac ccc gtg cgt ctg tgg ggc 732
   Thr Val Leu Ser Pro Glu His Pro Val Arg Leu Trp Gly
                   125                 130
   cgg agc aca cgg tca ggg cgc agc tcc tgc ctg tcc agc 771
   Arg Ser Thr Arg Ser Gly Arg Ser Ser Cys Leu Ser Ser
       135                 140                 145
   cgg gcc aat tcc aat ctc aca ctc acc gac acc gag cat 810
   Arg Ala Asn Ser Asn Leu Thr Leu Thr Asp Thr Glu His
               150                 155
   gaa aac act gag act gat cat ccg ggc ggc ctg cag aac 849
   Glu Asn Thr Glu Thr Asp His Pro Gly Gly Leu Gln Asn
   160                 165                 170
   cac gcg cgg ctc cgg acg ccg ccg ccg ccg ctc tcg cac 888
   His Ala Arg Leu Arg Thr Pro Pro Pro Pro Leu Ser His
           175                 180                 185
   gcc cac acc ccc aac cag cac cac gcg gcc tcc att aac 927
   Ala His Thr Pro Asn Gln His His Ala Ala Ser Ile Asn
                   190                 195
   tcc ctg aac cgg ggc aac ttc acg ccg agg agc aac ccc 966
   Ser Leu Asn Arg Gly Asn Phe Thr Pro Arg Ser Asn Pro
       200                 205                 210
   agc ccg gcc ccc acg gac cac tcg ctc tcc gga gag ccc 1005
   Ser Pro Ala Pro Thr Asp His Ser Leu Ser Gly Glu Pro
               215                 220
   cct gcc ggc ggc gcc cag gag cct gcc cac gcc cag gag 1044
   Pro Ala Gly Gly Ala Gln Glu Pro Ala His Ala Gln Glu
   225                 230                 235
   aac tgg ctg ctc aac agc aac atc ccc ctg gag acc aga 1083
   Asn Trp Leu Leu Asn Ser Asn Ile Pro Leu Glu Thr Arg
           240                 245                 250
   aac cta ggc aag cag cca ttc cta ggg aca ttg cag gac 1122
   Asn Leu Gly Lys Gln Pro Phe Leu Gly Thr Leu Gln Asp
                   255                 260
   aac ctc att gag atg gac att ctc ggc gcc tcc cgc cat 1161
   Asn Leu Ile Glu Met Asp Ile Leu Gly Ala Ser Arg His
       265                 270                 275
   gat ggg gct tac agt gac ggg cac ttc ctc ttc aag cct 1200
   Asp Gly Ala Tyr Ser Asp Gly His Phe Leu Phe Lys Pro
               280                 285
   gga ggc acc tcc ccg ctc ttc tgc acc aca tca cca ggg 1239
   Gly Gly Thr Ser Pro Leu Phe Cys Thr Thr Ser Pro Gly
   290                 295                 300
   tac cca ctg acg tcc agc aca gtg tac tct cct ccg ccc 1278
   Tyr Pro Leu Thr Ser Ser Thr Val Tyr Ser Pro Pro Pro
           305                 310                 315
   cga ccc ctg ccc cgc agc acc ttc gcc cgg ccg gcc ttt 1317
   Arg Pro Leu Pro Arg Ser Thr Phe Ala Arg Pro Ala Phe
                   320                 325
   aac ctc aag aag ccc tcc aag tac tgt aac tgg aag tgc 1356
   Asn Leu Lys Lys Pro Ser Lys Tyr Cys Asn Trp Lys Cys
       330                 335                 340
   gca gcc ctg agc gcc atc gtc atc tca gcc act ctg gtc 1395
   Ala Ala Leu Ser Ala Ile Val Ile Ser Ala Thr Leu Val
               345                 350
   atc ctg ctg gca tac ttt gtg gcc atg cac ctg ttt ggc 1434
   Ile Leu Leu Ala Tyr Phe Val Ala Met His Leu Phe Gly
   355                 360                 365
   cta aac tgg cac ctg cag ccg atg gag ggg cag atg tat 1473
   Leu Asn Trp His Leu Gln Pro Met Glu Gly Gln Met Tyr
           370                 375                 380
   gag atc acg gag gac aca gcc agc agt tgg cct gtg cca 1512
   Glu Ile Thr Glu Asp Thr Ala Ser Ser Trp Pro Val Pro
                   385                 390
   acc gac gtc tcc cta tac ccc tca ggg ggc act ggc tta 1551
   Thr Asp Val Ser Leu Tyr Pro Ser Gly Gly Thr Gly Leu
       395                 400                 405
   gag acc cct gac agg aaa ggc aaa gga acc aca gaa gga 1590
   Glu Thr Pro Asp Arg Lys Gly Lys Gly Thr Thr Glu Gly
               410                 415
   aag ccc agt agt ttc ttt cca gag gac agt ttc ata gat 1629
   Lys Pro Ser Ser Phe Phe Pro Glu Asp Ser Phe Ile Asp
   420                 425                 430
   tct gga gaa att gat gtg gga agg cga gct tcc cag aag 1668
   Ser Gly Glu Ile Asp Val Gly Arg Arg Ala Ser Gln Lys
           435                 440                 445
   att cct cct ggc act ttc tgg aga tct caa gtg ttc ata 1707
   Ile Pro Pro Gly Thr Phe Trp Arg Ser Gln Val Phe Ile
                   450                 455
   gac cat cct gtg cat ctg aaa ttc aat gtg tct ctg gga 1746
   Asp His Pro Val His Leu Lys Phe Asn Val Ser Leu Gly
       460                 465                 470
   aag gca gcc ctg gtt ggc att tat ggc aga aaa ggc ctc 1785
   Lys Ala Ala Leu Val Gly Ile Tyr Gly Arg Lys Gly Leu
               475                 480
   cct cct tca cat aca cag ttt gac ttt gtg gag ctg ctg 1824
   Pro Pro Ser His Thr Gln Phe Asp Phe Val Glu Leu Leu
   485                 490                 495
   gat ggc agg agg ctc cta acc cag gag gcg cgg agc cta 1863
   Asp Gly Arg Arg Leu Leu Thr Gln Glu Ala Arg Ser Leu
           500                 505                 510
   gag ggg acc ccg cgc cag tct cgg gga act gtg ccc ccc 1902
   Glu Gly Thr Pro Arg Gln Ser Arg Gly Thr Val Pro Pro
                   515                 520
   tcc agc cat gag aca ggc ttc atc cag tat ttg gat tca 1941
   Ser Ser His Glu Thr Gly Phe Ile Gln Tyr Leu Asp Ser
       525                 530                 535
   gga atc tgg cac ttg gct ttt tac aat gac gga aag gag 1980
   Gly Ile Trp His Leu Ala Phe Tyr Asn Asp Gly Lys Glu
               540                 545
   tca gaa gtg gtt tcc ttt ctc acc act gcc att gcc ttg 2019
   Ser Glu Val Val Ser Phe Leu Thr Thr Ala Ile Ala Leu
   550                 555                 560
   cct ccc cga ttg aaa gag atg aaa agc cag gaa tcg gct 2058
   Pro Pro Arg Leu Lys Glu Met Lys Ser Gln Glu Ser Ala
           565                 570                 575
   gca ggt tcc aaa cta gtc ctt cgg tgt gaa acc agt tct 2097
   Ala Gly Ser Lys Leu Val Leu Arg Cys Glu Thr Ser Ser
                   580                 585
   gaa tac tcc tct ctc aga ttc aag tgg ttc aag aat ggg 2136
   Glu Tyr Ser Ser Leu Arg Phe Lys Trp Phe Lys Asn Gly
       590                 595                 600
   aat gaa ttg aat cga aaa aac aaa cca caa aat atc aag 2175
   Asn Glu Leu Asn Arg Lys Asn Lys Pro Gln Asn Ile Lys
                   605                 610
   ata caa aaa aag cca ggg aag tca gaa ctt cgc att aac 2214
   Ile Gln Lys Lys Pro Gly Lys Ser Glu Leu Arg Ile Asn
   615                 620                 625
   aaa gca tca ctg gct gat tct gga gag tat atg tgc aaa 2253
   Lys Ala Ser Leu Ala Asp Ser Gly Glu Tyr Met Cys Lys
           630                 635                 640
   gtg atc agc aaa tta gga aat gac agt gcc tct gcc aat 2292
   Val Ile Ser Lys Leu Gly Asn Asp Ser Ala Ser Ala Asn
                   645                 650
   atc acc atc gtg gaa tca aac gag atc atc act ggt atg 2331
   Ile Thr Ile Val Glu Ser Asn Glu Ile Ile Thr Gly Met
       655                 660                 665
   cca gcc tca act gaa gga gca tat gtg tct tca gag tct 2370
   Pro Ala Ser Thr Glu Gly Ala Tyr Val Ser Ser Glu Ser
                   670                 675
   ccc att aga ata tca gta tcc aca gaa gga gca aat act 2409
   Pro Ile Arg Ile Ser Val Ser Thr Glu Gly Ala Asn Thr
   680                 685                 690
   tct tca tct aca tct aca tcc acc act ggg aca agc cat 2448
   Ser Ser Ser Thr Ser Thr Ser Thr Thr Gly Thr Ser His
           695                 700                 705
   ctt gta aaa tgt gcg gag aag gag aaa act ttc tgt gtg 2487
   Leu Val Lys Cys Ala Glu Lys Glu Lys Thr Phe Cys Val
                   710                 715
   aat gga ggg gag tgc ttc atg gtg aaa gac ctt tca aac 2526
   Asn Gly Gly Glu Cys Phe Met Val Lys Asp Leu Ser Asn
       720                 725                 730
   ccc tcg aga tac ttg tgc aag tgc cca aat gag ttt act 2565
   Pro Ser Arg Tyr Leu Cys Lys Cys Pro Asn Glu Phe Thr
               735                 740
   ggt gat cgc tgc caa aac tac gta atg gcc agc ttc tac 2604
   Gly Asp Arg Cys Gln Asn Tyr Val Met Ala Ser Phe Tyr
   745                 750                 755
   agt acg tcc act ccc ttt ctg tct ctg cct gaa tag 2640
   Ser Thr Ser Thr Pro Phe Leu Ser Leu Pro Glu
           760                 765         768
   gagcatgctc agttggtgct gctttcttgt tgctgcatct cccctcagat 2690
   tccacctaga gctagatgtg tcttaccaga tctaatattg actgcctctg 2740
   cctgtcgcat gagaacatta acaaaagcaa ttgtattact tcctctgttc 2790
   gcgactagtt ggctctgaga tactaatagg tgtgtgaggc tccggatgtt 2840
   tctggaattg atattgaatg atgtgataca aattgatagt caatatcaag 2890
   cagtgaaata tgataataaa ggcatttcaa agtctcactt ttattgataa 2940
   aataaaaatc attctactga acagtccatc ttctttatac aatgaccaca 2990
   tcctgaaaag ggtgttgcta agctgtaacc gatatgcact tgaaatgatg 3040
   gtaagttaat tttgattcag aatgtgttat ttgtcacaaa taaacataat 3090
   aaaaggaaaa aaaaaaaaaa a 3111
<210> 13
   <211> 1872
   <212> DNA
   <213> Homo sapiens
<400> 13
   gaattcggga cagcctctcc tgccgccgct gctgctgccg ccgccgccac 50
   cgccggctgg tcctccttct gcttttactt ctcctgcatg acagttgttt 100
   tcttcatctg agcagacacc agcttcagat gctcgaggtg agaaacatgc 150
   ctttcagttt gggctactgg tttacttaat taatcagccg gcagctccgt 200
   cgatctattt tcgtccctgt cctcttgacg agcccgggat ggtttggagt 250
   agcatttaaa agaactagaa aagtggccca gaaacagcag cttaaagaat 300
   tattacgata tactttgatt ttgtagttgc taggagcttt tcttcccccc 350
   ttgcatcttt ctgaactctt cttgatttta ataatggcct tggacttgga 400
   cgatttatcg atttccccct gtaagatgct gtatcatttg gttggggggg 450
   cctctgcgtg gtaatggacc gtgagagcgg ccaggccttc ttctggaggt 500
   gagccg  atg gag att tat tcc cca gac atg tct gag gtc 539
           Met Glu Ile Tyr Ser Pro Asp Met Ser Glu Val
            1               5                  10
   gcc gcc gag agg tcc tcc agc ccc tcc act cag ctg agt 578
   Ala Ala Glu Arg Ser Ser Ser Pro Ser Thr Gln Leu Ser
               15                  20
   gca gac cca tct ctt gat ggg ctt ccg gca gca gaa gac 617
   Ala Asp Pro Ser Leu Asp Gly Leu Pro Ala Ala Glu Asp
    25                  30                  35
   atg cca gag ccc cag act gaa gat ggg aga acc cct gga 656
   Met Pro Glu Pro Gln Thr Glu Asp Gly Arg Thr Pro Gly
            40                  45                  50
   ctc gtg ggc ctg gcc gtg ccc tgc tgt gcg tgc cta gaa 695
   Leu Val Gly Leu Ala Val Pro Cys Cys Ala Cys Leu Glu
                    55                  60
   gct gag cgc ctg aga ggt tgc ctc aac tca gag aaa atc 734
   Ala Glu Arg Leu Arg Gly Cys Leu Asn Ser Glu Lys Ile
        65                  70                  75
   tgc att gtc ccc atc ctg gct tgc ctg gtc agc ctc tgc 773
   Cys Ile Val Pro Ile Leu Ala Cys Leu Val Ser Leu Cys
                80                  85
   ctc tgc atc gcc ggc ctc aag tgg gta ttt gtg gac aag 812
   Leu Cys Ile Ala Gly Leu Lys Trp Val Phe Val Asp Lys
    90                  95                 100
   atc ttt gaa tat gac tct cct act cac ctt gac cct ggg 851
   Ile Phe Glu Tyr Asp Ser Pro Thr His Leu Asp Pro Gly
           105                 110                 115
   ggg tta ggc cag gac cct att att tct ctg gac gca act 890
   Gly Leu Gly Gln Asp Pro Ile Ile Ser Leu Asp Ala Thr
                   120                 125
   gct gcc tca gct gtg tgg gtg tcg tct gag gca tac act 929
   Ala Ala Ser Ala Val Trp Val Ser Ser Glu Ala Tyr Thr
       130                 135                 140
   tca cct gtc tct agg gct caa tct gaa agt gag gtt caa 968
   Ser Pro Val Ser Arg Ala Gln Ser Glu Ser Glu Val Gln
               145                 150
   gtt aca gtg caa ggt gac aag gct gtt gtc tcc ttt gaa 1007
   Val Thr Val Gln Gly Asp Lys Ala Val Val Ser Phe Glu
   155                 160                 165
   cca tca gcg gca ccg aca ccg aag aat cgt att ttt gcc 1046
   Pro Ser Ala Ala Pro Thr Pro Lys Asn Arg Ile Phe Ala
           170                 175                 180
   ttt tct ttc ttg ccg tcc act gcg cca tcc ttc cct tca 1085
   Phe Ser Phe Leu Pro Ser Thr Ala Pro Ser Phe Pro Ser
                   185                190
   ccc acc cgg aac cct gag gtg aga acg ccc aag tca gca 1124
   Pro Thr Arg Asn Pro Glu Val Arg Thr Pro Lys Ser Ala
       195                 200                205
   act cag cca caa aca aca gaa act aat ctc caa act gct 1163
   Thr Gln Pro Gln Thr Thr Glu Thr Asn Leu Gln Thr Ala
               210                 215
   cct aaa ctt tct aca tct aca tcc acc act ggg aca agc 1202
   Pro Lys Leu Ser Thr Ser Thr Ser Thr Thr Gly Thr Ser
   220                 225                 230
   cat ctt gta aaa tgt gcg gag aag gag aaa act ttc tgt 1241
   His Leu Val Lys Cys Ala Glu Lys Glu Lys Thr Phe Cys
           235                 240                 245
   gtg aat gga ggg gag tgc ttc atg gtg aaa gac ctt tca 1280
   Val Asn Gly Gly Glu Cys Phe Met Val Lys Asp Leu Ser
                   250                 255
   aac ccc tcg aga tac ttg tgc aag tgc cca aat gag ttt 1319
   Asn Pro Ser Arg Tyr Leu Cys Lys Cys Pro Asn Glu Phe
       260                 265                 270
   act ggt gat cgc tgc caa aac tac gta atg gcc agc ttc 1358
   Thr Gly Asp Arg Cys Gln Asn Tyr Val Met Ala Ser Phe
               275                 280
   tac agt acg tcc act ccc ttt ctg tct ctg cct gaa taggag 1400
   Tyr Ser Thr Ser Thr Pro Phe Leu Ser Leu Pro Glu
   285                 290                 295 296
   catgctcagt tggtgctgct ttcttgttgc tgcatctccc ctcagattcc 1450
   acctagagct agatgtgtct taccagatct aatattgact gcctctgcct 1500
   gtcgcatgag aacattaaca aaagcaattg tattacttcc tctgttcgcg 1550
   actagttggc tctgagatac taataggtgt gtgaggctcc ggatgtttct 1600
   ggaattgata ttgaatgatg tgatacaaat tgatagtcaa tatcaagcag 1650
   tgaaatatga taataaaggc atttcaaagt ctcactttta ttgataaaat 1700
   aaaaatcatt ctactgaaca gtccatcttc tttatacaat gaccacatcc 1750
   tgaaaagggt gttgctaagc tgtaaccgat atgcacttga aatgatggta 1800
   agttaatttt gattcagaat gtgttatttg tcacaaataa acataataaa 1850
   aggaaaaaaa aaacccgaat tc 1872
<210> 14
   <211> 296
   <212> PRT
   <213> Homo sapiens
<400> 14
   Met Glu Ile Tyr Ser Pro Asp Met Ser Glu Val Ala Ala Glu Arg
   1               5                   10                  15
   Ser Ser Ser Pro Ser Thr Gln Leu Ser Ala Asp Pro Ser Leu Asp
                   20                  25                  30
   Gly Leu Pro Ala Ala Glu Asp Met Pro Glu Pro Gln Thr Glu Asp
                   35                  40                  45
   Gly Arg Thr Pro Gly Leu Val Gly Leu Ala Val Pro Cys Cys Ala
                   50                  55                  60
   Cys Leu Glu Ala Glu Arg Leu Arg Gly Cys Leu Asn Ser Glu Lys
                   65                  70                  75
   Ile Cys Ile Val Pro Ile Leu Ala Cys Leu Val Ser Leu Cys Leu
                   80                  85                  90
   Cys Ile Ala Gly Leu Lys Trp Val Phe Val Asp Lys Ile Phe Glu
                   95                  100                 105
   Tyr Asp Ser Pro Thr His Leu Asp Pro Gly Gly Leu Gly Gln Asp
                   110                 115                 120
   Pro Ile Ile Ser Leu Asp Ala Thr Ala Ala Ser Ala Val Trp Val
                   125                 130                 135
   Ser Ser Glu Ala Tyr Thr Ser Pro Val Ser Arg Ala Gln Ser Glu
                   140                 145                 150
   Ser Glu Val Gln Val Thr Val Gln Gly Asp Lys Ala Val Val Ser
                   155                 160                 165
   Phe Glu Pro Ser Ala Ala Pro Thr Pro Lys Asn Arg Ile Phe Ala
                   170                 175                 180
   Phe Ser Phe Leu Pro Ser Thr Ala Pro Ser Phe Pro Ser Pro Thr
                   185                 190                 195
   Arg Asn Pro Glu Val Arg Thr Pro Lys Ser Ala Thr Gln Pro Gln
                   200                 205                 210
   Thr Thr Glu Thr Asn Leu Gln Thr Ala Pro Lys Leu Ser Thr Ser
                   215                 220                 225
   Thr Ser Thr Thr Gly Thr Ser His Leu Val Lys Cys Ala Glu Lys
                   230                 235                 240
   Glu Lys Thr Phe Cys Val Asn Gly Gly Glu Cys Phe Met Val Lys
                   245                 250                 255
   Asp Leu Ser Asn Pro Ser Arg Tyr Leu Cys Lys Cys Pro Asn Glu
                   260                 265                 270
   Phe Thr Gly Asp Arg Cys Gln Asn Tyr Val Met Ala Ser Phe Tyr
                   275                 280                 285
   Ser Thr Ser Thr Pro Phe Leu Ser Leu Pro Glu
                   290                 295 296